(19) Europäisches Patentamt
European Patent Office
Office européen des brevets

(11) Veröffentlichungsnummer: **0 003 960**
**B1**

(12) **EUROPÄISCHE PATENTSCHRIFT**

(45) Veröffentlichungstag der Patentschrift: **29.06.83**

(21) Anmeldenummer: **79100258.7**

(22) Anmeldetag: **30.01.79**

(51) Int. Cl.³: **C 07 D 499/00,**
C 07 F 9/65, A 61 K 31/43,
C 07 D 205/08

(54) **6-Substituierte Thia-Azaverbindungen, ihre Herstellung und diese enthaltende pharmazeutische Präparate.**

(30) Priorität: **02.02.78 CH 1140/78**

(43) Veröffentlichungstag der Anmeldung:
**19.09.79 Patentblatt 79/19**

(45) Bekanntmachung des Hinweises auf die Patenterteilung:
**29.06.83 Patentblatt 83/26**

(84) Benannte Vertragsstaaten:
**BE CH DE FR GB IT LU NL SE**

(56) Entgegenhaltungen:
**EP - A - 0 002 210**
**DE - A - 2 205 123**
**DE - A - 2 819 655**
**US - A - 4 123 539**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG**
**Patentabteilung Postfach**
**CH-4002 Basel (CH)**

(72) Erfinder: **Gosteli, Jacques, Dr.**
**Anwilerstrasse 10**
**CH-4059 Basel (CH)**
Erfinder: **Ernest, Ivan, Dr.**
**Rüttihard 10**
**CH-4127 Birsfelden (CH)**
Erfinder: **Lang, Marc, Dr.**
**Rue de Thann 4**
**F-68200 Mulhouse (FR)**
Erfinder: **Woodward, Robert Burns, Prof. Dr.**
**12 Oxford Street**
**Cambridge Massachusetts 02138 (US)**

(74) Vertreter: **Zumstein, Fritz sen., Dr. et al,**
**Bräuhausstrasse 4**
**D-8000 München 2 (DE)**

EP 0 003 960 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 6-Substituierte Thia-Azaverbindungen, ihre Herstellung und diese enthaltende pharmazeutische Präparate

Die vorliegende Erfindung betrifft neue bicyclische Thia-azaverbindungen enthaltend einen in 3-Stellung substituierten β-Lactamring mit antibiotischen Eigenschaften.

Seit der Entdeckung des Penicillins sind zahlreiche bicyclische Thia-azaverbindungen mit β-Lactamstruktur bekannt geworden. Eine Uebersicht über frühere Arbeiten gibt E. H. Flynn, "Cephalosporins and Penicillins", Academic Press, New York and London, 1972. Neuere Entwicklungen sind von J. Cs. Jászberényi et al., Progr. Med. Chem., Vol. 12, *1975* 395—477, P. G. Sammes, Chem. Rev. *1976*, Vol. 76, Nr. 1, 113—155, und von verschiedenen Autoren an einem internationalen Symposium der Chemical Society in Cambridge England, Juni 1976 (nachfolgende Publikation: J. Elks, "Recent Advances in the Chemistry of β-Lactam Antibiotics", The Chemical Society, Burlington House, London, 1977), beschrieben worden.

Neben den üblichen Penam- und Cephem-verbindungen, die in 6-bzw. 7-Stellung eine Acylaminogruppe tragen, sind auch solche bekannt geworden, die in diesen Stellungen unsubstituiert sind, z.B. 3-Carboxy-2,2-dimethylpenam (J. P. Clayton, J. Chem. Soc., *1969*, 2123) und 3-Methyl-4-carboxy-3-cephem (K. Kühlein, Liebigs Ann., *1974*, S. 369 und D. Bormann, ibid., S. 1391). 3-Carboxy-2,2-dimethylpenamverbindungen, die anstatt der üblichen 6β-Acylaminogruppe eine 6α-Chlor- oder 6α-Bromgruppierung aufweisen, sind durch I. McMillan und R. J. Stoodley, Tetrahedron Lett. 1205 (1966), und J. Chem. Soc. C 2533 (1968) bekannt geworden, während entsprechende 6α-Hydroxy-, 6α-Acetoxy- und 6α-Phenoxyacetoxy-2,2-dimethyl-penam-3-carbonsäuren von D. Hauser und H. P. Sigg, Helv. Chimica Acta *50*, 1327 (1967), beschrieben wurden. Von all diesen Verbindungen ist jedoch entweder gar keine oder keine wesentliche antibiotische Wirksamkeit bekannt geworden.

6-Acylamino-2-penem-3-carbonsäureverbindungen mit antibiotischer Wirkung, die das neuartige 2-Penamringsystem enthalten, sind in der DE—A—2.655.298 beschrieben.

In 6-Stellung andere als Acylaminosubstituenten tragende 2-Penemverbindungen sind bisher nicht bekannt geworden.

Der vorliegenden Erfindung liegt die Aufgabe zugrunde, einen β-Lactamring enthaltende bicyclische Thiaazaverbindungen herzustellen, die das in 6-Stellung substituierte 2-Penem-Ringsystem besitzen, und die sowohl gegen Penicillin-empfindliche als auch gegen Penicillinresistente Keime wirksam sind.

Die erfindungsgemässe Herstellung der neuartigen Verbindungen, eröffnen darüber hinaus neue Gebiete, auf denen nach weiteren, technisch verwertbaren Verbindungen geforscht werden kann.

Das Ringsystem der Verbindungen der vorliegenden Erfindung hat die Formel

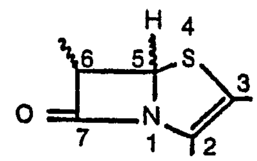

und kann systematisch als 7-Oxo-4-thia-1-azabicyclo[3.2.0] hept-2-en bezeichnet werden. Der Einfachheit halber wird es im folgenden als "2-Penem" bezeichnet, wobei die folgende in der Penicillinchemie übliche, vom Penam abgeleitete Bezifferung benutzt wird:

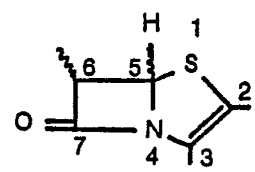

Gegenstand der vorliegenden Erfindung sind 2-Penem-3-carbonsäureverbindungen der Formel

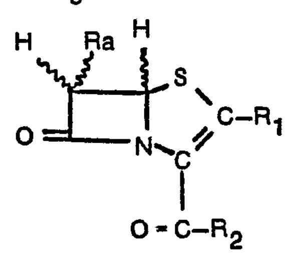

(I)

worin $R_a$ ein gesättigter oder ungesättigter, gegebenenfalls substituierter, aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen,

ein gegebenenfalls substituierter Heterocycl- oder Heterocyclylniederalkylrest mit bis zu 10

Kohlenstoffatomen und bis zu 4 Ringheteroatomen aus der Gruppe Stickstoff, Sauerstoff und/oder Schwefel,

in welchen Resten $R_a$ die gegebenenfalls vorhandenen Substituenten Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder $C_1$—$C_{20}$-Acyl mono- oder disubstituiertes oder durch Niederalkylen disubstituiertes Amino sind,

gegebenenfalls geschütztes Carboxyl,

Hydroxy,

Mercapto,

durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy oder Mercapto, worin

die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$—$C_{20}$-Acyl mono- oder disubstituiertes oder durch Niederalkylen disubstituiertes Amino sind,

durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen verestertes Hydroxy oder Mercapto, worin

die gegebenenfalls vorhandenen Substituenten Hydroxy, Niederalkoxy, Phenyloxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$—$C_{20}$-Acyl mono- oder disubstituiertes oder durch Niederalkylen disubstituiertes Amino sind, oder

Halogen ist,

$R_1$ Wasserstoff, ein gesättigter oder ungesättigter, gegebenenfalls substituierter aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, oder

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel ist,

in welchen Resten $R_1$ die gegebenenfalls vorhandenen Substituenten Hydroxy, Niederalkoxy, Niederalkanoyloxy, Halogen, Mercapto, Niederalkylmercapto, gegebenenfalls durch Niederalkyl, Hydroxyniederalkyl, Carboxyniederalkyl, Aminoniederalkyl, Diniederalkylaminoniederalkyl, in Salzform vorliegendes Sulfoniederalkyl, Cycloalkyl, Aryl, Arylniederalkyl, Halogen, Amino, Mono- oder Diniederalkylamino, Nitro, Hydroxy, Niederalkoxy, Carboxyl, Niederalkoxycarbonyl, Carbamoyl, N-Mono- oder N,N-Diniederalkylcarbamoyl, Cyan, Oxo oder Oxido substituiertes Heterocyclylmercapto, Carboxyl, Niederalkoxycarbonyl, Carbamoyl, Cyan, Nitro, Amino, Niederalkylamino, Diniederalkylamino, $C_1$—$C_{20}$-Acylamino, Di-$C_1$—$C_{20}$-acylamino oder Niederalkylenamino sind, oder worin $R_1$ eine durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen, verätherte Mercaptogruppe ist,

worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, gegebenenfalls durch Niederalkyl oder $C_1$—$C_{20}$-Acyl mono- oder disubstituiertes oder durch Niederalkylen disubstituiertes Amino, Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$—$C_{20}$-Acylamino, oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl oder Oxazolyl sind, oder worin

$R_1$ eine durch einen gegebenenfalls substituierten heterocyclischen Rest verätherte Mercaptogruppe ist, worin der Heterocyclylrest über ein Ringkohlenstoffatom an die Mercaptogruppe gebunden ist und 1 bis 4 Ringstoffatome und gegebenenfalls ein weiteres Ringheteroatom der Gruppe Sauerstoff und Schwefel enthält und worin die gegebenenfalls vorhandenen Substituenten Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$—$C_{20}$-Acylamino oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl, Oxazolyl, Halogen, gegebenenfalls durch Niederalkyl mono- oder disubstituiertes Amino, $C_1$—$C_{20}$-Acylamino, Nitro, Hydroxy, Niederalkoxy, Carboxyl, Niederalkoxycarbonyl, gegebenenfalls N-mono- oder N,N-diniederalkyliertes Carbamoyl, Cyan, Oxo oder Oxido sind,

wobei die mit "Nieder" bezeichneten Gruppen bis zu 7, insbesondere bis zu 4 Kohlenstoffatome enthalten, und $R_2$ Hydroxy oder einen zusammen mit der Carbonylgruppierung —C(=O)— eine geschützte Carboxylgruppe bildenden Rest $R_2^A$ bedeutet, sowie Salze von solchen Verbindungen mit salzbildenden Gruppen.

Ein gesättigter oder ungesättigter, gegebenenfalls substituierter, aliphatischer, cycloaliphatischer, cycloaliphatisch-aliphatischer, aromatischer oder araliphatischer Kohlenwasserstoffrest $R_a$ mit bis zu 18, bevorzugt bis zu 10 Kohlenstoffatomen, ist insbesondere gegebenenfalls substituiertes Niederalkyl oder Niederalkenyl, oder gegebenenfalls substituiertes Cycloalkyl, Cycloalkenyl, Cycloalkylniederalkyl, Cycloalkylniederalkenyl, Cycloalkenylniederalkyl, Phenyl, Phenylniederalkyl, oder Phenylniederalkenyl.

Substituenten von solchen Resten und von gegebenenfalls substituierten Heterocyclyl- oder Heterocyclylniederalkylresten mit bis zu 10 Kohlenstoffatomen und bis zu 4 Ringheteroatomen aus der Gruppe Stickstoff, Sauerstoff und/oder Schwefel sind z.B. gegebenenfalls funktionell abgewandelte, wie gegebenenfalls verätherte oder veresterte Hydroxy- oder Mercaptogruppen, z.B. Hydroxy, Niederalkoxy, z.B. Methoxy oder Aethoxy, Niederalkanoyloxy, z.B. Acetyloxy oder Propionyloxy, in Salzform vorliegendes Hydroxysulfonyloxy, Halogen, z.B. Chlor oder Brom, oder Niederalkylmercapto, z.B. Methylthio, gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro, in Salzform vorliegendes Sulfo, oder gegebenenfalls substituiertes, wie durch Niederalkyl, z.B. Methyl oder Aethyl, Acyl, wie Niederalkanoyl, z.B. Acetyl, mono- oder disubstituiertes, oder durch Niederalkylen, z.B. 1,4-Butylen oder 1,5-Pentylen, disubstituiertes Amino.

Ein Niederalkylrest $R_a$ enthält bis zu 7, insbesondere bis zu 4 Kohlenstoffatome, und ist z.B. Methyl, Aethyl, Propyl, Isopropyl, Butyl, Isobutyl, tert.-Butyl oder Pentyl. Substituiertes Niederalkyl $R_a$ ist in erster Linie substituiertes Methyl, Aethyl oder Propyl, wobei Substituenten insbesondere in 1-, aber auch in 2- oder in 3-Stellung stehen, und ist beispielsweise Hydroxyniederalkyl, wie Hydroxymethyl, Hydroxyäthyl oder Hydroxypropyl, Niederalkoxyniederalkyl, wie Niederalkoxymethyl, Niederalkoxyäthyl oder Niederalkoxypropyl, z.B. Methoxymethyl, Methoxyäthyl oder Methoxypropyl, Niederalkanoyloxyniederalkyl, wie Niederalkanoyloxymethyl, Niederalkanoyloxyäthyl oder Niederalkanoyloxypropyl, z.B. Acetyloxymethyl, Propionyloxymethyl, Acetyloxyäthyl, Acetyloxypropyl, in Salzform, z.B. als Alkalimetall-, wie Natriumsalz, oder als Ammoniumsalz vorliegendes Hydroxysulfonyloxyniederalkyl, wie Hydroxysulfonyloxymethyl, Hydroxysulfonyloxyäthyl oder Hydroxysulfonyloxypropyl, Halogenniederalkyl, wie Halogenmethyl, Halogenathyl oder Halogenpropyl, z.B. Chlor- oder Bromäthyl, oder Chlor- oder Brompropyl, Niederalkylthioniederalkyl, wie Methylthiomethyl, Methylthioäthyl, Methylthiopropyl oder tert.-Butylthiomethyl, Niederalkoxycarbonylniederalkyl, wie Niederalkoxycarbonylmethyl oder Niederalkoxycarbonyläthyl, z.B. Methoxycarbonylmethyl, Methoxycarbonyläthyl, Aethoxycarbonylmethyl oder Aethoxycarbonyläthyl, Cyanniederalkyl, wie Cyanmethyl oder Cyanäthyl, Sulfoniederalkyl, wie Sulfomethyl, Sulfoäthyl oder Sulfopropyl, worin die Sulfogruppe in Salzform, z.B. als Alkalimetall-, wie Natriumsalz oder auch als Ammoniumsalz vorliegt, oder gegebenenfalls geschütztes, z.B. auch acetyliertes, Aminoniederalkyl, wie Aminomethyl Aminoäthyl oder Aminopropyl.

Ein Niederalkenylrest $R_a$ enthält 2 bis 7, insbesondere 2 bis 4 Kohlenstoffatome, und ist z.B. Vinyl, Allyl oder 2- oder 3-Butenyl. Substituiertes Niederalkenyl kann die gleichen Substituenten tragen wie substituiertes Niederalkyl.

Eine gegebenenfalls geschützte Carboxylgruppe $R_a$ ist eine freie oder eine der unter den Gruppen $-C(=O)-R_2^A$ genannten, beispielsweise veresterten oder amidierten Carboxylgruppen, wie Niederalkoxycarbonyl, z.B. Methoxy-, Aethoxy- oder tert.-Butoxycarbonyl, Arylniederalkoxycarbonyl, wie Benzyloxy-, p-Nitrobenzyloxy- oder Diphenylmethoxycarbonyl, Aryloxycarbonyl, wie gegebenenfalls, z.B. durch Halogen, wie Chlor, Niederalkoxy, wie Methoxy, oder Nitro, substituiertes Phenyloxycarbonyl, wie Phenyloxycarbonyl, o-, m- oder p-Chlorphenyloxy-, Pentachlorphenyloxy-, o-, m- oder p-Methoxyphenyloxy- oder p-Nitrophenyloxycarbonyl, Aminocarbonyl oder substituiertes, wie durch Niederalkyl, z.B. Methyl oder Aethyl, mono- oder disubstituiertes Aminocarbonyl.

Ein Cycloalkylrest $R_a$ weist z.B. 3 bis 7 Kohlenstoffatome auf und ist z.B. Cyclopropyl, Cyclobutyl, Cyclopentyl oder Cyclohexyl, während ein Cycloalkylniederalkylrest $R_a$ beispielsweise 4 bis 7 Kohlenstoffatome enthält und z.B. Cyclopropylmethyl, Cyclobutylmethyl, Cyclopentylmethyl oder Cyclohexylmethyl darstellt.

Ein Cycloalkenylrest $R_a$ ist ein entsprechender Cycloalkylrest mit ein oder gegebenenfalls zwei C—C-Doppelbindungen, wie Cyclohexenyl, z.B. 1-Cyclohexenyl, oder Cyclohexadienyl, z.B. 1,4-Cyclohexadienyl.

Ein Cycloalkylniederalkenylrest oder Cycloalkenylniederalkylrest $R_a$ ist z.B. Cyclohexylvinyl, Cyclohexylallyl, bzw. Cyclohexenylmethyl oder 1,4-Cyclohexadienylmethyl.

Ein Phenyl- oder ein Phenylniederalkyl-, z.B. Benzyl- oder 1- oder 2-Phenyläthylrest $R_a$ kann, vorzugsweise im aromatischen Rest, z.B. durch Niederalkyl, wie Methyl oder Aethyl, Niederalkoxy, wie Methoxy, oder Halogen, wie Fluor oder Chlor, ferner durch Nitro oder Amino substituiert sein, wobei Phenylniederalkyl in $\alpha$-Stellung z.B. durch Hydroxy, Hydroxysulfonyloxy, Carboxyl, Sulfo oder Amino substituiert sein kann.

In einem Heterocyclyl- oder Heterocyclylniederalkylrest $R_a$ ist Heterocyclyl ein über ein Kohlenstoffatom gebundener Rest vorzugsweise aromatischen Charakters, wie Pyridyl, z.B. 2-, 3- oder 4-Pyridyl, Thienyl, z.B. 2-Thienyl, oder Furyl, z.B. 2-Furyl, oder ein entsprechender Pyridyl-, Thienyl- oder Furylniederalkyl-, insbesondere -methylrest, wobei Heterocyclylniederalkyl in $\alpha$-Stellung z.B. durch Hydroxy, Hydroxysulfonyloxy, Carboxy, Sulfo oder Amino substituiert sein kann.

Ein Phenyl- oder Heterocyclylniederalkenylrest $R_a$ ist ein wie ein entsprechender Niederalkylrest substituierten Niederalkenylrest, z.B. Phenylvinyl oder Furylallyl.

Ein Phenyl-, Naphthyl- oder Heterocyclylniederalkenylrest $R_a$ ist ein wie ein entsprechender Niederalkylrest substituierter Niederalkenylrest, z.B. Phenyl vinyl oder Furylallyl.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy $R_a$ ist insbesondere gegebenenfalls substituiertes Niederalkoxy, Cycloalkoxy, Cycloalkylniederalkoxy, Phenoxy, Naphthyloxy oder Phenylniederalkoxy. Substituenten von solchen Resten sind z.B. gegebenenfalls funktionell abgewandeltes, wie gegebenenfalls veräthertes oder verestertes Hydroxy oder Mercapto, z.B. Hydroxy, Niederalkoxy, z.B. Methoxy oder Aethoxy, Niederalkanoyloxy, z.B. Acetyloxy oder Propionyloxy, Halogen, z.B. Chlor oder Brom, oder Niederalkylmercapto, z.B. Methylthio, oder gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro oder gegebenenfalls, wie durch Niederalkyl, z.B. Methyl oder Aethyl, durch Acyl, wie Niederalkanoyl, z.B. Acetyl, mono- oder disubstituiertes, oder durch Niederalkylen, z.B. 1,4-Butylen oder 1,5-Pentylen disubstituiertes Amino.

Ein Niederalkoxyrest $R_a$ enthält bis zu 7, insbesondere bis zu 4 Kohlenstoffatome, und ist u.a. Methoxy, Aethoxy, Propoxy, Isopropoxy, Butoxy, Isobutoxy, tert.-Butoxy oder Pentoxy. Substituiertes Niederalkoxy $R_a$ ist in erster Linie substituiertes Methoxy, Aethoxy oder Propoxy, wobei Substituenten in 1-, 2- oder 3-Stellung stehen, wie Methoxymethoxy, Aethoxymethoxy, Methoxyäthoxy oder Methoxypropoxy, Niederalkanoyloxymethoxy, Niederalkanoyloxyäthoxy oder Niederalkanoyloxypropoxy, wie Acetoxymethoxy, Acetoxyäthoxy oder Acetoxypropoxy, Halogenmethoxy, Halogenäthoxy oder Halogenpropoxy, z.B. Chlor- oder Bromäthoxy oder Chlor- oder Brompropoxy, Niederalkoxycarbonylmethoxy oder Niederalkoxycarbonyläthoxy, z.B. Methoxycarbonylmethoxy, Aethoxycarbonylmethoxy oder Methoxycarbonyläthoxy, Cyanmethoxy, Cyanäthoxy, oder gegebenenfalls geschütztes Aminomethoxy, Aminoäthoxy oder Aminopropoxy.

Eine Cycloalkoxygruppe $R_a$ weist z.B. 3 bis 7 Kohlenstoffatome auf und ist z.B. Cyclopropyloxy, Cyclobutyloxy, Cyclopentyloxy oder Cyclohexyloxy.

Ein Cycloalkylniederalkoxyrest $R_a$ hat z.B. 4 bis 7 Kohlenstoffatome und ist z.B. Cyclopropylmethoxy, Cyclobutylmethoxy, Cyclopentylmethoxy oder Cyclohexylmethoxy.

Ein Phenoxy- oder ein Phenylniederalkoxy-, z.B. Benzyl- oder 1- oder 2-Phenyläthoxyrest $R_a$ kann, vorzugsweise im aromatischen Rest, z.B. durch Niederalkyl, wie Methyl oder Aethyl, Niederalkoxy, wie Methoxy, Halogen, wie Fluor oder Chlor, Nitro oder Amino substituiert sein.

Durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen veresterte Hydroxygruppen $R_a$ sind insbesondere gegebenenfalls substituiertes Niederalkanoyloxy, Cycloalkanoyloxy, Cycloalkyl-niederalkanoyloxy, Benzoyloxy, oder Phenylniederalkanoyloxy. Substituenten solcher Reste sind z.B. gegebenenfalls funktionell abgewandeltes, wie gegebenenfalls veräthertes oder verestertes Hydroxy oder Mercapto, z.B. Hydroxy, Niederalkoxy, z.B. Methoxy, oder Aethoxy, Aryloxy, z.B. Phenyloxy, Niederalkanoyloxy, z.B. Acetyloxy oder Propionyloxy, Halogen, z.B. Chlor oder Brom, oder Niederalkylmercapto, z.B. Methylthio, oder gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro oder gegebenenfalls, wie durch Niederalkyl, z.B. Methyl oder Aethyl, durch Acyl, wie Niederalkanoyl, z.B. Acetyl, mono- oder disubstituiertes, oder durch Niederalkylen, z.B. 1,4-Butylen oder 1,5-Pentylen, disubstituiertes Amino.

Ein Niederalkanoyloxyrest $R_a$ enthält bis zu 7, insbesondere bis zu 4 Kohlenstoffatome und ist z.B. Formyloxy, Acetoxy, Propionyloxy oder Butyryloxy. Substituiertes Niederalkanoyloxy $R_a$ ist in erster Linie substituiertes Acetoxy, z.B. Hydroxyacetoxy, Methoxyacetoxy, Phenyloxyacetoxy, Halogenacetoxy, z.B. Chlor- oder Bromacetoxy, Cyanacetoxy, oder gegebenenfalls geschütztes Glycyloxy.

Ein Cycloalkanoyloxyrest $R_a$ hat 4 bis 8 Kohlenstoffatome und ist z.B. Cyclopropylcarbonyloxy, Cyclobutylcarbonyloxy, Cyclopentylcarbonyloxy oder Cyclohexylcarbonyloxy, oder ein entsprechender, z.B. in 1-Stellung, z.B. durch Hydroxy oder Amino, substituierter Rest.

Ein Cycloalkylniederalkanoyloxyrest $R_a$ hat 5 bis 9 Kohlenstoffatome und ist z.B. Cyclopropylacetoxy, Cyclobutylacetoxy, Cyclohexylacetoxy oder Cyclohexylpropionoxy, oder ein entsprechender, z.B. in 1-Stellung, z.B. durch Hydroxy oder Amino, substituierter Rest.

Ein Benzoyloxy- oder Phenylniederalkanoyloxy-, z.B. Phenylacetyloxyrest $R_a$ kann, vorzugsweise im aromatischen Rest, z.B. durch Niederalkyl, wie Methyl oder Aethyl, Niederalkoxy, wie Methoxy, Halogen, wie Fluor oder Chlor, Nitro, oder gegebenenfalls geschütztes Hydroxy oder Amino, substituiert sein. Im Phenylniederalkanoyloxyrest kann gegebenenfalls substituiertes, z.B. geschütztes Hydroxy oder gegebenenfalls substituiertes, z.B. geschütztes Amino auch im aliphatischen Teil stehen, insbesondere in 2-Stellung.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Mercapto $R_a$ ist insbesondere gegebenenfalls substituiertes Niederalkylthio, Cycloalkylthio, Cycloalkylniederalkylthio, Phenylthio oder Phenylniederalkylthio. Substituenten von solchen Resten sind z.B. gegebenenfalls funktionell abgewandeltes, wie gegebenenfalls veräthertes oder verestertes Hydroxy oder Mercapto, z.B. Hydroxy, Niederalkoxy, z.B. Methoxy oder Aethoxy, Niederalkanoyloxy, z.B. Acetyloxy oder Propionyloxy, Halogen, z.B. Chlor oder Brom, oder Niederalkylmercapto, z.B. Methylthio, oder gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro oder gegebenenfalls, wie durch Niederalkyl, z.B. Methyl oder Aethyl, durch Acyl, wie Niederalkanoyl, z.B. Acetyl, mono- oder disubstituiertes, oder durch Niederalkylen, z.B. 1,4-Butylen oder 1,5-Pentylen disubstituiertes Amino.

Ein Niederalkylthiorest $R_a$ enthält bis zu 7, insbesondere bis zu 4 Kohlenstoffatome, und ist u.a. Methylthio, Aethylthio, Propylthio, Isopropylthio, Butylthio, Isobutylthio, tert.-Butylthio oder Pentylthio. Substituiertes Niederalkylthio $R_a$ ist in erster Linie substituiertes Methylthio, Aethylthio oder Propylthio, wobei Substituenten in 1-, 2- oder 3-Stellung stehen, wie Methoxymethylthio, Aethoxymethylthio, Methoxyäthylthio oder Methoxypropylthio, Niederalkanoyloxymethylthio, Niederalkanoyloxyäthylthio oder Niederalkanoyloxypropylthio, wie Acetoxymethylthio, Acetoxyäthylthio oder Acetoxypropylthio, Halogenmethylthio, Halogenäthylthio oder Acetoxypropylthio, z.B. Chlor- oder Bromäthylthio oder Chlor- oder Brompropylthio, Niederalkoxycarbonylmethylthio oder Niederalkoxycarbonyläthylthio, z.B. Methoxycarbonyläthylthio, Cyanmethylthio, Cyanäthylthio oder gegebenenfalls geschütztes, z.B. N-acyliertes Aminomethylthio, Aminoäthylthio oder Aminopropylthio.

Eine Cycloalkylthiogruppe $R_a$ weist z.B. 3 bis 7 Kohlenstoffatome auf und ist z.B. Cyclopropylthio, Cyclobutylthio, Cyclopentylthio oder Cyclohexylthio.

Ein Cycloalkylniederalkylthiorest $R_a$ hat z.B. 4 bis 7 Kohlenstoffatome und ist z.B. Cyclopropylmethylthio, Cyclobutylmethylthio, Cyclopentylmethylthio oder Cyclohexylmethylthio.

Ein Phenylthio- oder ein Phenylniederalkylthio-, z.B. Benzyl- oder 1- oder 2-Phenylniederalkylthio-, z.B. Benzyl- oder 1- oder 2-Phenyläthylthiorest $R_a$ kann, vorzugsweise im aromatischen Rest, z.B. durch Niederalkyl, wie Methyl oder Aethyl, Niederalkoxy, wie Methoxy, Halogen, wie Fluor oder Chlor, Nitro oder Amino substituiert sein.

Durch einen Acylrest einer gegebenenfalls substituierterten aliphatischen, cycloaliphatischen, cycloaliphatisch-aliphatischen, aromatischen oder araliphatischen Carbonsäure mit bis zu 18 Kohlenstoffatomen veresterte Mercaptogruppen $R_a$ sind insbesondere gegebenenfalls substituiertes Niederalkanoylthio, Cycloalkanoylthio, Cycloalkylniederalkanoylthio, Benzoylthio oder Phenylniederalkanoylthio. Substituenten solcher Reste sind z.B. gegebenenfalls funktionell abgewandeltes, wie gegebenenfalls veräthertes oder verestertes Hydroxy oder Mercapto, z.B. Hydroxy, Niederalkoxy, z.B. Methoxy oder Aethoxy, Aryloxy, z.B. Phenyloxy, Niederalkanoyloxy, z.B. Acetyloxy oder Propionyloxy, Halogen, z.B. Chlor oder Brom, oder Niederalkylmercapto, z.B. Methylthio, oder gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro oder gegebenenfalls, wie durch Niederalkyl, z.B. Methyl oder Aethyl, durch Acyl, wie Niederalkanoyl, z.B. Acetyl, mono- oder disubstituiertes oder durch Niederalkylen, z.B. 1,4-Butylen oder 1,5-Pentylen, disubstituiertes Amino.

Ein Niederalkanoylthiorest $R_a$ enthält bis zu 7, insbesondere bis zu 4 Kohlenstoffatome und ist z.B. Formylthio, Acetylthio, Propionylthio oder Butyrylthio. Substituiertes Niederalkanoylthio $R_a$ oder $R_b$ is in erster Linie substituiertes Acetylthio, z.B. Hydroxyacetylthio, Methoxyacetylthio, Phenyloxyacetylthio, Halogenacetylthio, z.B. Chlor- oder Bromacetylthio, Cyanacetylthio oder gegebenenfalls geschütztes Glycylthio.

Ein Cycloalkanoylthiorest $R_a$ hat 4 bis 8 Kohlenstoffatome und ist z.B. Cyclopropylcarbonylthio, Cyclobutylcarbonylthio, Cyclopentylcarbonylthio oder Cyclohexylcarbonylthio oder ein entsprechender, z.B. in 2-Stellung, z.B. durch Hydroxy oder Amino, substituierter Rest.

Ein Cycloalkylniederalkanoylthiorest $R_a$ hat 5 bis 9 Kohlenstoffatome und ist z.B. Cyclopropylacetylthio, Cyclobutylacetylthio, Cyclohexylacetylthio oder Cyclohexylacetylthio oder Cyclohexylpropionylthio, oder ein entsprechender, z.B. in 2-Stellung, z.B. durch Hydroxy oder Amino, substituierter Rest.

Ein Benzoylthio- oder Phenylniederalkanoylthio-, z.B. Phenylacetylthiorest $R_a$ kann, vorzugsweise im aromatischen Rest, z.B. durch Niederalkyl, wie Methyl oder Aethyl, Niederalkoxy, wie Methoxy, Halogen, wie Fluor oder Chlor, Nitro, oder gegebenenfalls geschütztes Hydroxy oder Amino, substituiert sein. Im Phenylniederalkanoylthiorest kann gegebenenfalls substituiertes, z.B. geschütztes Hydroxy oder gegebenenfalls substituiertes, z.B. geschütztes Amino auch im aliphatischen Teil stehen, insbesondere in 2-Stellung.

$R_a$ als Halogen ist Jod oder insbesondere Fluor, Chlor oder Brom.

Ein gesättigter oder ungesättigter, gegebenenfalls substituierter aliphatischer, cycloaliphatischer, cycloaliphatisch-aliphatischer, aromatischer oder araliphatischer Kohlenwasserstoffrest $R_1$ mit bis zu 18, bevorzugt bis zu 10 Kohlenstoffatomen, ist insbesondere gegebenenfalls substituiertes Niederalkyl oder Niederalkenyl, gegebenenfalls funktionell abgewandeltes Carboxyl, oder gegebenenfalls substituiertes Cycloalkyl, Cycloalkenyl, Cycloalkylniederalkyl, Cycloalkylniederalkenyl, Cycloalkenylniederalkyl, Phenyl, Phenylniederalkyl, oder Phenylniederalkenyl.

Substituenten von solchen Resten und von gegebenenfalls substituierten Heterocyclyl- oder Heterocyclylniederalkylresten mit bis zu 10 Kohlenstoffatomen und bis zu 4 Ringheteroatomen aus der Gruppe

Stickstoff, Sauerstoff und/oder Schwefel sind z.B. gegebenenfalls funktionell abgewandelte, wie gegebenenfalls verätherte oder veresterte Hydroxy- oder Mercaptogruppen, z.B. Hydroxy, Niederalkoxy, z.B. Methoxy oder Aethoxy, Niederalkanoyloxy, z.B. Acetyloxy oder Propionyloxy, Halogen, z.B. Chlor oder Brom, oder Niederalkylmercapto, z.B. Methylthio, oder Heterocyclylmercapto dieser Heterocyclylrest ist gegebenenfalls substituiert, hat aromatische Eigenschaften oder ist partiell gesättigt; Substituenten sind u.a. Niederalkyl, insbesondere Methyl, Hydroxy-niederalkyl, z.B. Hydroxymethyl, Carboxyniederalkyl, z.B. Carboxymethyl oder 1- oder 2-Carboxyäthyl, gegebenenfalls N-substituiertes Aminoniederalkyl, wie Diniederalkylaminoniederalkyl, z.B. Dimethylaminoäthyl, in Salzform vorliegendes Sulfoniederalkyl, z.B. als Natriumsalz vorliegendes Sulfomethyl oder 1- oder 2-Sulfoäthyl, Cycloalkyl, z.B.

Cyclopentyl oder Cyclohexyl, Aryl, wie gegebenenfalls durch Halogen, z.B. Chlor, oder Nitro substituiertes Phenyl, Arylniederalkyl, z.B. Benzyl, oder funktionelle Gruppen, wie Halogen, z.B. Fluor, Chlor oder Brom, gegebenenfalls substituiertes Amino, wie gegebenenfalls durch Niederalkyl mono- oder disubstituiertes Amino, z.B. Amino, Methylamino oder Dimethylamino, Nitro, Hydroxy, Niederalkoxy, z.B. Methoxy oder Aethoxy, oder gegebenenfalls funktionell abgewandeltes Carboxyl, wie Carboxyl, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl, oder N,N-Dimethylcarbamoyl, oder Cyan, sowie Oxo oder Oxido, wobei einer oder mehrere solcher Substituenten, die in erster Linie mit Ringkohlenstoffatomen, aber auch, insbesondere Niederalkyl und Oxido, mit Ringstickstoffatomen verbunden sind, vorhanden sein können; solche heterocyclische Reste sind in erster Linie gegebenenfalls die obgenannten Substituenten enthaltende, monocyclische, fünfgliedrige diaza-, triaza-, tetraza-, thiaza-, thiadiaza-, thiatriaza-, oxaza- oder oxadiazacyclische Reste aromatischen Charakters oder entsprechende, gegebenenfalls substituierte, z.B. die obgenannten Substituenten enthaltende Reste mit ankondensiertem Benzolring, wie benzodiaza- oder benzooxazacyclische Reste, gegebenenfalls die obgenannten Substituenten, in erster Linie Oxido enthaltende, monocyclische, sechsgliedrige monoaza- oder diazacyclische Reste aromatischen Charakters oder entsprechende, partiell gesättigte, gegebenenfalls substituierte, z.B. die obgenannten Substituenten, in erster Linie Oxo, enthaltende Reste, oder gegebenenfalls substituierte, z.B. die obgenannten Substituenten enthaltende, bicyclische triaza- oder tetrazacyclische Reste aromatischen Charakters oder entsprechende partiell gesättigte, gegebenenfalls substituierte, z.B. die obgenannten Substituenten, in erster Linie Oxo, enthaltende Reste; Beispiele solcher Heterocyclylreste sind Imidazolyl, z.B. 2-Imidazolyl, gegebenenfalls durch Niederalkyl und/oder Phenyl substituiertes Triazolyl, z.B. 1,2,3 - Triazol - 4 - yl, 1 - Methyl - 1H - 1,2,3 - triazol - 4 - yl, 1H - 1,2,4 - Triazol - 3 - yl, 5 - Methyl - 1H - 1,2,4-triazol - 3 - yl, 3 - Methyl - 1 - phenyl - 1H - 1,2,4 - triazol - 5 - yl, 4,5 - Dimethyl - 4H - 1,2,4 - triazol-3 - yl oder 4 - Phenyl - 4H - 1,2,4 - triazol - 3 - yl, gegebenenfalls durch Niederalkyl, Phenyl oder Halogenphenyl substituiertes Tetrazolyl, z.B. 1H - Tetrazol - 5 - yl, 1 - Methyl - 1H - tetrazol - 5 - yl, 1 - Phenyl - 1H - tetrazol - 5 - yl, 1 - (4 - Chlorphenyl) - 1H - tetrazol - 5 - yl, 1 - Carboxymethyl-1H - tetrazol - 5 - yl, 1 - (2 - Dimethylaminoäthyl) - 1H - tetrazol - 5 - yl oder 1 - Natriumsulfomethyl-1H - tetrazol - 5 - yl, gegebenenfalls durch Niederalkyl oder Thienyl substituiertes Thiazolyl oder Isothiazolyl, z.B. 2 - Thiazolyl 4 - (2 - Thienyl) - 2 - thiazolyl, 4,5 - Dimethyl - 2 - thiazolyl, 3 - Isothiazolyl, 4 - Isothiazolyl oder 5 - Isothiazolyl, gegebenenfalls durch Niederalkyl substituiertes Thiadiazolyl z.B. 1,2,3 - Thiadiazol - 4 - yl, 1,2,3 - Thiadiazol - 5 - yl, 1,3,4 - Thiadiazol - 2 - yl, 2 - Methyl - 1,3,4 - thiadiazol - 5 - yl, 1,2,4 - Thiadiazol - 5 - yl oder 1,2,5 - Thiadiazol - 3 - yl, Thiatriazolyl, z.B. 1,2,3,4 - Thiatriazolyl - 5 - yl, gegebenenfalls durch Niederalkyl oder Phenyl substituiertes Oxazolyl oder Isoxazolyl, z.B. 5 - Oxazolyl, 4 - Methyl - 5 - oxazolyl, 2 - Oxazolyl, 4,5 - Diphenyl - 2 - oxazolyl oder 3 - Methyl - 5 - isoxazolyl, gegebenenfalls durch Niederalkyl, Phenyl, Nitrophenyl oder Thienyl substituiertes Oxadiazolyl, z.B. 1,2,4 - Oxadiazol - 5 - yl, 2 - Methyl - 1,3,4 - oxadiazol - 5 - yl, 2 - Phenyl - 1,3,4 - oxadiazol - 5 - yl, 5 - (4 - Nitrophenyl) - 1,3,4-oxadiazol - 2 - yl oder 2 - (Thienyl) - 1,3,4 - oxadiazol - 5 - yl, gegebenenfalls durch Halogen substituiertes Benzimidazolyl, z.B. 2 - Benzimidazolyl oder 5 - Chlor - 2 - benzimidazolyl, oder gegebenenfalls durch Halogen oder Nitro substituiertes Benzoxazolyl, z.B. 2 - Benzoxazolyl, 5 - Nitro - 2-benzoxazolyl oder 5 - Chlor - 2 - benzoxazolyl, 1 - Oxido - pyridyl, z.B. 1 - Oxido - 2 - pyridyl oder 4 - Chlor - 1 - oxido - 2 - pyridyl, gegebenenfalls durch Hydroxy substituiertes Pyridazinyl, z.B. 3 - Hydroxy - 6 - pyridazinyl, gegebenenfalls durch Niederalkyl, Niederalkoxy oder Halogen substituiertes N - Oxido - pyridazinyl, z.B. 2 - Oxido - 6 - pyridazinyl, 3 - Chlor - 1 - oxido-6 - pyridazinyl, 3 - Methyl - 2 - oxido - 6 - pyridazinyl, 3 - Methoxy - 1 - oxido - 6 - pyridazinyl, 3 - Aethoxy - 1 - oxido - 6 - pyridazinyl, 3 - n - Butyloxy - 1 - oxido - 6 - pyridazinyl oder 3 - (2 - Aethylhexyloxy) - 1 - oxido - 6 - pyridazinyl, oder gegebenenfalls durch Niederalkyl, Amino, Diniederalkylamino oder Carboxy substituiertes 2 - Oxo - 1,2 - dihydro - pyrimidinyl, z.B. 2 - Oxo - 1,2 - dihydro - 4 - pyrimidinyl, 6 - Methyl - 2 - oxo - 1,2 - dihydro - 4 - pyrimidinyl, 5-Methyl - 2 - oxo - 1,2 - dihydro - 4 - pyrimidinyl, 6 - Amino - 2 - oxo - 1,2 - dihydro - 4 - pyrimidinyl, 6 - Dimethylamino - 2 - oxo - 1,2 - dihydro - 4 - pyrimidinyl, 5 - Carboxy - 2 - oxo - 1,2 - dihydro-4 - pyrimidinyl oder 6 - Carboxy - 2 - oxo - 1,2 - dihydro - 4 - pyrimidinyl, Triazolopyridyl, z.B. s - Triazolo[4,3 - a]pyrid - 3 - yl oder 3H - v - Triazolo[4,5-b]pyrid - 5 - yl, oder gegebenenfalls durch Halogen und/oder Niederalkyl substituiertes Purinyl, z.B. 2 - Purinyl, 6 - Purinyl oder 8 - Chlor-2 - methyl - 6 - purinyl, ferner 2 - Oxo - 1,2 - dihydro - purinyl, z.B. 2 - Oxo - 1,2 - dihydro - 6 - purinyl] oder gegebenenfalls funktionell abgewandelte Carboxylgruppen, wie Carboxyl, Niederalkoxycarbonyl, z.B. Methoxycarbonyl oder Aethoxycarbonyl, Carbamoyl oder Cyan, ferner Nitro oder gegebenenfalls substituiertes, wie durch Niederalkyl, z.B. Methyl oder Aethyl, Acyl, wie Niederalkanoyl, z.B. Acetyl, mono- oder disubstituiertes, oder durch Niederalkylen, z.B. 1,4-Butylen oder 1,5 - Pentylen, disubstituiertes Amino.

Ein Niederalkylrest $R_1$ enthält bis zu 7, insbesondere bis zu 4 Kohlenstoffatome, und ist z.B. Methyl, Aethyl, Propyl, Isopropyl, Butyl, Isobutyl, tert.-Butyl oder Pentyl. Substituiertes Niederalkyl ist in erster Linie substituiertes Methyl, Aethyl oder Propyl, wobei Substituenten insbesondere in 1-, aber auch in 2- oder in 3-Stellung stehen, wie Hydroxymethyl, Hydroxyäthyl oder Hydroxypropyl, Niederalkoxymethyl, Niederalkoxyäthyl, oder Niederalkoxypropyl, z.B. Methoxymethyl, Methoxyäthyl oder Methoxypropyl, Niederalkanoyloxymethyl, Niederalkanoyloxyäthyl oder Niederalkanoyloxypropyl, z.B. Acetyloxymethyl, Propionyloxymethyl, Acetyloxyäthyl oder Acetyloxypropyl, Halogenmethyl, Halogenäthyl oder Halogenpropyl, z.B. Chlor- oder Bromäthyl, oder Chlor- oder Brompropyl, Methylthiomethyl, Methylthioäthyl, Methylthiopropyl, tert.-Butylthiomethyl, 1,2,3 - Triazol - 4 - ylthiomethyl, 1H - Tetrazol - 5 - ylthiomethyl, 1 - Methyl - 1H - tetrazol - 5 - ylthiomethyl, 1 - Carboxymethyl - 1H - tetrazol - 5 - ylthiomethyl, 1 - (2 - Dimethylaminoäthyl) - 1H - tetrazol - 5 - ylthiomethyl oder 1 - Natriumsulfomethyl - 1H - tetrazol - 5 - ylthiomethyl, 1H - Tetrazol - 5 - ylthioäthyl, (1 - Methyl - 1H - tetrazol - 5 - ylthio) - äthyl, 2 - Methyl - 1,3,4 - thiadiazol - 5 - ylthiomethyl, Niederalkoxycarbonylmethyl oder Niederalkoxycarbonyläthyl, z.B. Methoxycarbonylmethyl, Aethoxycarbonylmethyl oder Methoxycarbonyläthyl, Cyanmethyl, Cyanäthyl, oder gegebenenfalls geschütztes Aminomethyl, Aminoäthyl oder Aminopropyl.

Ein Niederalkenylrest $R_1$ enthält 2 bis 7, insbesondere 2 bis 4 Kohlenstoffatome, und ist z.B. Vinyl, Allyl oder 2- oder 3-Butenyl. Substituiertes Niederalkenyl kann die gleichen Substituenten tragen wie substituiertes Niederalkyl und ist beispielsweise 2-Aminovinyl oder 2-Acylamino-, wie 2-Acetylaminovinyl.

Eine gegebenenfalls funktionell abgewandelte Carboxylgruppe $R_1$ ist eine freie oder eine der unter den Gruppen $-C(=O)-R_2^A$ genannten, beispielsweise veresterten oder amidierten Carboxylgruppen, wie Niederalkoxycarbonyl, z.B. Methoxy-, Aethoxy- oder tert.-Butoxycarbonyl, Arylniederalkoxycarbonyl, wie Benzyloxy-, p-Nitrobenzyl- oder Diphenylmethoxycarbonyl, Aryloxycarbonyl, wie gegebenenfalls, z.B. durch Halogen, wie Chlor, Niederalkoxy, wie Methoxy, oder Nitro, substituiertes Phenyloxycarbonyl, wie Phenyloxycarbonyl, o-, m- oder p-Chlorphenyloxy-, Pentachlorphenyloxy-, o-, m- oder p-Methoxyphenyloxy- oder p-Nitrophenyloxycarbonyl, Aminocarbonyl oder substituiertes, wie durch Niederalkyl, z.B. Methyl oder Aethyl, mono- oder disubstituiertes Aminocarbonyl.

Ein Cycloalkylrest $R_1$ weist z.B. 3 bis 7 Kohlenstoffatome auf und ist z.B. Cyclopropyl, Cyclobutyl, Cyclopentyl oder Cyclohexyl, während ein Cycloalkylniederalkylrest $R_1$ beispielsweise 4 bis 7 Kohlenstoffatome enthält und z.B. Cyclopropylmethyl, Cyclobutylmethyl, Cyclopentylmethyl oder Cyclohexylmethyl darstellt.

Ein Cycloalkenylrest $R_1$ ist ein entsprechender Cycloalkylrest mit ein oder gegebenenfalls zwei C—C-Doppelbindungen, wie Cyclohexenyl, z.B. 1-Cyclohexenyl, oder Cyclohexadienyl, z.B. 1,4-Cyclohexadienyl.

Ein Cycloalkylniederalkenylrest oder Cycloalkenylniederalkylrest $R_1$ ist z.B. Cyclohexylvinyl, Cyclohexylallyl, bzw. Cyclohexenylmethyl oder 1,4-Cyclohexadienylmethyl.

Ein Phenyl- oder ein Phenylniederalkyl-, z.B. Benzyl- oder 1- oder 2-Phenyläthylrest $R_1$ kann, vorzugsweise im aromatischen Rest, z.B. durch Niederalkyl, wie Methyl oder Aethyl, Niederalkoxy, wie Methoxy, oder Halogen, wie Fluor oder Chlor, ferner durch Nitro oder Amino substituiert sein.

Ein Rest $R_1$ kann auch einen, über ein Kohlenstoffatom gebundenen heterocyclischen oder heterocyclisch-aliphatischen Rest, vorzugsweise aromatischen Charakters darstellen, wie Pyridyl, z.B. 2-, 3- oder 4-Pyridyl, Thienyl, z.B. 2-Thienyl, oder Furyl, z.B. 2-Furyl, oder entsprechende Pyridyl-, Thienyl- oder Furylniederalkyl-, insbesondere -methylreste, darstellen.

Ein Phenyl- oder Heterocyclylniederalkenylrest $R_1$ ist ein wie ein entsprechender Niederalkylrest substituierter Niederalkenylrest, z.B. Phenylvinyl oder Furylallyl.

Durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen verätherte Mercaptogruppen $R_1$ sind gegebenenfalls substituiertes Niederalkylthio, Niederalkenylthio, Cycloalkylthio, Cycloalkylniederalkylthio, Phenylthio oder Phenylniederalkylthio. Substituenten von solchen Resten sind z.B. gegebenenfalls funktionell abgewandeltes, wie gegebenenfalls veräthertes oder verestertes Hydroxy oder Mercapto, z.B. Hydroxy, Niederalkoxy, z.B. Methoxy oder Aethoxy, Niederalkanoyloxy, z.B. Acetyloxy oder Propionyloxy, Halogen, z.B. Chlor oder Brom, oder Niederalkylmercapto, z.B. Methylthio, oder gegebenenfalls funktionell abgewandelte Carboxylgruppen, wie Carboxy, Niederalkoxycarbonyl, z.B. Methoxycarbonyl oder Aethoxycarbonyl, Carbamoyl oder Cyan, ferner Nitro oder gegebenenfalls, wie durch Niederalkyl, z.B. Methyl oder Aethyl, durch Acyl, wie Niederalkanoyl, z.B. Acetyl, mono- oder disubstituiertes, oder durch Niederalkylen, z.B. 1,4-Butylen oder 1,5-Pentylen disubstituiertes Amino, oder die unten bei den einzelnen verätherten Mercaptogruppen $R_1$ aufgeführten Substituenten.

Ein Niederalkylthiorest $R_1$ enthält bis zu 7, insbesondere bis zu 4 Kohlenstoffatome, und ist z.B. Methylthio, Aethylthio, Propylthio, Isopropylthio, Butylthio, Isobutylthio, tert.-Butylthio oder Pentylthio. Substituiertes Niederalkylthio $R_1$ ist in erster Linie substituiertes Methylthio, Aethylthio oder Propylthio, wobei Substituenten in 1-, 2- oder 3-Stellung stehen, wie Methoxymethylthio, Aethoxymethylthio, Methoxyäthylthio, oder Methoxypropylthio, Niederalkanoyloxymethylthio, Niederalkanoyloxyäthylthio oder Niederalkanoyloxymethylthio, wie Acetoxymethylthio, Acetoxyäthylthio oder Acetoxypropylthio, Halogenmethylthio, Halogenäthylthio oder Halogenpropylthio, z.B. Chlor- oder Bromäthylthio oder Chlor- oder Brompropylthio, Niederalkoxycarbonylmethylthio oder Niederalkoxycarbonyläthylthio, z.B. Methodcarbonyläthylthio, Cyanmethylthio, Cyanäthylthio oder gegebenenfalls geschütztes, z.B. acetyliertes, Aminomethylthio, Aminoäthylthio oder Aminopropylthio.

Ein Niederalkenylthiorest $R_1$ enthält 2 bis 7, insbesondere 2 bis 4 Kohlenstoffatome und ist insbesondere 1-Niederalkenylthio, z.B. Vinylthio, 1-Propenylthio, 1-Butenylthio oder 1-Pentenylthio oder auch 2-Niederalkenylthio, z.B. Allylthio. Substituiertes Niederalkenylthio $R_1$ ist in erster Linie in 2-Stellung substituiert, wobei als Substituenten vor allem Niederalkoxy, Niederalkanoyloxy und gegebenenfalls geschütztes Amino in Frage kommen, und ist beispielsweise 2-Methoxyvinylthio, 2-Acetoxyvinylthio, 2-Acetylaminovinylthio oder entsprechend substituiertes 1-Propenylthio.

Eine Cycloalkylthiogruppe $R_1$ weist z.B. 3 bis 7 Kohlenstoffatome auf und ist z.B. Cyclopropylthio, Cyclobutylthio, Cyclopentylthio oder Cyclohexylthio.

Ein Cycloalkylniederalkylthiorest $R_1$ hat z.B. 4 bis 7 Kohlenstoffatome und ist z.B. Cyclopropylmethylthio, Cyclobutylmethylthio, Cyclopentylmethylthio oder Cyclohexylmethylthio.

Ein Phenylthio- oder ein Phenylniederalkylthio-, z.B. Benzyl- oder 1- oder 2-Phenyläthylthiorest $R_1$ kann, vorzugsweise im aromatischen Rest, z.B. durch Niederalkyl, wie Methyl oder Aethyl, Niederalkoxy, wie Methoxy, Halogen, wie Fluor oder Chlor, Nitro oder Amino substituiert sein.

Durch einen gegebenenfalls substituierten, über ein Ringkohlenstoffatomen an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel verätherte Mercaptogruppen $R_1$ sind in erster Linie gegebenenfalls substituierte, z.B. die unten genannten Substituenten enthaltende, monocyclische, fünfgliedrige diaza-, triaza-, tetraza-, thiaza-, thiadiaza-, thiatriaza-, oxaza- oder oxadiazacyclische Reste aromatischen Charakters, oder gegebenenfalls substituierte monocyclische sechsgliedrige aza- oder diazacyclische Reste aromatischen oder partiell gesättigten Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen $R_1$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H - 1,2,3 - Triazol - 4 - ylthio, 1 - Methyl - 1H - 1,2,3 - triazol - 4 - ylthio, 1H - 1,2,4 - Triazol - 3 - ylthio, 5 - Methyl 1H - 1,2,4 - triazol - 3 - ylthio, 3 - Methyl - 1 - phenyl - 1H - 1,2,4 - triazol - 5 - ylthio, 4,5 - Dimethyl - 4H - 1,2,4 - triazol - 3 - ylthio oder 4 - Phenyl - 4H - 1,2,4 - triazol - 3 - ylthio, insbesondere gegebenenfalls wie angegeben substituiertes Tetrazolylthio, z.B. 1H - Tetrazol - 5 - ylthio, 1 - Methyl - 1H - tetrazol - 5 - ylthio, 1 - Carboxymethyl - 1H - tetrazol - 5 - ylthio, 1 - (2 - Carboxyäthyl) - 1H - tetrazol - 5 - ylthio, 1 - Sulfomethyl - 1H - tetrazol - 5 - ylthio, 1 - (2 - Sulfoäthyl) - 1H - tetrazol - 5 - ylthio, 1 - (2 - Dimethylaminoäthyl) - 1H - tetrazol - 5 - ylthio, 1 - Phenyl - 1H-tetrazol - 5 - ylthio oder 1 - (4 - Chlorphenyl) - 1H - tetrazol - 5 - ylthio, gegebenenfalls durch Niederalkyl oder Thienyl substituiertes Thiazolylthio oder Isothiazolylthio, z.B. 2 - Thiazolylthio, 4 - (2 - Thienyl) - 2 - thiazolylthio, 4,5 - Dimethyl - 2 - thiazolylthio, 3 - Isothiazolylthio, 4 - Isothiazolylthio oder 5-Isothiazolylthio, insbesondere auch gegebenenfalls wie angegeben substituiertes Thiadiazolylthio, z.B. 1,2,3 - Thiadiazol - 4 - ylthio, 1,2,3 - Thiadiazol - 5 - ylthio, 1,3,4-Thiadiazol - 2 - ylthio, 2 - Methyl - 1,3,4 - thiadiazol - 5 - ylthio, 2 - (3 - Carboxypropionylamino) - 1,3,4 - thiadiazol - 5 - ylthio, 1,2,4 - Thiadiazol - 5 - ylthio oder 1,2,5 - Thiadiazol - 3 - ylthio, Thiatriazolylthio, z.B. 1,2,3,4-Thiatriazolyl - 5 - ylthio, gegebenenfalls wie angegeben substituiertes Oxazolylthio oder Isoxazolylthio, z.B. 5-Oxazolylthio, 4 - Methyl - 5 - oxazolylthio, 2 - Oxazolylthio, 4,5 - Diphenyl - 2 - oxazolylthio oder 3-Methyl - 5 - isoxazolylthio, oder gegebenenfalls wie angegeben substituiertes Oxadiazolylthio, z.B. 1,2,4 - Oxadiazol - 5 - ylthio, 2 - Methyl - 1,3,4 - oxadiazol - 5 - ylthio, 2 - Phenyl - 1,3,4-oxadiazol - 5 - ylthio, 5 - (4 - Nitrophenyl) - 1,3,4 - oxadiazol - 2 - ylthio oder 2 - (2 - Thienyl)-1,3,4 - oxadiazol - 5 - ylthio.

Bevorzugte heterocyclisch verätherte Mercaptogruppen $R_1$, worin der heterocyclische Rest einen entsprechenden monocyclischen, sechsgliedrigen Rest oder einen entsprechenden partiel gesättigten Rest darstellt, sind u.a. gegebenenfalls durch Halogen substituiertes 1-Oxidopyridylthio, z.B. 1 - Oxido - 2 - pyridylthio oder 4 - Chlor - 1 - oxido - 2 - pyridylthio, gegebenenfalls durch Hydroxy substituiertes Pyridazinylthio, z.B. 3 - Hydroxy - 6 - pyridazinylthio, gegebenenfalls durch Niederalkyl, Niederalkoxy oder Halogen substituiertes N - Oxido - pyridazinylthio, z.B. 2 - Oxido-6 - pyridazinylthio, 3 - Chlor - 1 - oxido - 6 - pyridazinylthio, 3 - Methyl - 2 - oxido - 6 - pyridazinylthio, 3 - Methoxy - 1 - oxido - 6 - pyridazinylthio, 3 - Aethoxy - 1 - oxido - 6 - pyridazinylthio, 3 - n-Butyloxy - 1 - oxido - 6 - pyridazinylthio oder 3 - (2 - Aethylhexyloxy) - 1 - oxido - 6 - pyridazinylthio, oder gegebenenfalls durch Niederalkyl, Amino, Diniederalkylamino oder Carboxy substituiertes 2-Oxo - 1,2 - dihydro - pyrimidinylthio, z.B. 2 - Oxo - 1,2 - dihydro - 4 - pyrimidinylthio, 6 - Methyl-2 - oxo - 1,2 - dihydro - 4 - pyrimidinylthio, 5 - Methyl - 2 - oxo - 1,2 - dihydro - 4 - pyrimidinylthio, 6 - Amino - 2 - oxo - 1,2 - dihydro - 4 - pyrimidinylthio, 6 - Dimethylamino - 2 - oxo - 1,2-dihydro - 4 - pyrimidinylthio, 5 - Carboxy - 2 - oxo - 1,2 - dihydro - 4 - pyrimidinylthio oder 6 - Carboxy - 2 - oxo - 1,2 - dihydro - 4 - pyrimidinylthio.

Eine geschützte Carboxylgruppe der Formel —C(=O)—$R_2^A$ ist in erster Linie eine veresterte Carboxylgruppe, worin $R_2^A$ eine durch einen organischen Rest oder eine organische Silyl- oder Stannylgruppe verätherte Hydroxygruppe darstellt. Organische Reste, auch als Substituenten in organischen Silyl- oder Stannylgruppen, sind aliphatische, cycloaliphatische, cycloaliphatisch-aliphatische, aromatische oder araliphatische Reste, insbesondere gegebenenfalls substituierte Kohlenwasserstoffreste dieser Art, sowie heterocyclische oder heterocyclischaliphatische Reste, vorzugsweise mit bis zu 18 Kohlenstoffatomen.

Eine verätherte Hydroxygruppe $R_2^A$ bildet zusammen mit der Carbonylgruppierung eine, vorzugsweise leicht spaltbare, z.B. reduktiv, wie hydrogenolytisch, oder solvolytisch, wie acidolytisch oder insbesondere basisch oder neutral hydrolytisch, sowie oxidativ oder unter physiologischen Bedingungen spaltbare, oder leicht in eine andere funktionell abgewandelte Carboxylgruppe, wie in eine andere veresterte Carboxylgruppe oder in eine Hydrazinocarbonylgruppe umwandelbare, veresterte Carboxylgruppe. Eine solche Gruppe $R_2^A$ ist z.B. 2-Halogen-niederalkoxy, worin Halogen vorzugsweise ein Atomgewicht von über 19 hat, z.B. 2,2,2-Trichloräthoxy oder 2-Jodäthoxy, ferner 2-Chloräthoxy oder 2-Bromäthoxy, das sich leicht in letzteres überführen lässt, oder 2-Niederalkylsulfonylniederalkoxy, z.B. 2-Methylsulfonyläthoxy. Die Gruppe $R_2^A$ ist ferner eine durch gegebenenfalls substituierte Kohlenwasserstoffreste, insbesondere gesättigte aliphatische oder aromatische Kohlenwasserstoffreste, wie Niederalkyl, z.B. Methyl und/oder Phenyl, polysubstituierte Methoxygruppe oder eine durch einen ungesättigten aliphatischen Kohlenwasserstoffrest, wie Niederalkenyl, z.B. 1-Niederalkenyl, wie Vinyl, durch eine Elektronen-abgebende Substituenten aufweisende carbocyclische Arylgruppe oder durch eine Sauerstoff oder Schwefel als Ringglied aufweisende heterocyclische Gruppe aromatischen Charakters monosubstituierte Methoxygruppe, wie tert.-Niederalkoxy, z.B. tert.-Butyloxy oder tert.-Pentyloxy, gegebenenfalls substituiertes Diphenylmethoxy, z.B. Diphenylmethoxy oder 4,4'-Dimethoxydiphenylmethoxy, Niederalkenyloxy, insbesondere 2-Niederalkenyloxy, z.B. Allyloxy, Niederalkoxy-phenylniederalkoxy, z.B. Niederalkoxy-benzyloxy, wie Methoxybenzyloxy (wobei Methoxy in erster Linie in 3-, 4- und/oder 5-Stellung steht), in erster Linie 3- oder 4-Methoxybenzyloxy, 3,4-Dimethoxybenzyloxy, oder vor allem Nitrobenzyloxy, z.B. 4-Nitrobenzyloxy, 2-Nitrobenzyloxy oder 4,5-Dimethoxy-2-nitro-benzyloxy, bzw. Furfuryloxy, wie 2-Furfuryloxy. Die Gruppe $R_2^A$ ist ferner eine 2-Oxoäthoxygruppe, die in 2-Stellung gegebenenfalls durch Niederalkyl, wie Methyl, Niederalkoxy, wie Methoxy oder Aethoxy, durch Aralkyl, wie Benzyl, oder durch Aryl, wie Phenyl, und in 1-Stellung durch Niederalkyl, wie Methyl, Niederalkoxycarbonyl, wie Methoxycarbonyl, Niederalkylcarbonyl, wie Methylcarbonyl, Aralkylcarbonyl, wie Benzylcarbonyl oder Arylcarbonyl, wie Benzoyl, substituiert ist, und stellt beispielsweise Acetonyloxy, Phenacyloxy, 2,4-Dioxo-3-pentyloxy, 1-Methoxycarbonyl-2-oxo-propyloxy oder 1-Aethoxycarbonyl-2-oxo-propyloxy dar. Die Gruppe $R_2^A$ ist auch eine 2-Cyanäthoxygruppe, die gegebenenfalls in 1- und/oder in 2-Stellung beispielsweise durch Niederalkyl, wie Methyl, oder Aryl, wie gegebenenfalls substituiertes Phenyl, substituiert ist, und stellt beispielsweise 2-Cyanäthoxy oder 2-Cyan-2-phenyläthoxy dar. $R_2^A$ ist auch eine 2-$(S_1)(S_2)(S_3)$-Silyläthoxygruppe, worin die Substituenten $S_1$, $S_2$ und $S_3$ unabhängig voneinander je einen gegebenenfalls substituierten Kohlenwasserstoffrest bedeuten und die einzelnen Reste durch eine einfache C—C-Bindung verknüpft sein können. Ein Kohlenwasserstoffrest $S_1$, $S_2$ und $S_3$ ist bei-

spielsweise ein Alkyl-, Cycloalkyl- oder Arylrest, vorzugsweise ein solcher mit höchstens 12 Kohlenstoffatomen, wobei der Rest einer Art durch einen Rest einer anderen Art oder durch Niederalkoxy, wie Methoxy, oder Halogen, wie Fluor oder Chlor, substituiert sein kann, und ist insbesondere Niederalkyl mit bis zu 7, bevorzugt bis zu 4 Kohlenstoffatomen, wie Methyl, Aethyl, Propyl oder Butyl, Cycloalkyl mit bis zu 7 Kohlenstoffatomen, wie Cyclopropyl oder Cyclohexyl, Cycloalkylalkyl, wie Cyclopentylmethyl, Aryl mit bis zu 10 Kohlenstoffatomen, wie Phenyl, Tolyl oder Xylyl, Arylniederalkyl, wie Benzyl oder Phenyläthyl. Hervorzuhebende Reste $R_2^A$ dieser Art sind 2-Triniederalkylsilyläthoxy, wie 2-Trimethylsilyläthoxy oder 2-(Dibutyl-methyl-silyl)-äthoxy, sowie 2-Triarylsilyläthoxy-, wie 2-Triphenylsilyläthoxy.

$R_2^A$ kann auch 2-Oxa- oder 2-Thia-cycloalkoxy oder -cycloalkenyloxy mit 5—7 Ringgliedern, wie 2-Tetrahydrofuryloxy, 2-Tetrahydropyranyloxy oder 2,3-Dihydro-2-pyranyloxy oder eine entsprechende Thiagruppe sein oder $R_2^A$ bildet zusammen mit der —C(=O)-Gruppierung eine aktivierte Estergruppe und ist beispielsweise Nitrophenyloxy, z.B. 4-Nitrophenyloxy oder 2,4-Dinitrophenyloxy, oder Polyhalogenphenyloxy, z.B. Pentachlorphenyloxy. $R_2^A$ kann aber auch Niederalkoxy, z.B. Methoxy oder Aethoxy, sein.

Eine organische Silyloxy- oder organische Stannyloxygruppe $R_2^A$ ist insbesondere eine durch 1 bis 3 gebenenfalls substituierte Kohlenwasserstoffreste, vorzugsweise mit bis zu 18 Kohlenstoffatomen, substituierte Silyloxy- oder Stannyloxygruppe. Sie enthält als Substituenten vorzugsweise gegebenenfalls substituierte, beispielsweise durch Niederalkoxy, wie Methoxy, oder durch Halogen, wie Chlor, substituierte aliphatische, cycloaliphatische, aromatische oder araliphatische Kohlenwasserstoffreste, wie Niederalkyl, Halogen-niederalkyl, Cycloalkyl, Phenyl oder Phenylniederalkyl, und stellt in erster Linie Triniederalkylsilyloxy, z.B. Trimethylsilyloxy, Halogen-niederalkoxy-niederalkylsilyloxy, z.B. Chlormethoxy-methyl-silyloxy, oder Triniederalkylstannyloxy, z.B. Trin-n-butylstannyloxy, dar.

Die Gruppe $R_2^A$ kann auch eine verätherte Hydroxygruppe sein, die zusammen mit der Carbonylgruppierung —C(=O)— eine unter physiologischen Bedingungen spaltbare veresterte Carboxylgruppe bildet.

Diese Estergruppen verleihen den · eingentlich wirksamen Carbonsäuren eine verbesserte Absorption bei oraler Applikation und/oder eine verlängerte Wirksamkeit. Zahlreiche solcher Estergruppen sind auf dem Gebiet der Penicilline und Cephalosporine bekannt. Genannt seien beispielsweise —C(=O)—$R_2^A$-Gruppen, worin $R_2^A$ eine durch Acyl, Acyloxy, Acylthio, Acylamino, oder veräthertes Hydroxy und gegebenenfalls einen weiteren organischen Rest substituierte Methyloxygruppe, wobei die Methylgruppe mit dem Carbonyl der Acylgruppe auch über eine Kohlenstoff enthaltende Brücke verbunden sein kann, oder eine 2-Aminoaliphatyloxygruppe darstellt. In solchen Gruppen stellt Acyl den Rest einer organischen Carbonsäure mit etwa bis zu 18 C-Atomen dar und ist beispielsweise gegebenenfalls substituiertes Alkanoyl, Cycloalkanoyl, Aroyl, Heterocyclylcarbonyl, z.B. auch der Heterocyclylcarbonylrest einer Carbonsäure der Formel I, oder einer biologisch aktiven Penam-3- oder Cephem-4-carbonsäure, oder der Acylrest eines Halbesters der Kohlensäure. Veräthertes Hydroxy in der Methoxygruppe ist durch einen Kohlenwasserstoffrest, insbesondere durch Niederalkyl veräthert. Der gegebenenfalls die Methyloxygruppe zusätzlich substituierende organische Rest hat bis zu 7-C-Atome und ist insbesondere Niederalkyl, wie Methyl, oder Aryl, wie Phenyl. Die genannte Kohlenstoffbrücke enthält ein bis drei, insbesondere 2 C-Atome, so dass ein Lacton, insbesondere ein $\gamma$-Lacton vorliegt. Die Aliphatylgruppe in der genannten 2-Aminoaliphatylgruppe kann aliphatischer oder cycloaliphatischer Natur sein und ist gesättigt oder ungesättigt. Die 2-Aminogruppe ist bevorzugt durch zwei Niederalkylgruppen oder gegebenenfalls eine Oxagruppe enthaltendes Alkylen substituiert. In solchen physiologisch spaltbaren Estergruppen —C(=O)—$R_2^A$ ist $R_2^A$ beispielsweise Niederalkanoyloxy-methoxy, z.B. Acetyloxymethoxy oder Pivaloyloxymethoxy, Aminoniederalkanoyloxymethoxy, insbesondere $\alpha$-Aminoniederalkanoyloxymethoxy, z.B. Glycyloxymethoxy, L-Valyloxymethoxy, L-Leucyloxymethoxy, Niederalkoxycarbonyloxymethoxy oder 1-Niederalkoxycarbonyloxyäthoxy, z.B. 1-Aethyloxycarbonyloxyäthoxy, Niederalkanoylthiomethoxy, z.B. Acetylthiomethoxy oder Pivaloylthiomethoxy, Niederalkanoylaminomethoxy, worin Niederalkanoyl gegebenenfalls durch Halogen, wie Chlor, substituiert sein kann, z.B. Acetylaminomethoxy oder 2,2-Dichloracetylaminomethoxy, Aroylaminomethoxy, z.B. Benzoylaminomethoxy, oder, als Beispiel für ein Lacton enthaltendes $R_2^A$, Phthalidyloxy. Die verätherte Hydroxymethoxygruppe $R_2^A$ ist beispielsweise Niederalkoxymethoxy, insbesondere Methoxymethoxy. Eine 2-Aminoaliphatyloxygruppe $R_2^A$ ist beispielsweise 2-Aminoniederalkoxy, wie eine 2-Aminoäthyloxygruppe, worin Amino durch zwei Niederalkyl oder gegebenenfalls eine Oxagruppe enthaltendes Alkylen substituiert ist, z.B. 2-Dimethylaminoäthoxy, 2-Diäthylaminoäthoxy oder 2-(1-Morpholino)-äthoxy, oder 2-Aminocycloalkyloxy, z.B. 2-Dimethylaminocyclohexyloxy.

Eine zusammen mit einer —C(=O)-Gruppierung eine gegebenenfalls substituierte Hydrazinocarbonylgruppe bildender Rest $R_2^A$ ist z.B. Hydrazino oder 2-Niederalkylhydrazino, z.B. 2-Methylhydrazino.

Bevorzugte Gruppen $R_2^A$ sind solche, die unter neutralen, basischen oder auch unter physiologischen Bedingungen in eine freie Hydroxygruppe übergeführt werden können.

Salze sind insbesondere diejenigen von Verbindungen der Formel I mit einer sauren Gruppierung, wie einer Carboxygruppe, oder auch einer Hydroxysulfonyloxy- oder Sulfogruppe, in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in

erster Linie aliphatische, cycloaliphatische, cycloaliphatisch-aliphatische und araliphatische primäre, sekundäre oder tertiäre Mono, Di- oder Polyamine, sowie heterocyclische Basen für die Salzbildung in Frage kommen, wie Niederalkylamine, z.B. Triäthylamin, Hydroxy-niederalkylamine, z.B. 2-Hydroxy äthylamin, Di-(2-hydroxyäthyl)amin oder Tri-(2-hydroxyäthyl)-amin, basische aliphatische Ester von Carbonsäuren, z.B. 4-Aminobenzosäure-2-diäthylamino-äthylester, Niederalkylenamine, z.B. 1-Aethylpiperidin, Cycloalkylamine, z.B. Bicyclohexylamin, oder Benzylamine, z.B. N,N'-Dibenzyläthylendiamin, ferner Basen vom Pyridintyp, z.B. Pyridin, Collidin oder Chinolin. Verbindungen der Formel I, die eine basische Gruppe aufweisen, können ebenfalls Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure oder p-Toluolsulfonsäure, bilden. Verbindungen der Formel I mit einer sauren und einer basischen Gruppe können auch in Form von inneren Salzen, d.h. in zwitterionischer Form, vorliegen. Bevorzugt sind pharmazeutisch verwendbare Salze.

In den Penemverbindungen der Formel I können die beiden asymmetrischen Kohlenstoffatome in 5- und 6-Stellung in der R-, der S-oder der racemischen R,S-Konfiguration vorliegen. Bevorzugt sind die Verbindungen, in dennen die Konfiguration des 5-Kohlenstoffatoms derjenigen des natürlichen Penicillins entspricht (5R-Konfiguration). Die Substituenten in 5- und 6-Stellung können cis oder trans zueinander stehen.

Die Verbindungen der vorliegenden Erfindung weisen wertvolle pharmakologische Eigenschaften auf oder können als Zwischenprodukte zur Herstellung von solchen verwendet werden. Verbindungen der Formel I, worin $R_a$, und $R_1$ die oben gegebenen Bedeutungen haben, und $R_2$ Hydroxy oder eine zusammen mit der Carbonylgruppe eine vorzugsweise unter physiologischen Bedingungen leicht spaltbare, veresterte Carboxylgruppe bildende verätherte Hydroxygruppe $R_2^A$ bedeutet, oder pharmakologisch verwendbaren Salze von solchen Verbindungen mit salzbildenden Gruppen haben antibakterielle Wirkungen. Sie hemmen beispielsweise das Wachstum von gram-positiven und gramnegativen Keimen, wie *Staphylococcus aureus* und *Penicillin-resistentem Staphylococcus aureus, Escherichia coli, Proteus vulgaris, Pseudomonas aeruginosa* und *Pseudomonas aeruginosa R.* Im Dics-Plattentest werden mit erfindungsgemässen Verbindungen der Formel I bei den genannten Keimen mit einer 0,5%-igen Lösung auf Filterpapier (6 mm Durchmesser) Hemmzonen von etwa 12 bis 33 mm Durchmesser festgestellt.

Gleichzeitig analog getestetes Penicillin V verursacht bei Penicillin-empfindlichen *Staphylococcus aureus* Keimen Hemmzonen von 29 bis 33 mm Durchmesser und bei Penicillin-resistenten Keimen von höchstens 9 bis 12 mm. Gegen *Pseudomonas aeruginosa* ist weder Penicillin V noch Penicillin G wirksam.

Die antibakterielle Wirkungs *in vitro* kann auch im Agar-Verdünnungs-Test (nach Ericsson) festgestellt werden, wobei gegen grampositive und gramnegative Kokken MIC-Werte von 0,06 bis 8 $\mu$g/ml, und gegen gramnegative Stäbchenbakterien, wie Enterobakterien, Pseudomonas und Haemophilus, von 2 bis 128 $\mu$g/ml ermittelt werden.

*In vivo*, bei der systemischen Infektion an der Maus durch *Streptococcus pyogenes Aronson*, ergeben sich bei subcutaner Application erfindungsgemässer Verbindungen $ED_{50}$-Werte von etwa $\leq 1$ bis etwa 50 mg/kg.

Hervorzuheben ist insbesondere die Wirksamkeit gegen *Pseudomonas aeruginosa.*

Die Verbindungen hemmen $\beta$-Lactamasen und wirken synergistisch in Kombination mit anderen $\beta$-Lactamantibiotika.

Diese neuen Verbindungen, insbesondere die bevorzugten, oder ihre pharmakologisch verwendbaren Salze, können deshalb, allein oder in Kombination mit anderen Antimicrobica z.B. in Form von antibiotisch wirksamen Präparaten, zur Behandlung von entsprechenden System- oder Organinfektionen, ferner als Futtermittelzusätze, zur Konservierung von Nahrungsmitteln oder als Desinfektionsmittel Verwendung finden.

Verbindungen der Formel I, worin $R_a$ und $R_1$ die oben gegebenen Bedeutungen haben, worin gegebenenfalls vorhandene funktionelle Gruppen in geschützer Form vorliegen können, und worin $R_2$ für einen, zusammen mit der —C(=O)-Gruppierung eine vorzugsweise leicht spaltbare, geschützte Carboxylgruppe bildenden Rest $R_2^A$ darstellt, wobei eine so geschützte Carboxylgruppe von einer physiologisch spaltbaren Carboxylgruppe verschieden ist, sind wertvolle Zwischenprodukte, die in einfacher Weise, z.B. wie unten beschrieben wird, in die obgenannten, pharmakologisch wirksamen Verbindungen übergeführt werden können.

Die Erfindung betrifft insbesondere die 2-Penem-Verbindungen der Formel I, worin $R_a$ Niederalkyl, Hydroxyniederalkyl, Niederalkoxyniederalkyl, Niederalkanoyloxyniederalkyl, in Salzform vorliegendes Hydroxysulfonyloxyniederalkyl, Hydroxy, Niederalkoxy, Niederalkanoyloxy, Niederalkanoyloxy substituiert durch Phenyloxy, Halogen, Amino oder Cyan, Phenylniederalkanoyloxy, oder durch Hydroxy oder Amino substituiertes Phenylniederalkanoyloxy ist, $R_1$ Wasserstoff, Niederalkyl, Hydroxyniederalkyl, Niederalkoxyniederalkyl, Niederalkanoyloxyniederalkyl, Niederalkylthioniederalkyl, Heterocyclylthioniederalkyl, worin Heterocyclyl einen gegebenenfalls durch Niederalkyl, Carboxyniederalkyl, Diniederalkylaminoniederalkyl oder in Salzform vorliegendes Sulfoniederalkyl substituierten fünfgliedrigen aromatischen diaza-, triaza-, tetraza-, thiaza-, thiadiaza-, thiatriaza-, oxaza- oder oxadiaza-cyclischen Rest darstellt, Aminoniederalkyl, Acylaminoniederalkyl, worin Acyl Niederalkanoyl oder eine als

Aminoschutzgruppe gebräuchliche substituierte Oxycarbonylgruppe, z.B. eine tert.-Butyl-, 2,2,2-Trichloräthyl-, Diphenylmethyl- oder p-Nitrobenzyl-oxycarbonylgruppe, ist, Carboxyniederalkyl, Niederalkoxycarbonylniederalkyl, Phenylniederalkyl, Phenyl, Hydroxyphenyl, Aminophenyl, Furyl, Thienyl, Pyridyl, Niederalkylthio, durch Amino, Mono- oder Diniederalkylamino oder Niederalkanoylamino substituiertes Niederalkylthio oder Niederalkenylthio, oder gegebenenfalls durch Niederalkyl, Sulfoniederalkyl, Carboxyniederalkyl oder Diniederalkylaminoniederalkyl substituiertes Tetrazolylthio oder Thiadiazolylthio darstellt, und $R_2$ für Hydroxy, gegebenenfalls $\alpha$-polyverzweigtes Niederalkoxy, 2-Halogenniederalkoxy, Phenacyloxy, 1-Phenylniederalkoxy mit 1—3, gegebenenfalls durch Niederalkoxy und/oder Nitro substituierten Phenylresten, Acetonyloxy, 2-Cyanäthoxy, 2-Triniederalkylsilyläthoxy, Niederalkanoyloxymethoxy, $\alpha$-Aminoniederalkanoyloxymethoxy, Phthalidyloxy, Pentachlorphenyloxy, Triniederalkylsilyloxy oder Niederalkenyloxy steht, wobei die mit "Nieder" bezeichneten Gruppen bis zu 7, insbesondere bis zu 4 Kohlenstoffatome enthalten, und Salze von solchen Verbindungen mit salzbildenden Gruppen.

Die Erfindung betrifft in erster Linie Verbindungen der Formel I, worin $R_a$ Niederalkyl, 1-Hydroxyniederalkyl, Phenylniederalkyl, Phenoxyniederalkanoyloxy oder Niederalkoxy ist und $R_1$ Wasserstoff, Niederalkyl, Aminoniederalkyl, Acylaminoniederalkyl, worin Acyl Niederalkanoyl oder eine als Aminoschutzgruppe gebräuchliche substituierte Oxycarbonylgruppe, z.B. eine tert.-Butyl-, 2,2,2-Trichloräthyl-, Diphenylmethyl- oder p-Nitrobenzyl-oxycarbonylgruppe, ist, Niederalkylthio, durch Amino, Mono- oder Diniederalkylamino oder Niederalkanoylamino substituiertes Niederalkylthio oder Niederalkenylthio, 1-Methyl-1H-tetrazol-5-ylthio, 1-(2-Dimethylaminoäthyl)-1H-tetrazol-5-ylthio, 2-Methyl-1, 3,4-thiadiazol-5-ylthio oder 1,3,4-Thiadiazol-2-ylthio bedeutet, und worin Niederalkyl-, Niederalkenyl- und Niederalkanoylgruppen bis zu 4 Kohlenstoffatome enthalten, die unter neutralen, basischen oder physiologischen Bedingungen spaltbaren Ester und die Salze von solchen Verbindungen mit salzbildenden Gruppen.

Die Erfindung betrifft in erster Linie die in den Beispielen genannten Verbindungen der Formel I und deren Salze, insbesondere die pharmazeutisch verwendbaren Salze.

Wegen ihrer besonders guten antibakteriellen Wirkung, ist die 6-Aethyl-2-(3-aminopropyl)-2-penem-3-carbonsäure, insbesondere die entsprechende 5R-Verbindung, sowie deren pharmakologisch verwendbaren Salze und physiologisch spaltbaren Ester hervorzuheben.

Die neuen Verbindungen werden hergestellt, indem man eine Ylid-Verbindung der Formel

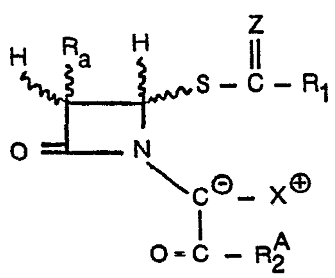

(II)

worin $R_a$, $R_1$ und $R_2^A$ die oben gegebenen Bedeutungen haben, wobei funktionelle Gruppen in diesen Resten vorzugsweise in geschützter Form vorliegen, Z Sauerstoff oder Schwefel bedeutet, und worin $X^{\oplus}$ entweder eine dreifach substituierte Phosphoniogruppe oder eine zweifach veresterte Phosphonogruppe zusammen mit einem Kation bedeutet, ringschliesst, und, wenn erwünscht oder notwendig, in einer erhaltenen Verbindung der Formel I die geschützte Carboxylgruppe der Formel —C(=O)—$R_2^A$ in die freie oder in eine andere geschützte Carboxylgruppe überführt, und/oder, wenn erwünscht, in einer erhaltenen Verbindung der Formel I innerhalb der Definition eine Gruppe $R_a$ und/oder $R_1$ in eine andere Gruppe $R_a$ und/oder $R_1$ überführt, und/oder, wenn erwünscht, eine erhaltene Verbindung mit salzbildender Gruppe in ein Salz oder ein erhaltenes Salz in die freie Verbindung oder in ein anderes Salz überführt, und/oder, wenn erwünscht, ein erhaltenes Gemisch von isomeren Verbindungen in die einzelnen Isomeren auftrennt.

Die Verbindungen der Formel (II) sind. Gegenstand der europäischen Anmeldung EP—A—0042026.

Im Ausgangsmaterial der Formel II haben $R_a$, $R_1$ und $R_2^A$ insbesondere die bevorzugten Bedeutungen, wobei funktionelle Gruppen üblicherweise in geschützter Form, Amino z.B. auch in Form der Nitro- oder Azidogruppe vorliegen.

In einem Ausgangsmaterial der Formel II steht $R_2^A$ vorzugsweise für eine, mit der —C(=O)-Gruppierung eine, insbesondere unter milden Bedingungen, leicht spaltbare, veresterte Carboxylgruppe bildende, verätherte Hydroxygruppe, wobei gegebenenfalls vorhandene funktionelle Gruppen in einer Carboxylschutzgruppe $R_2^A$ in an sich bekannter Weise, z.B. wie oben angegeben, geschützt sein können. Eine Gruppe $R_2^A$ ist u.a. Niederalkoxy, insbesondere $\alpha$-polyverzweigtes Niederalkoxy, z.B. Methoxy oder tert.-Butyloxy, Niederalkenyloxy, insbesondere 2-Niederalkenyloxy, z.B. Allyloxy, oder 2-Halogenniederalkoxy, z.B. 2,2,2-Trichloräthoxy, 2-Bromäthoxy, oder 2-Jodäthoxy, 2-Niederalkylsulfonyl-niederalkoxy, z.B. 2-Methylsulfonyläthoxy, oder eine gegebenenfalls substituierte, wie Niederalkoxy, z.B.

Methoxy, oder Nitro enthaltende 1-Phenylniederalkoxygruppe, wie gegebenenfalls, z.B. wie angegeben, substituiertes Benzyloxy oder Diphenylmethoxy, z.B. Benzyloxy, 4-Methoxybenzyloxy, 4-Nitrobenzyloxy, Diphenylmethoxy oder 4,4'Dimethoxy-diphenylmethoxy, Pentachlorphenyloxy, Acetonyloxy, 2-Cyanäthoxy, eine 2-$(S_1)(S_2)(S_3)$-Silyläthoxygruppe, wie 2-Trimethylsilyläthoxy, 2-(Dibutyl-methylsilyl)-äthoxy oder 2-Triphenylsilyläthoxy, ferner eine organische Silyloxy- oder Stannyloxygruppe, wie Triniederalkylsilyloxy, z.B. Trimethylsilyloxy, oder eine der genannten physiologisch spaltbaren verätherten Hydroxygruppen.

Die Gruppe $X^{\oplus}$ im Ausgangsmaterial der Formel II ist eine der bei Wittig-Kondensationsreaction gebräuchlichen Phosphonio- oder Phosphonogruppen, insbesondere eine Triaryl-, z.B. Triphenyl-, oder Triniederalkyl-, z.B. Tributylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetall, z.B. Lithium-, Natrium- oder Kaliumion, umfasst. Bevorzugt als Gruppe $X^{\oplus}$ sind einerseits Triphenylphosphonio und anderseits Diäthylphosphono zusammen mit einem Alkalimetall-, z.B. Natrium-ion.

In Phosphonio-Verbindungen der Formel II, die in der isomeren Ylenform auch als Phosphoran-Verbindungen bezeichnet werden, wird die negative Ladung durch die positive geladene Phosphoniogruppe neutralisiert. In Phosphono-Verbindung der Formel II, die man in ihrer isomeren Form auch als Phosphonat-Verbindungen bezeichnen kann, wird die negative Ladung durch das Kation einer starken Base neutralisiert, das je nach Herstellungsweise des Phosphono-Ausgangsmaterials z.B. ein Alkalimetall-, z.B. Natrium-, Lithium oder Kaliumion, sein kann. Die Phosphonat-Ausgangsstoffe werden daher als Salze in die Reaktion eingesetzt.

Die Formel II gibt das Ausgangsmaterial in der Form wieder, in welcher der Ringschluss stattfindet. Ueblicherweise wird die entsprechende Phosphoranyliden-Verbindung der Formel

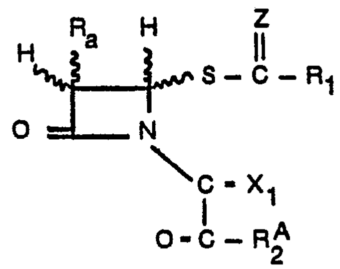

(IIA)

worin $X_1$ für einen trisubstituierten, insbesondere einen Triaryl-, z.B. Triphenyl-, oder einen Triniederalkyl-, z.B. Tri-n-butyl-phosphoranylidenrest, steht, oder die entsprechende Phosphono-Verbindung der Formel

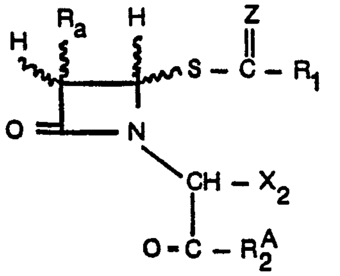

(IIB)

worin $X_2$ für eine Phosphono-, insbesondere eine Dialkylphosphono-, z.B. Diäthylphosphonogruppe steht, eingesetzt, wobei ein Phosphono-Ausgangsmaterial der Formel IIB durch Behandeln mit einem geeigneten basischen Reagens, wie einer anorganischen Base, z.B. einem Alkalimetallcarbonat, wie Natrium- oder Kaliumcarbonat, oder einer organischen Base, wie einem Triniederalkylamin, z.B. Triäthylamin, oder einer cyclischen Base vom Amidin-typ, wie einer entsprechenden Diazabicycloalkenverbindung, z.B. 1,5-Diazabicyclo[5.4.0]undec-5-en, in die zum Ringschluss geeignete Form, d.h. in die Verbindung der Formel II, übergeführt wird.

Bevorzugte Ausgangsmaterialien sind die Phosporanyliden-Verbindungen der Formel IIA.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 50°C und etwa 100°C, erfolgen.

Die Reaktion wird vorzugsweise in Gegenwart eines geeigneten inerten Lösungsmittels, wie in einem aliphatischen, cycloaliphatischen oder aromatischen Kohlenwasserstoff, z.B. Hexan, Cyclo-Hexan, Benzol, Toluol, Xylol oder Mesitylen, einem halogenierten Kohlenwasserstoff, z.B. Methylenchlorid, einem Aether, z.B. Diäthyläther, einem Niederalkylenglycoldiniederalkyläther, z.B. Dimethoxyäthan oder Diäthylenglycoldimethyläther, einem cyclischen Aether, z.B. Dioxan oder Tetrahydrofuran,

einem Carbonsäureamid, z.B. Dimethylformamid, einem Diniederalkylsulfoxid, z.B. Dimethylsulfoxid, oder einem Niederalkanol, z.B. Methanol, Aethanol oder tert.-Butanol, oder in einem Gemisch davon, und, falls notwendig, in einer Inertgas-, z.B. Argon- oder Stickstoffatmosphäre, durchgeführt.

In einer erfindungsgemäss erhältlichen Verbindung der Formel I mit einer geschützten Carboxylgruppe, insbesondere einer veresterten Carboxylgruppe der Formel $-C(=O)-R_2^A$ kann diese in an sich bekannter Weise, z.B. je nach Art der Schutzgruppe in die freie Carboxylgruppe übergeführt werden. So kann eine durch eine geeignete 2-Halogenniederalkyl-, eine Arylcarbonylmethyl- oder eine 4-Nitrobenzylgruppe veresterte Carboxylgruppe z.B. durch Behandeln mit einem chemischen Reduktionsmittel, wie einem Metall, z.B. Zink, oder einem reduzierenden Metallsalz, wie einem Chrom-II-saly, y.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie Essig-, sowie Ameisensäure, oder eines Alkohols, wobei man vorzugsweise Wasser zugibt, eine durch eine Arylcarbonylmethylgruppe veresterte Carboxylgruppe auch durch Behandeln mit einem nucleophilen, vorzugsweise salzbildenden Reagens, wie Natriumthiophenolat oder Natriumjodid, und eine durch 4-Nitrobenzyl veresterte Carboxylgruppe auch durch Behandeln mit einem Alkalimetall-, z.B. Natriumdithionit, in die freie Carboxylgruppe übergeführt werden. Eine durch eine 2-Niederalkylsulfonyl-niederalkylgruppe veresterte Carboxylgruppe kann z.B. durch Behandeln mit einem basischen Mittel, z.B. einem der weiter unten genannten nucleophil reagierenden Basen, und eine durch eine geeignete Arylmethylgruppierung veresterte Carboxylgruppe z.B. durch Bestrahlen, vorzugsweise mit ultraviolettem Licht, z.B. unter 290 mμ, wenn die Arylmethylgruppe z.B. einen gegebenenfalls in 3-, 4- und/oder 5-Stellung, z.B. durch Niederalkoxy- und/oder Nitrogruppen substituierten Benzylrest darstellt, oder mit längerwelligem ultraviolettem Licht, z.B. über 290 mμ, wenn die Arylmethylgruppe z.B. einen in 2-Stellung durch eine Nitrogruppe substituierten Benzylrest bedeutet, ferner eine durch eine geeignet substituierte Methylgruppe, wie tert.-Butyl oder Diphenylmethyl, veresterte Carboxylgruppe z.B. durch Behandeln mit einem geeigneten sauren Mittel, wie Ameisensäure oder Trifluoressigsäure, gegebenenfalls unter Zugabe einer nucleophilen Verbindung, wie Phenol oder Anisol, sowie eine hydrogenolytisch spaltbare veresterte Carboxylgruppe, z.B. Benzyloxycarbonyl oder 4-Nitrobenzyloxycarbonyl, durch Hydrogenolyse, z.B. durch Behandeln mit Wasserstoff in Gegenwart eines Edelmetall-, z.B. Palladiumkatalysators, gespalten und freigesetzt werden. Zudem kann eine mit einer Niederalkenylgruppe, wie mit 2-Niederalkenyl, insbesondere Allyl, veresterte Carboxylgruppe oxidativ, z.B. durch Behandeln mit Ozon, gefolgt von einem Reduktionsmittel, z.B. Dimethylsulfid, in eine Formylmethoxycarbonylgruppe übergeführt werden, aus der die Carboxylgruppe durch Behandeln mit einer Base, wie einem sekundären Amin, z.B. Dimethylamin, freisetzbar ist, oder man kann eine 2-Niederalkenyloxycarbonylgruppe, z.B. Allyloxycarbonyl, z.B. durch Behandeln mit Tristriphenylphosphinrhodiumchlorid, Palladium-auf-Kohle, oder einem Alkalimetallniederalkanolat, z.B. Kalium-tert.-butylat, in Dimethylsufoxid zu einer 1-Niederalkenyloxycarbonylgruppe isomerisieren und diese unter schwach-sauren oder schwach-basischen Bedingungen hydrolytisch spalten. Eine gegebenenfalls in 2-Stellung durch Niederalkyl oder Aryl substituierte 2-Oxoäthoxycarbonyl- oder 2-Cyanäthoxycarbonylgruppe, z.B. die Acetonyloxycarbonyl- oder 2-Cyanäthoxycarbonylgruppe, kann unter milden Bedingungen, d.h. bei Raumtemperatur oder unter Kühlen, durch Behandeln mit einer geeigneten Base in das entsprechende Salz dieser Carboxylgruppe übergeführt werden, aus dem die freie Carboxylgruppe durch Ansäuern erhältlch ist. Geeignete Basen sind nucleophil reagierende Metall-, wie Erdalkalimetall- und insbesondere Alkalimetallbasen, wie entsprechende Hydroxide, Carbonate, Bicarbonate, Alkoxide, Phenolate, Mercaptide, Thiophenolate, oder Amide, z.B. Natriumhydroxide, Natriumcarbonat, Natriumbicarbonat, Natriumäthanolat, Natriumthiophenolat, Natriumamid oder Natrium-morpholid, oder entsprechende Lithium oder Kaliumverbindungen, die in Wasser, wässrigen oder Hydroxylgruppen enthaltenden oder auch in polaren inerten Lösungsmitteln unter nachträglicher Behandlung mit Wasser zur Anwendung gelangen. Zur Spaltung der 2-Cyanäthoxycarbonylgruppen können auch tertiäre Amine, wie Triniederalkylamin, z.B. Triäthylamin oder Hünigbase, oder cyclische oder bicyclische Amine oder Imine, wie N-Methylmorpholin oder 1,5-Diazabicyclo[5.4.0]undec-5-en, in einem inerten Lösungsmittel, wie Methylenchlorid oder Tetrahydrofuran, benutzt werden, wobei direkt die entsprechenden Ammoniumsalze der Carboxylverbindung erhalten werden. Eine substituierte-Silyläthoxycarbonylgruppe kann durch Behandeln mit einem Salz der Fluorwasserstoffsäure, das Fluoridanionen liefert, wie einem Alkalimetallfluorid, z.B. Natrium- oder Kaliumfluorid, in Anwesenheit eines macrocyclischen Polyäthers ("Kronenäther"), oder mit einem Fluorid einer organischen quaternären Base, wie Tetraalkylammoniumfluorid oder Trialkylarylammoniumfluorid, z.B. Tetraäthylammoniumfluorid oder Tetrabutylammoniumfluorid, in Gegenwart eines aprotischen polaren Lösungsmittels, wie Dimethylsulfoxid oder N,N-Dimethylacetamid, in die freie Carboxylgruppe übergeführt werden. Eine Pentachlorphenyloxycarbonylgruppe kann unter milden Bedingungen, z.B. durch verdünnte Natriumcarbonat- oder Natriumbicarbonatlösung oder durch eine organische Base in Gegenwart von Wasser, in eine freie Carboxylgruppe übergeführt werden.

Eine z.B. durch Silylierung oder Stannylierung geschützte Carboxylgruppe kann in üblicher Weise solvolytisch, z.B. durch Behandeln mit Wasser oder einem Alkohol, freigesezt werden.

Falls mehr als eine geschützte Carboxylgruppe in einer erfindungsgemässen erhältlichen Verbindung vorhanden sind, können diese entweder gemeinsam oder selektiv in freie Carboxylgruppen übergeführt werden.

In einer verfahrensgemäss erhältlichen Verbindung der Formel I, die eine freie Carboxylgruppe enthält, kann eine solche in an sich bekanter Weise in eine geschützte Carboxvlqruppe übergeführt werden. So erhält man Ester z.B. durch Behandeln mit einer geeigneten Diazoverbindung, wie einem Diazoniederalkan, z.B. Diazomethan oder Diazobutan, oder einem Phenyldiazoniederalkan, z.B. Diphenyldiazomethan, wenn notwendig, in Gegenwart einer Lewissäure, wie z.B. Bortrifluorid, oder durch Umsetzen mit einem zur Veresterung geeigneten Alkohol in Gegenwart eines Veresterungsmittels, wie eines Carbodiimids, z.B. Dicyclohexylcarbodimid, sowie Carbonyldiimidazol, ferner mit einem N,N'-disubstituierten O- bzw. S-substituierten Isoharnstoff oder Isothioharnstoff, worin ein O- und S- Substituent z.B. Niederalkyl, insbesondere tert.-Butyl, Phenylniederalkyl oder Cycloalkyl, und N- bzw. N'-Substituenten z.B. Niederalkyl, insbesondere Isopropyl, Cycloalkyl oder Phenyl sind, oder nach irgendeinem anderen bekannten und geeigneten Veresterungsverfahren, wie Reaktion eines gegebenenfalls *in situ* hergestellten Salzes der Säure mit einem reaktionsfähigen Ester eines Alkohols und einer starken anorganischen Säure, sowie einer starken organischen Sulfonsäure. Ferner können Säurehalogenide, wie -chloride (hergestellt z.B. durch Behandeln mit Oxalylchlorid), aktivierte Ester (gebildet z.B. mit N-Hydroxystickstoffverbindungen, wie N-Hydroxysuccinimid) oder gemischte Anhydride (erhalten z.B. mit Halogenameisensäure-niederalkylestern, wie Chlorameisensäureäthyl- oder Chlorameisensäureisobutylester, oder mit Halogenessigsäurehalogeniden, wie Trichloressigsäurechlorid) durch Umsetzen mit Alkoholen, gegebenenfalls in Gegenwart einer Base, wie Pyridin, in eine veresterte Carboxylgruppe übergeführt werden.

In einer Verbindung der Formel I mit einer veresterten Carboxylgruppe kann diese in eine andere veresterte Carboxylgruppe übergeführt werden, z.B. 2-Chloräthoxycarbonyl oder 2-Bromäthoxycarbonyl durch Behandeln mit einem Jodsalz, wie Natriumjodid, in Gegenwart eines geeigneten Lösungsmittels, wie Aceton, in 2-Jodäthoxycarbonyl.

In einer verfahrensgemäss erhältlichen Verbindung mit einer freien Carboxylgruppe kann eine solche auch in eine gegebenenfalls substituierte Hydrazinocarbonylgruppe übergeführt werden, wobei man vorzugsweise reaktionsfähige funktionell abgewandelte Derivate, wie die obgenannten aktivierten Ester, oder gemischte Anhydride der entsprechenden Säure mit Hydrazinen umsetzt.

Eine durch eine organische Silyl, oder Stannylgruppe geschützte Carboxylgruppe kann in an sich bekannter Weise gebildet werden, z.B. indem man die Carboxyl enthaltende Verbindung oder ein Salz davon, wie ein Alkalimetall-, z.B. Natriumsalz davon, mit einem geeigneten Silylierungs- oder Stannylierungsmittel behandelt.

Im erfindungsgemässen Verfahren, sowie in gegebenenfalls oder notwendigerweise durchzuführenden Zusatzmassnahmen, sind, wenn notwendig, an der Reaktion nicht teilnehmende, freie funktionelle Gruppen, wie freie Aminogruppen, z.B. durch Acylieren, Tritylieren oder Silylieren, freie Hydroxy- und Mercaptogruppen z.B. durch Veräthern oder Verestern, inkl. Silylieren, in an sich bekannter Weise vorübergehend geschützt und können, falls erwünscht, nach erfolgter Reaktion, in an sich bekannter Weise einzeln oder gemeinsam freigesetzt werden. So können in einem Ausgangsmaterial vorhandene Amino-, Hydroxy-, Mercapto-, Carboxyl- oder Sulfogruppen z.B. in Form von Acylamino-, wie den obgenannten, z.B. 2,2,2-Trichloräthoxycarbonylamino-, 2-Bromäthoxycarbonylamino-, 4-Methoxybenzyloxycarbonylamino-, oder tert.-Butyloxycarbonylamino-, von Aryl- oder Arylniederalkylthioamino-, z.B. 2-Nitrophenylthioamino-, oder Arylsulfonylamino-, z.B. 4-Methylphenylsulfonylamino-, von 1-Niederalkoxycarbonyl-2-propylidenaminogruppen, oder von o-Nitrophenyloxyacetylaminogruppen bzw. von Acyloxy-, wie den obgenannten, z.B. tert. Butyloxycarbonyloxy-, 2,2,2-Trichloräthoxycarbonyloxy-, 2-Bromäthoxycarbonyloxy- oder p-Nitrobenzyloxycarbonyloxygruppen, oder entsprechenden Acylmercaptogruppen, bzw. von veresterten Carboxy-, wie den obgenannten, z.B. Diphenylmethoxycarbonyl-, p-Nitrobenzyloxycarbonyl-, Acetonyloxycarbonyl- oder 2-Cyanäthyloxycarbonylgruppen, bzw. von substituierten Sulfo-, wie den obgenannten Niederalkylsulfo-, z.B. Methylsulfogruppen, geschützt sein und nach erfolgter Reaktion, gegebenenfalls nach Umwandlung der Schutzgruppe, freigesetzt werden. Beispielsweise kann eine 2,2,2-Trichloräthoxycarbonylamino- oder 2-Jodäthoxycarbonylaminogruppe oder auch eine p-Nitrobenzyloxycarbonylaminogruppe durch Behandeln mit geeigneten Reduktionsmitteln, wie Zink in Gegenwart von wässriger Essigsäure bzw. Wasserstoff in Gegenwart eines Palladiumkatalysators, eine Diphenylmethoxycarbonylamino- oder tert.-Butylcarbonylaminogruppe durch Behandeln mit Ameisen- oder Trifluoressigsäure, eine Aryl- oder Arylniederalkylthioaminogruppe durch Behandeln mit einem nucleophilen Reagens, wie schwefliger Säure, eine Arylsulfonylaminogruppe mittels elektrolytischer Reduktion, eine 1-Niederalkoxycarbonyl-2-propylidenaminogruppe durch Behandeln mit wässriger Mineralsäure bzw. eine tert.-Butyloxycarbonyloxygruppe durch Behandeln mit Ameisen- oder Trifluoressigsäure, oder eine 2,2,2-Trichloräthoxycarbonyloxygruppe oder p-Nitrobenzyloxycarbonyloxygruppe durch Behandeln mit einem chemischen Reduktionsmittel, wie Zink in Gegenwart von wässriger Essigsäure, oder mit Wasserstoff in Gegenwart eines Palladiumcatalysators, bzw. eine Diphenylmethoxycarbonylgruppe durch Behandeln mit Ameisen- oder Trifluoressigsäure oder durch Hydrogenolyse, eine Acetonyloxy- oder Cyanäthoxycarbonylgruppe durch Behandeln mit Basen, wie Natriumbicarbonat oder 1,5-Diazabicyclo[5.4.0]undec-5-en, bzw. eine substituierte Sulfogruppe durch Behandeln mit einem Alkalimetallhalogenid, wenn erwünscht stufenweise, abgespalten werden.

Ferner kann man in einer erhaltenen Verbindung funktionelle Substituenten, wie freie Amino-, Hydroxy-, Mercapto-, Carboxy- oder Sulfogruppen nach an sich bekannten Verfahren, z.B. Durch Alkylieren, Acylieren bzw. Verestern bzw. Substituieren, funktionell abwandeln.

So kann man eine Amino-, Hydroxy-, Mercapto- oder Carboxygruppe durch Behandeln mit einem Alkylierungsreagens, wie einer Diazoverbindung, z.B. Diazomethan, oder einem reaktionsfähigen Ester eines Alkohols, z.B. Dimethylsulfat, alkylieren, z.B. methylieren, oder Amino-, Hydroxy- oder Mercaptogruppen durch Behandeln mit einem reaktionsfähigen funktionellen Derivat einer Säure, z.B. einem Anhydrid oder Säurechlorid, wie Acetanhydrid oder Acetylchlorid, acylieren, z.B. acetylieren.

Eine Hydroxygruppe im Substituent $R_a$, insbesondere die Hydroxygruppe in einem 1-Hydroxyniederalkylrest, lässt sich durch Behandeln mit einem Schwefeltrioxyd Komplex, z.B. dem Komplex mit Dioxan oder mit einer tertiären Stickstoffbase, wit Triniederalkylamin, z.B. Triäthylamin, N,N-Diniederalkylanilin, z.B. N,N-Dimethylanilin oder insbesondere mit Pyridin oder auch durch Behandeln mit der Amidosulfonsäure, gegebenenfalls in Gegenwart von Pyridin, in eine in entsprechender Salzform vorliegende Hydroxysulfonyloxygruppe umwandeln, die durch doppelten Umsatz mit einem entsprechenden Metallhydroxyd, Metallcarbonat oder Metallhydrogencarbonat, wie einem Alkalimetall-, z.B. Natriumhydroxyd, — carbonat oder -hydrogen-carbonat, in eine in Metallsalzform vorliegende Hydroxysulfonyloxygruppe umgewandelt werden kann.

In erfindungsgemäss erhältlichen Verbindungen können ferner, auf an sich bekannte Weise, primäre und sekundäre Hydroxygruppen durch Oxidation, z.B. nach Pfitzner-Moffatt, in Aldehyd- bzw. Ketogruppen übergeführt werden, oder, gegebenenfalls nach Acylierung, zusammen mit einem benachbarten aktivierten Wasserstoffatom unter Ausbildung einer C-C-Doppelbindung abgespalten werden. Aldehyd- oder Ketogruppen können durch Reduktion, z.B. mit komplexen Metallhydriden, in Hydroxygruppen oder durch Behandeln mit Alkoholen in Acetale bzw. Ketale, durch Behandeln mit einem Amin, Hydroxylamin oder Hydrazin in die entsprechenden Imine, Oxime oder Hydrazone, oder durch Behandeln mit einem Wittig-Reagens in die entsprechenden Methylidenverbindungen übergeführt werden. Erhaltene Acetale oder Ketale können in die entsprechenden Aldehyde bzw. Ketone überführt werden, z.B. durch Behandeln mit Trimethyljodsilan. In erfindungsgemäss erhaltenen Verbindungen können ferner, auf an sich bekannte Weise, C-C-Doppelbindungen reduziert werden, z.B. mit katalytisch angeregtem Wasserstoff. Halogen-, wie Brom- oder Jodsubstituenten können, z.B. durch Behandeln mit Zink/Silber in Methanol oder Methanol-Essigsäure, durch Wasserstoff ersetzt werden oder durch Behandeln mit einer metallorganischen Verbindung, wie Methylmagnesiumbromid oder Butyllithium, gefolgt von einem Aldehyd, z.B. Acetaldehyd, in 1-substituierte-1-Hydroxymethylgruppen, z.B. die 1-Hydroxyäthylgruppe, übergeführt werden. Eine Nitro- oder Azidogruppe kann man z.B. durch Behandeln mit katalytisch, wie durch einen Palladium- bzw. Platinoxidkatalysator aktivierten Wasserstoff in eine Aminogruppe, umwandeln. Die genannten Nachfolgereaktionen können sowohl an entsprechenden Stellen in den Resten $R_a$ als auch in den Resten $R_1$, durchgeführt werden.

Salze von Verbindungen der Formel I können in an sich bekannter Weise hergestellt werden. So kann man Salze von solchen Verbindungen mit sauren Gruppen z.B. durch Behandeln mit Metallverbindungen, wie Alkalimetallsalzen von geeigneten Carbonsäuren, z.B. dem Natriumsalz der $\alpha$-Aethylcapronsäure, oder mit Ammoniak oder einem geeigneten organischen Amin bilden, wobei man vorzugsweise stöchiometrische Mengen oder nur einen kleinen Ueberschuss des salzbildenden Mittels verwendet. Salze von Carbonsäuren der Formel I können auch durch basische Spaltung von den genannten, unter basischen Bedingungen spaltbaren Estern solcher Verbindungen, z.B. von 2-Cyanäthyl- oder von Acetonylestern, erhalten werden. Säureadditionssalze von Verbindungen der Formeln I mit basischen Gruppierungen erhält man in üblicher Weise, z.B. durch Behandeln mit einer Säure oder einem geeigneten Anionenaustauschreagens. Innere Salze von Verbindungen der Formel I, welche z.B. eine salzbildende Aminogruppe und eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden. Salze von 1-Oxyden von Verbindungen der Formel I mit salzbindenden Gruppen können in analoger Weise hergestellt werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Erhaltene Gemische von Isomeren können nach an sich bekannten Methoden, in die einzelnen Isomeren getrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren. Erhaltene Racemate können in üblicher Weise, gegebenenfalls nach Einführen von geeigneten salzbildenden Gruppierungen, z.B. durch Bilden eines Gemisches von diastereomeren Salzen und Überführung der optisch aktiven Salze in die freien Verbindungen oder durch fraktioniertes Kristallisieren aus optisch aktiven Lösungsmitteln, in die Antipoden getrennt werden.

Bei allen nachträglichen Umwandlungen erhaltener Verbindungen werden solche Reaktionen bevorzugt, die unter neutralen, alkalisch oder schwach basischen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbedingungen, gebildet werden. Beispielsweise kann ein Ausgangsmaterial der Formel II, worin Z Sauerstoff ist, aus einer Verbindung der Formel II, worin Z eine gegebenenfalls substituierte Methylidengruppe ist, durch Ozonisierung und anschliessende Reduktion des gebildeten Ozonids, analog dem in der Stufe 2.5 angegebenen Verfahren, in situ hergestellt werden, worauf, insbesondere wenn $R_1$ Wasserstoff ist, in der Reaktionslösung die Cyclisierung zur Verbindung der Formel I erfolgt.

Die Ausgangsverbindungen der Formel II und die Vorstufen können, wie in den Reaktionsschemata 1, 2 und 3 angegeben, wie folgt hergestellt werden:

REAKTIONSSCHEMA 1

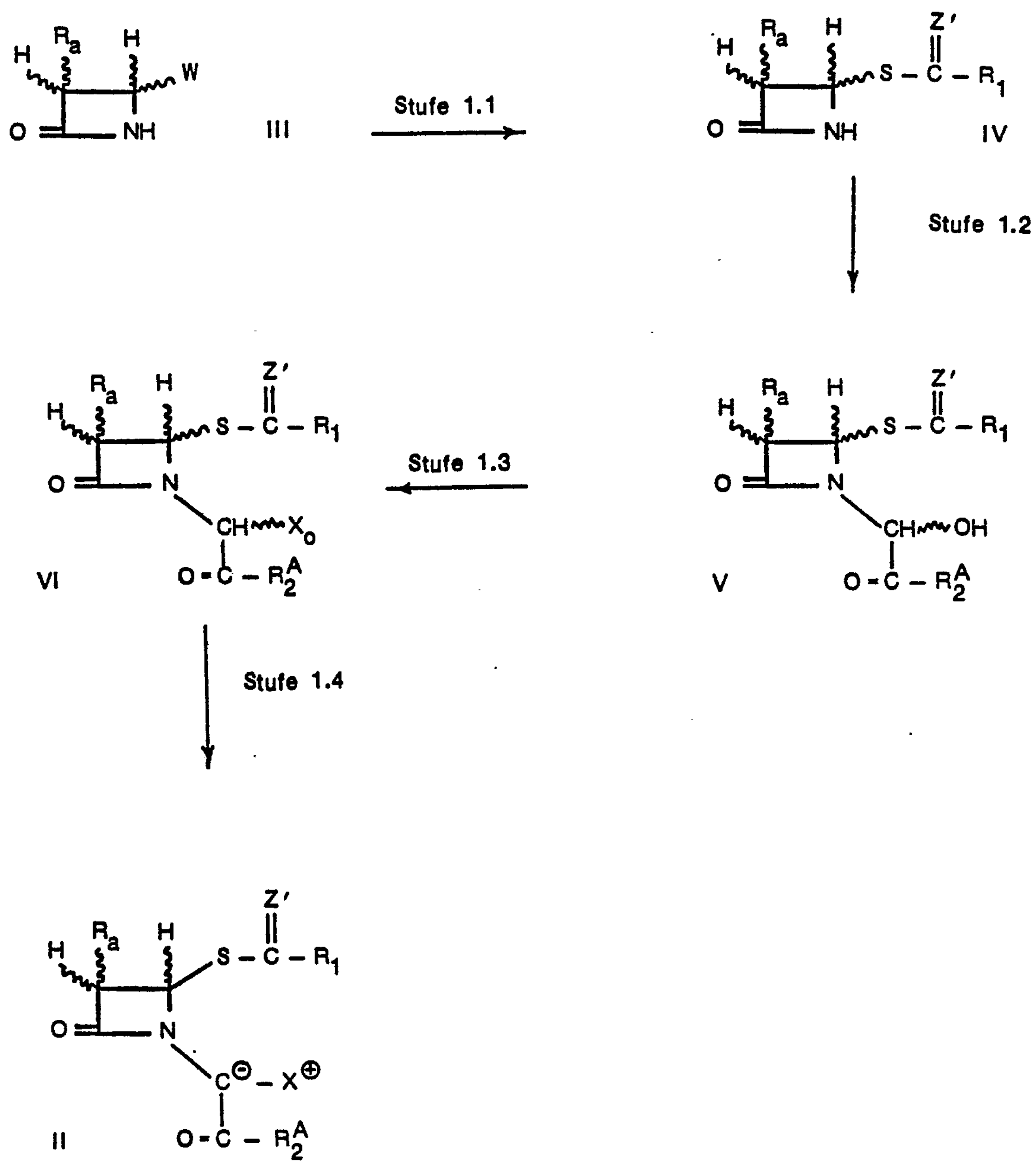


In den Verbindungen der Formeln IV, V, VI und II im Reaktionsschema I und in den Verbindungen der Formeln Xa, XI, XII und IVa im Reaktionsschema 2 ist Z' Sauerstoff, Schwefel oder auch, insbesondere wenn $R_1$ Wasserstoff ist, eine gegebenenfalls durch einen oder zwei Substituenten Y substituierte Methylidengruppe, die durch Oxidation in eine Oxogruppe Z übergeführt werden kann. Ein Substituent Y dieser Methylidengruppe ist ein organischer Rest, beispielsweise einer der unter $R_1$ erwähnten

organischen Reste, wie einer der genannten gegebenenfalls substituierten Niederalkyl-, Cycloalkyl-, Cycloalkylniederalkyl-, Phenyl- oder Phenylniederalkylreste, und insbesondere eine der funktionell abgewandelten, wie veresterten, inclusive mit einem optisch aktiven Alkohol, wie 1-Menthol, veresterten Carboxylgruppen. Diese Methylidengruppe trägt bevorzugt einen der genannten Subtituenten. Hervorzuheben sind die 2-Carbomethoxymethyliden- und die 2-Carbo-1-menthyloxymethylidengruppe Z'. Letztere kann zur Herstellung optisch aktiver Verbindungen der Formeln IV bis VI und II verwendet werden.

*Stufe 1.1:* Ein Thio-azetidinon der Formel IV wird erhalten, indem man ein 4-W-Azetidinon der Formel III, worin W eine nucleofuge Abgangsgruppe bedeutet, mit einer Mercaptoverbindung $R_1$—C(=Z')—SH oder einem Salz, z.B. einem Alkalimetall-, wie Natrium- oder Kaliumsalz davon, behandelt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die nucleofuge Abgangsgruppe W in einem Ausgangsmaterial der Formel III ist ein durch den nucleophilen Rest $R_1$—C(=Z')—S— ersetzbarer Rest. Solche Gruppen W sind beispielsweise Acyloxyreste, Sulfonylreste $R_0$—$SO_2$—, worin $R_0$ ein organischer Rest ist, Azido, oder Halogen. In einem Acyloxyrest W ist Acyl der Rest einer organischen Carbonsäure, inclusive einer optischen aktiven Carbonsäure, und hat beispielsweise die gleiche Bedeutung wie der Acylrst $R_1$—CO—, worin $R_1$ Wasserstoff oder einen der genannten, über ein Kohlenstoffatom gebundenen organischen Reste, z.B. einen der genannten, gegebenenfalls substituierten Niederalkyl-, Cycloalkyl-, Cycloalkylniederalkyl-, Phenyl- oder Phenylniederalkylreste, darstellt. In einem Sulfonylrest $R_0$—$SO_2$— ist $R_0$ beispielsweise ein gegebenenfalls substituierter aliphatischer, araliphatischer oder aromatischer Kohlenwasserstoffrest mit bis zu 12 C-Atomen, und ist insbesondere Niederalkyl, wie Methyl, Aethyl, oder ein durch einen optisch aktiven Rest substituiertes Methyl, wie Campheryl, oder Benzyl, Phenyl oder Toluyl. Ein Halogenrest W ist Brom, Jod oder insbesondere Chlor. W ist bevorzugt Acetoxy oder Chlor.

Die nucleophile Substitution kann unter neutralen oder schwach basischen Bedingungen in Gegenwart von Wasser und gegebenenfalls einem, mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, und ähnliche verwendet werden. Die Reaktion wird üblicherweise bei Raumtemperatur durchgeführt, kann aber auch bei erhöhter oder erniedrigter Temperatur durchgeführt werden. Durch Zugabe eines Salzes der Jodwasserstoffsäure oder der Thiocyansäure, z.B. eines Alkalimetall-, wie Natriumsalzes, kann die Reaktion beschleunigt werden.

In die Reaktion können sowohl optisch inaktive cis- oder trans-Verbindungen der Formel III, als auch Mischungen davon, oder entsprechende optisch aktive Verbindungen eingesetzt werden. Die eintretende Gruppe $R_1$—C(=Z')—S— wird von der Gruppe $R_a$ bevorzugt in die trans-Stellung dirigiert, unabhängig davon, ob W zur Gruppe $R_a$ in cis- oder trans-Stellung steht. Obwohl die trans-Isomeren überwiegend gebildet werden, können doch gelegentlich auch die cis-Isomeren isoliert werden. Die Auftrennung der cis- und trans-Isomeren erfolgt nach konventionellen Methoden, insbesondere durch Chromatographie und/oder durch Kristallisation.

Die nachträgliche Ozonisierung einer Methyliden-Gruppe Z' kann wie weiter unter angegeben durchgeführt werden. Ein erhaltenes Racemat der Formel IV kann in die optisch aktiven Verbindungen getrennt werden.

Die von der Formel IV umfassten optisch aktiven Verbindungen der Formel IVa können auch nach dem unten angegebenen Reaktionsschema 2 hergestellt werden.

Azetidinone der Formel III, worin $R_a$ Methyl ist und W Acetyl, Phenylsulfonyl oder Campher-10-sulfonyl ist, sind bekannt (Deutsche Offenlegungsschrift 1 906 401 oder K. Clauss et al., Liebigs Ann. Chem., *1974,* 539—560). Die übrigen Verbindungen der Formel III können nach an sich bekannten Methoden hergestellt werden.

Die Azetidinone der Formel III werden beispielsweise durch Addition von Chlorsulfonylisocyanat an entsprechend substituierte Vinylester und nachträgliche Abspaltung der Chlorsulfonylgruppe hergestellt. Bei dieser Synthese werden gewöhnlich Mischungen von cis- und trans-Isomeren erhalten, die gewünschtenfalls in die reinen cis- oder trans- Isomeren, z.B. durch Chromatographie, und/oder Kristallisation oder Destillation aufgetrennt werden können. Die reinen cis- und trans-Isomeren liegen als Racemate vor und können in ihre optischen Antipoden getrennt werden, beispielsweise wenn Acyl in einem Acyloxyrest W in Verbindungen der Formel III von einer optisch aktiven Säure stammt. Die von der Formel III umfassten optisch aktiven Verbindungen der Formel IIIa können auch nach den unten angegebenen Reaktionsschema 3 hergestellt werden.

*Stufe 1.2:* Eine $\alpha$-Hydroxycarbonsäureverbindung der Formel V wird erhalten, indem man eine Verbindung der Formel IV mit einer Glyoxylsäure-Verbindung der Formel OHC—C(=O)—$R_2^A$ oder einem geeigneten Derivat, wie einem Hydrat, Hemihydrat oder Halbacetal, z.B. einem Halbacetal mit einem

Niederalkanol, z.B. Methanol oder Aethanol, umsetzt, und gewünschtenfalls ein erhaltenes Isomerengemisch in die einzelnen Isomeren auftrennt, und/oder gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die Verbindung V erhält man üblicherweise als Gemisch der beiden Isomeren (bezüglich der Gruppierung >CH∿OH). Man kann aber auch die reinen Isomeren davon isolieren.

Die Anlagerung der Glyoxylsäureesterverbindung an das Stickstoffatom des Lactamrings findet bei Raumtemperatur oder, falls notwendig, unter Erwärmen, z.B. bis etwa 100°C, und zwar in Abwesenheit eines eigentlichen Kondensationsmittels und/oder ohne Bildung eines Salzes statt. Bei Verwendung des Hydrats der Glyoxylsäureverbindung wird Wasser gebildet, das, wenn notwendig, durch Destillation, z.B. azeotrop, oder durch Verwendung eines geeigneten Dehydrationsmittels, wie eines Molekularsiebs, entfernt wird. Vorzugsweise arbeitet man in Gegenwart eines geeigneten Lösungsmittels, wie z.B. Dioxan, Toluol oder Dimethylformamid, oder Lösungsmittelgemisches, wenn erwünscht oder notwendig, in der Atmosphäre eines Inertgases, wie Stickstoff.

In die Reaktion können sowohl reine optisch inaktive cis- oder trans-Verbindungen der Formel IV, als auch Mischungen davon, oder entsprechende optisch aktive Verbindungen eingesetzt werden. Ein erhaltenes Racemat der Formel V kann in die optisch aktiven Verbindungen getrennt werden.

*Stufe 1.3:* Verbindungen der Formel VI, worin $X_0$ für eine reaktionsfähige veresterte Hydroxygruppe, insbesondere für Halogen oder organisches Sulfonyloxy steht, werden hergestellt, indem man in einer Verbindung der Formel V die sekundäre Hydroxygruppe in eine reaktionsfähige veresterte Hydroxygruppe, insbesondere in Halogen, z.B. Chlor oder Brom, oder in eine organische Sulfonyloxygruppe, wie Niederalkylsulfonyloxy, z.B. Methylsulfonyloxy, oder Arylsulfonyloxy, z.B., 4-Methylphenylsulfonyloxy, umwandelt, gewünschtenfalls ein erhaltenes Isomerengemisch in die einzelnen Isomeren auftrennt, und gewünschtenfalls in einer erhaltenen Verbindung eine Gruppe $R_a$ und/oder $R_1$ in einer andere Gruppe $R_a$ bzw. $R_1$ überführt, und/oder gewünschtenfalls eine gegebenenfalls substituierte Methylidengruppe Z' in eine Oxogruppe Z überführt.

Die Verbindung VI kann man in Form von Gemischen der Isomeren (bezüglich der Gruppierung >CH∿$X_0$) oder in Form von reinen Isomeren erhalten.

Die obige Reaktion wird durch Behandeln mit einem geeigneten Veresterungsmittel, durchgeführt, indem man, z.B. ein Halogenierungsmittel, wie ein Thionylhalogenid, z.B. -chlorid, ein Phosphoroxyhalogenid besonders -chlorid, oder ein Halogenphosphoniumhalogenid, wie Triphenylphosphindibromid oder -dijodid, sowie ein geeignetes organisches Sulfonsäurehalogenid, wie -chlorid, vorzugsweise in Gegenwart eines basischen, in erster Linie eines organischen basischen Mittels, wie eines aliphatischen tertiären Amins, z.B. Triäthylamin, Diisopropyläthylamin oder "Polystyrol-Hünigbase," oder einer heterocyclischen Base vom Pyridintyp, z.B. Pyridin oder Collidin, verwendet. Vorzugsweise arbeitet man in Gegenwart eines geeigneten Lösungsmittels, z.B. Dioxan oder Tetrahydrofuran, oder eines Lösungsmittelsgemisches, wenn notwendig, unter Kühlen und/oder in der Atmosphäre eines Intergases, wie Stickstoff.

In einer so erhältlichen Verbindung der Formel VI kann eine reaktionsfähige verestere Hydroxygruppe $X_0$ in eine andere reaktionsfähige veresterte Hydroxygruppe in an sich bekannter Weise umgewandelt werden. So kann man z.B. ein Chloratom durch Behandeln der entsprechenden Chlorverbindung mit einem geeigneten Brom- oder Jodreagens, insbesondere mit einem anorganischen Bromid- oder Jodid- salz, wie Lithiumbromid, vorzugsweise in Gegenwart eines geeigneten Lösungsmittels, wie Aether, durch ein Brom- bzw. Jodatom austauschen.

In die Reaktion können sowohl reine optisch inaktive cis- oder trans-Verbindungen der Formel V als auch Mischungen davon, oder entsprechende optisch aktive Verbindung eingesetzt werden. Ein erhaltenes Racemat der Formel VI kann in die optisch aktiven Verbindungen getrennt werden.

*Stufe 1.4:* Ein Ausgangsmaterial der Formel II wird erhalten, indem man eine Verbindung der Formel VI, worin $X_0$ für eine reaktionsfähige veresterte Hydroxygruppe steht, mit einer geeigneten Phosphin-Verbindung, wie eine Triniederalkylphosphin, z.B. Tri-n-butyl-phosphin, oder einem Triarylphosphin, z.B. Triphenylphosphin, oder mit einer geeigneten Phosphit-Verbindung, wie einem Triniederalkylphosphit, z.B. Triäthylphosphit, oder einem Alkalimetalldimethylphosphit behandelt, wobei man je nach Wahl des Reagenzes eine Verbindung der Formel IIA bzw. IIB erhalten kann, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die obige Reaktion wird vorzugsweise in Gegenwart eines geeigneten inerten Lösungsmittels, wie eines Kohlenwasserstoffs, z.B. Hexan, Cyclohexan, Benzol, Toluol oder Xylol, oder eines Aethers, z.B. Dioxan, Tetrahydrofuran oder Diäthylenglykol-dimethyläther, oder eines Lösungsmittelgemisches vorgenommen. Je nach Reaktionsfähigkeit arbeitet man unter Kühlen oder bei erhöhter Temperatur, etwa zwischen −10° und +100°, bevorzugt bei etwa 20° bis 80°, und/oder in der Atmosphäre eines inerten Gases, wie Stickstoff. Zur Verhinderung oxidativer Prozesse können katalytische Mengen eines Antioxidans, z.B. Hydrochinon, zugesetzt werden.

Dabei arbeitet man bei der Verwendung einer Phosphinverbindung üblicherweise in Gegenwart eines basischen Mittels, wie einer organischen Base, z.B. eines Amins wie Triäthylamin, Diisopropyl-

äthyl-amin oder "Polystyrol-Hünigbase", und gelangt so direkt zum Phosphoranyliden- Ausgangsmaterial der Formel IIA, das aus dem entsprechenden Phosphoniumsalz gebildet wird.

In die Reaktion können sowohl reine, optisch inaktive cis- oder trans-Verbindungen der Formel VI, als auch Mischungen davon, oder entsprechende optisch aktive Verbindung eingesetzt werden. Ein erhaltenes Racemat der Formel II kann in die optisch aktiven Verbindungen getrennt werden.

In den Verbindungen der Formeln II bis VI bedeutet $R_a$ bevorzugt einen der genannten gesättigten oder ungesättigten, gegebenenfalls substituierten aliphatischen, cycloaliphatischen, cycloaliphatisch-aliphatischen, aromatischen oder araliphatischen Kohlenwasserstoffreste oder gegebenenfalls substituierten Heterocyclyl- oder Heterocyclylniederalkylreste, oder auch eine verätherte Hydroxygruppe, wobei gegebenenfalls vorhandene funktionelle Gruppen in einem solchen Rest $R_a$ bevorzugt in geschützter Form vorliegen.

Die Auftrennung der vorstehend genannten cis, trans- Verbindungen in die reinen cis- und trans-Isomere erfolgt nach den üblichen Trennungsmethoden, z.B. chromatographisch und/oder durch Destillation oder Kristallisation.

Die Spaltung der vorstehend genanten Racemate in ihre optischen Antipoden erfolgt nach an sich bekannten Methoden.

Eine dieser Methoden besteht darin, dass man ein Racemat mit einem optisch aktiven Hilfstoff reagieren lässt, das dabei entstandene Gemisch zweier diastereomerer Verbindungen mit Hilfe von geeigneten physikalischchemischen Methoden trennt und die einzelnen diastereomeren Verbindungen dann in die optisch aktiven Verbindungen spaltet.

Zur Trennung in Antipoden besonders geeignete Racemate sind solche, die eine saure Gruppe besitzen, wie z.B. Racemate von Verbindungen der Formel I. Andere der beschriebenen Racemate kann man durch einfache Reaktionen in saure Racemate umwandeln. Beispielsweise reagieren Aldehyde- oder Ketogruppen tragende Racemate mit einem saure Gruppen tragenden Hydrazinderivat, z.B. 4-(4-Carboxyphenyl)-semicarbazid zu den entsprechenden Hydrazonderivaten oder Alkoholgruppen enthaltende Verbindungen mit einem Dicarbonsäureanhydrid, z.B. Phthalsäureanhydrid, zum Racemat eines sauren Halbesters.

Diese sauren Racemate können mit optisch aktiven Basen, z.B. Estern von optisch aktiven Aminosäuren, oder (—)-Brucin, (+)-Chinidin, (—)-Chinin, (+)-Cinchonin, (+)-Dehydroabietylamin, (+)- und (—)-Ephedrin, (+)- und (—)-1-Phenyl-äthylamin oder deren N-mono- oder N,N-dialkylierten Derivaten zu Gemischen, bestehend aus zwei diastereomeren Salzen, umgesetzt werden.

In Carboxylgruppen enthaltenden Racematen, z.B. auch in Racematen, die eine funktionell abgewandelte Carboxymethylidengruppe Z' enthalten, kann diese Carboxylgruppe durch einen optisch aktiven Alkohol, wie (—)-Menthol, (+)-Borneol, (+)- oder (—)-2-Octanol verestert sein oder werden, worauf nach erfolgter Isolierung des gewünschten Diastereomeren die Carboxylgruppe freigesetzt wird, oder der die veresterte Carboxylgruppe enthaltende Molekülteil, z.B. der veresterte Carboxymethylidenrest, abgespalten wird.

Hydroxygruppen enthaltende Racemate können ebenfalls in ihre optischen Antipoden gespalten werden, wobei insbesondere optisch aktive Säuren oder deren reaktionsfähige, funktionelle Derivate Verwendung finden, die mit den genannten Alkoholen diastereomere Ester bilden. Solche Säuren sind beispielsweise (—)-Abietinsäure, D(+)- und L(—)-Aepfelsäure, N-acylierte optisch aktive Aminosäuren, (+) und (—)-Camphansäure, (+) und (—)-Ketopinsäure, L(+)-Ascorbinsäure, (+)-Camphersäure, (+)-Campher-10-sulfonsäure($\beta$), (+) oder (—)-$\alpha$-Bromcampher-$\pi$-sulfonsäure, D(—)-Chinasäure, D(—)-Isoascorbinsäure, D(—)- und L(+)-Mandelsäure, (+)-1-Menthoxyessigsäure, D(—)- und L(+)-Weinsäure und deren Di-O-benzoyl- und Di-O-p-toluylderivate. Die Acylreste der genannten optisch aktiven Säuren können beispielsweise als Acyl in Verbindungen der Formel III oder als $R_1$—C(=O)- in Verbindungen der Formeln II und IV bis VI vorliegen und die Racematspaltung solcher Verbindungen ermöglichen. Wenn erwünscht oder notwendig kann nach erfolgter Racematspaltung die optisch aktive Gruppe $R_1$—C(=O)- in eine gewünschte optisch inaktive Gruppe $R_1$—C(=O)- übergeführt werden.

Hydroxygruppen enthaltende Racemate können in ein Gemisch diastereomerer Urethane umgewandelt werden, beispielsweise durch Umsetzung mit optisch aktiven Isocyanaten, wie mit (+)- oder (—)-1-Phenyläthylisocyanat.

Basische Racemate können mit den optisch aktiven Säuren diastereomere Salze bilden. Doppelbindungen enthaltende Racemate können beispielsweise mit Platinchlorid und (+)-1-Phenyl-2-aminopropan in Gemische diastereomerer Komplexsalze übergeführt werden.

Zur Trennung der Diastereomerengemische eignen sich physikalisch-chemische Methoden, in erster Linie die fraktionierte Kristallisation. Brauchbar sind aber auch chromatographische Methoden, vor allem fest-flüssig-Chromatographie. Leichtflüchtige Diastereomerengemische können auch durch Destillation oder Gaschromatographie getrennt werden.

Die Spaltung der aufgetrennten Diastereomeren in die optisch aktiven Ausgangsmaterialien erfolgt ebenfalls nach ublichen Methoden. Aus den Salzen befreit man die Säuren oder die Basen z.B. durch Behandeln mit stärkeren Säuren bzw. Basen als die ursprünglich eingesetzten. Aus den Estern und Urethanen erhält man die gewünschten optisch aktiven Verbindungen beispielsweise nach alkalischer Hydrolyse oder nach Reduktion mit einem komplexen Hydrid, wie Lithiumaluminiumhydrid.

Eine weitere Methode zur Auftrennung der Racemate besteht in der Chromatographie an optisch

aktiven Absorptionsschichten, beispielsweise an Rohrzucker.

Nach einer dritten Methode können die Racemate in optisch aktiven Lösungsmitteln gelöst und der schwerer lösliche optische Antipode auskristallisiert werden.

Bei einer vierten Methode benützt man die verschiedene Reaktionsfähigkeit der optischen Antipoden genüber biologischem Material wie Mikroorganismen oder isolierten Enzymen.

Nach einer fünften Methode löst man die Racemate und kristallisiert einen der optischen Antipoden durch Animpfen mit einer kleinen Menge eines nach den obigen Methoden erhaltenen optisch aktiven Produkten aus.

Die optisch aktiven trans-Verbindungen der Formel IVa können auch nach dem folgenden Reaktionsschema hergestellt werden:

REAKTIONSSCHEMA 2

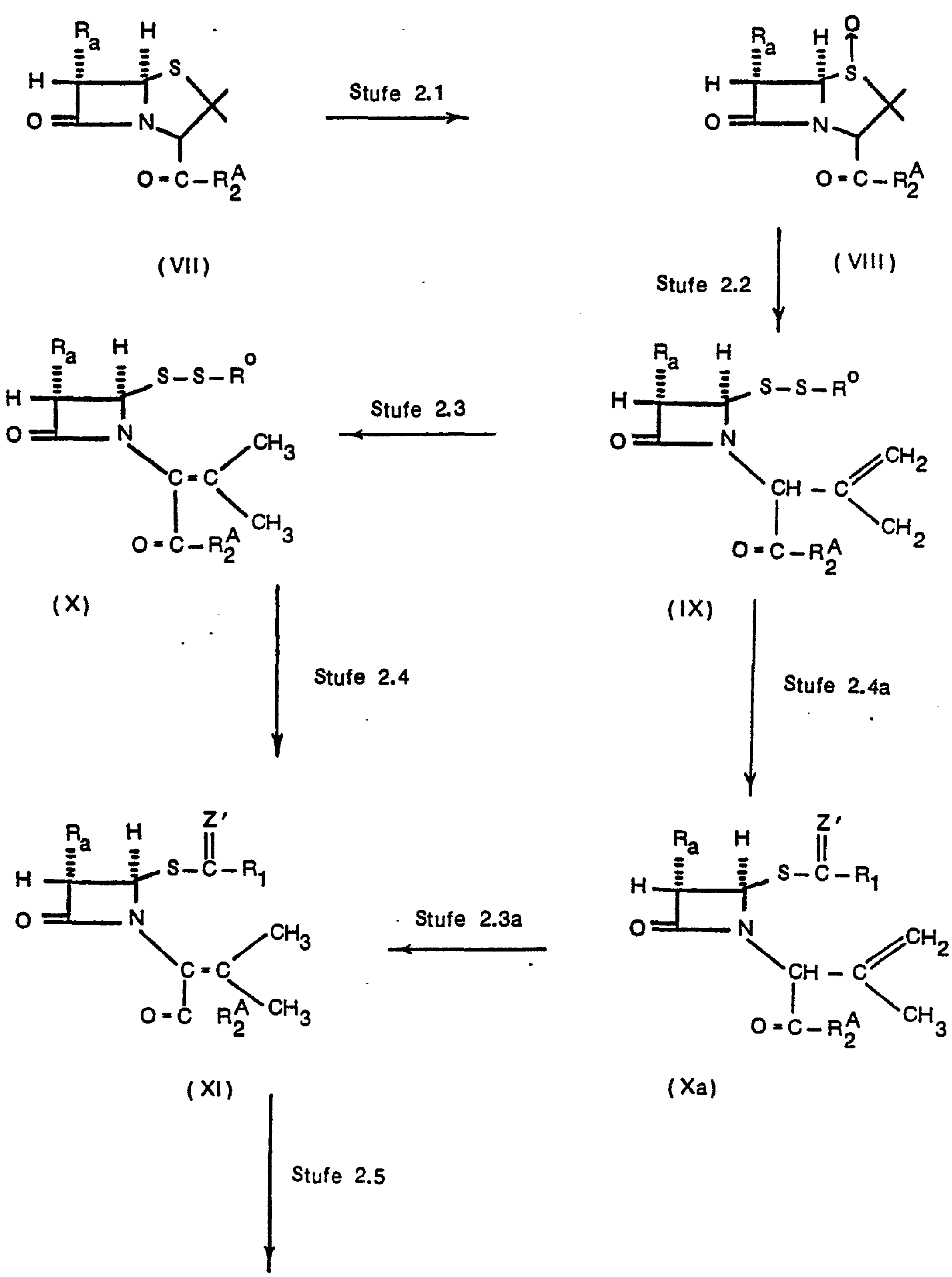


(VII) (VIII) (X) (IX) (XI) (Xa)

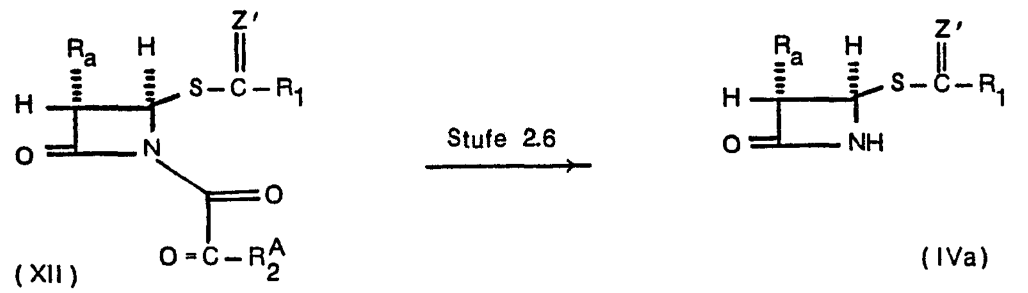


(XII) (IVa)

*Stufe 2.1:* Ein Oxid einer Penicillansäureverbindung der Formel VIII wird erhalten, indem man eine Penicillansäureverbindung der Formel VII in 1-Stellung oxidiert, und gewünschtenfalls in einer erhaltenen Verbindung eine Gruppe $R_a$ in eine andere Gruppe $R_a$ überführt.

Die Oxidation erfolgt auf an sich bekannte Weise mit geeigneten Oxidationsmitteln, wie Wasserstoffperoxid oder anorganischen oder organischen Persäuren. Geeignete anorganische Persäuren sind z.B. Perjod- oder Perschwefelsäure. Geeignete organische Persäuren sind z.B. Percarbonsäuren wie Perameisensäure, Peressigsäure, Trifluorperessigsäure, Permaleinsäure, Perbenzoesäure, 3-Chlorperbenzoesäure oder Monoperphthalsäure, oder Persulfonsäuren, z.B. p-Toluolpersulfonsäure. Die Persäuren können auch *in situ* aus Wasserstoffperoxid und den entsprechenden Säuren hergestellt werden. Die Oxidation erfolgt unter milden Bedingungen, z.B. bei Temperaturen von etwa —50° bis etwa +100°, vorzugsweise bei etwa —10° bis etwa +40°, in einem inerten Lösungsmittel.

Racemische 1-Oxide der Formel VIII, worin $R_a$ Phenoxy oder Methoxy und $R_2^A$ Methoxy bedeuten, sind bekannt [A.K. Bose et al., Tetrahedron 28, 5977 (1972)].

Ausgangsverbindungen der Formel VII sind bekannt oder können analog bekannten Verfahren hergestellt werden. Beispielsweise können sie nach D. Hauser und H. P. Sigg, Helv. Chimica Acta *50*, 1327 (1967), erhalten werden, indem man einen 6-Diazopenicillansäureester, der gegebenenfalls *in situ* aus einem 6-Aminopenicillansäureester und salpetriger Säure hergestellt wird, mit Wasser oder einem Alkohol oder einer Säure der Formel H—$R_a$ umsetzt. Verbindungen der Formel VII, worin $R_a$ eine Acyloxygruppe ist, können ebenfalls nach D. Hauser erhalten werden, indem man einen entsprechenden 6$\alpha$- oder 6$\beta$-N-Nitrosoacylaminopenicillansäureester in einem inerten Lösungsmittel pyrolysiert. Verbindungen der Formel VII, worin $R_a$ Hydroxy ist, sind auch durch J. C. Sheehan et al., J. Org. Chem. *39*, 1444 (1974) beschrieben worden (Herstellung aus entsprechenden 6-Diazopenicillansäureverbindungen). Weitere Ausgangsstoffe der Formel VII, worin $R_a$ gegebenenfalls geschütztes 1-Hydroxyäthyl, Brom oder Jod ist, sind durch DiNinno et al. bekannt geworden (J. Org. Chem. *42* (1967), 2960). In einer erhaltenen Verbindung der Formel VIII kann eine Gruppe $R_a$ in eine andere Gruppe $R_a$ übergeführt werden.

*Stufe 2.2:* Eine 3-Methylenbuttersäureverbindung der Formel IX wird erhalten, indem man ein 1-Oxid einer Penicillansäureverbindung der Formel VIII mit einer Mercaptoverbindung R°-SH behandelt, und gewünschtenfalls in einer erhaltenen Verbindung eine Gruppe $R_a$ in eine andere Gruppe $R_a$ umwandelt.

In der Mercaptoverbindung R°—SH und in dem Reaktionsprodukt der Formel IX ist R° ein gegebenenfalls substituierter aromatischer heterocyclischer Rest mit bis zu 15, bevorzugt bis zu 9 Kohlenstoffatomen, und mindestens einem Ringstickstoffatom und gegebenenfalls einem weiteren Ringheteroatom, wie Sauerstoff oder Schwefel, welcher Rest mit einem seiner Ringkohlenstoffatome, das mit einem Ringstickstoffatom durch eine Doppelbindung verbunden ist, an die Thiogruppe —S— gebunden ist. Solche Reste sind monocyclisch oder bicyclisch und können beispielsweise durch Niederalkyl, wie Methyl oder Aethyl, Niederalkoxy, wie Methoxy oder Aethoxy, Halogen, wie Fluor oder Chlor, oder Aryl, wie Phenyl, substituiert sein.

Solche Reste R° sind z.B. monocyclische fünfgliedrige thiadiazacyclische, thiatriazacyclische, oxadiazacyclische oder oxatriazacyclische Reste aromatischen Charakters, insbesondere aber monocyclische fünfgliedrige diazacyclische, oxazacyclische und thiazacyclische Reste aromatischen Charakters, und oder in erster Linie die entsprechenden benzdiazacyclischen, benzoxazacyclischen oder benzthiazacyclischen Reste, worin der heterocyclische Teil fünfgliedrig ist und aromatischen Charakter aufweist, wobei in Resten R° ein substituierbares Ringstickstoffatom z.B. durch Niederalkyl substituiert sein kann. Repräsentativ für solche Gruppen R° sind 1-Methyl-imidazol-2-yl, 1,3-Thiazol-2-yl, 1,3,4-Thiadiazol-2-yl, 1,3,4,5-Thiatriazol-2-yl, 1,3-Oxazol-2-yl, 1,3,4-Oxadiazol-2-yl, 1,3,4,5-Oxatriazol-2-yl, 2-Chinolyl, 1-Methyl-benzimidazol-2-yl, Benzoxazol-2-yl und insbesondere Benzthiazol-2-yl.

Die Reaktion wird in einem inerten Lösungsmittel, wie einem aliphatischen oder aromatischen Kohlenwasserstoff, z.B. Benzol oder Toluol, unter Erwärmen bis zur Rückflusstemperatur des verwendeten Lösungsmittels ausgeführt.

*Stufe 2.3:* Eine 3-Methylcrotonsäureverbindung der Formel X wird erhalten, indem man eine 3-Methylenbuttersäureverbindung der Formel IX durch Behandeln mit einem geeigneten basischen Mittel isomerisiert, und gewünschtenfalls in einer erhaltenen Verbindung eine Gruppe $R_a$ in eine andere Gruppe $R_a$ umwandelt.

Geeignete basische Mittel sind beispielsweise organische Stickstoffbasen, wie tertiäre Amine, z.B. Triniederalkylamine, wie Triäthylamin oder Hünigbase, oder auch anorganische Basen, die in einem inerten Lösungsmittel, wie einem gegebenenfalls halogenierten Kohlenwasserstoff, z.B. Methylenchlorid, bei Raumtemperatur oder gegebenenfalls leicht erniedrigter oder erhöhter Temperatur, zur Anwendung galangen.

*Stufe 2.4:* Eine Thioverbindung der Formel XI wird erhalten, indem man eine Verbindung der Formel X mit einem geeigneten Reduktionsmittel behandelt und gleichzeitig oder nachträglich mit einem Acylierungsderivat einer Saure der Formel $R_1$—C(=Z)—OH, oder, wenn Z' eine gegebenenfalls durch Y substituierte Methylidengruppe bedeutet, mit einem Alkin der Formel $R_1$—C ≡ C—Y umsetzt, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

Geeignete Reduktionsmittel sind beispielsweise Hydridreduktionsmittel, wie Alkalimetallborohydride, z.B. Natriumborhydrid, oder auch Zink in Gegenwart einer Carbonsäure, z.B. einer Carbonsäure der Formel $R_1$—C(=O)—OH. Die Hydridreduktionsmittel werden üblicherweise in Gegenwart von geeigneten Lösungsmitteln, wie Dimethylformamid eingesetzt. Die Hydridreduktion wird bevorzugt in Dimethylformamid mit Natriumborhydrid bei Temperaturen von etwa —50° bis etwa —10°, bevorzugt bei etwa —20°, durchgeführt, worauf bei der gleichen Temperatur das Acylierungsmittel und gegebenenfalls eine tertiäre Base, wie Pyridin, zugefügt wird. Die Reduktion mit Zink und einer Carbonsäure wird gegebenenfalls in einem Lösungsmittel, wozu die Carbonsäure, falls sie flüssig ist, selbst dienen kann, bei Temperaturen von etwa —10 bis etwa +50° bevorzugt bei etwa 0° bis Raumtemperatur durchgeführt. Das Acylierungsmittel kann der Reduktionsmischung von Anfang an zugegeben werden oder nach Beendigung der Reduktion und gegebenenfalls nach Abdampfen der verwendeten Carbonsäure und/oder des Lösungsmittels. Geeignete Acylierungsmittel sind insbesondere die Anhydride der genannten Carbonsäuren, wie symmetrische Anhydride, z.B. Acetanhydrid, oder gemischte Anhydride, vorzugsweise solche mit Halogenwasserstoffsäuren, d.h. die entsprechenden Carbonsäurehalogenide, z.B. die Chloride und Bromide, wie Acetylbromid. Beispielsweise kann eine Verbindung der Formel X mit Zink in einer Mischung von Essigsäure und Acetanhydrid bei 0° bis etwa 20° in eine Verbindung der Formel XI übergeführt werden, worin $R_1$ Methyl ist. Wegen der geringeren Racemisierungsgefahr wird die Zink/Carbonsäure-Reduktion bevorzugt. Das Alkin kann ebenfalls von Anfang an oder auch erst nach Beendigung der Reduktion zum Reduktionsgemisch zugegeben werden. Die Addition des bei der Reduktion intermediär entstehenden 4-Mercaptoazetidin-2-ons an die Dreifachbindung des Alkins erfolgt spontan bei der Reduktionstemperatur.

*Stufe 2.3a:* Eine Thioverbindung der Formel XI wird auch erhalten, indem man eine Verbindung der Formel Xa gemäss den Reaktionsbedingungen der Stufe 2.3 durch Behandeln mit einem geeigneten basischen Mittel isomerisiert, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

*Stufe 2.4a*: Eine Verbindung der Formel Xa wird erhalten, indem man eine 3-Methylenbuttersäureverbindung der Formel IX gemäss den Reaktionsbedingungen der Stufe 2.4 mit einem geeigneten Reduktionsmittel behandelt und gleichzeitig oder nachträglich mit einem Acylierungsderivat einer Carbonsäure der Formel $R_1$—C(=Z)—OH, oder, wenn Z' eine gegebenenfalls durch Y substituierte Methylidengruppe bedeutet, mit einem Alkin der Formel $R_1$—C ≡ C—Y, umsetzt, und gewünschtenfalls in einer erhaltenen Verbindung eine Gruppe $R_a$ oder $R_1$ in eine andere Gruppe $R_a$ bzw. $R_1$ umwandelt, und/oder gewünschtenfalls eine gegebenenfalls substituierte Methylidengruppe Z' in eine Oxogruppe Z überführt.

*Stufe 2.5:* Eine 2-Oxoessigsäureverbindung der Formel XII wird erhalten, indem man eine Verbindung der Formel XI ozonisiert und das gebildete Ozonid reduktiv zur Oxo-Verbindung spaltet, und gewünschtenfalls in einer erhaltenen Verbindung eine Gruppe $R_a$ oder $R_1$ in eine andere Gruppe $R_a$ bzw. $R_1$ überführt, und/oder gewünschtenfalls eine gegebenenfalls substituierte Methylidengruppe Z' in eine Oxogruppe Z überführt.

Die Ozonisierung wird üblicherweise mit einem Ozon-Sauerstoff-Gemisch in einem inerten Lösungsmittel, wie einem Niederalkanol, z.B. Methanol oder Aethanol, einem Niederalkanol, z.B. Aceton, einem gegebenenfalls halogenierten aliphatische, cycloaliphatischen oder aromatischen Kohlenwasserstoff, z.B. einem Halogenniederalkan, wie Methylenchlorid oder Tetrachlorkohlenstoff, oder in einem Lösungsmittelgemisch, inkl. einem wässrigen Gemisch, vorzugsweise unter Kühlen z.B. bei Temperaturen von etwa —90° bis etwa 0° durchgeführt.

Ein als Zwischenprodukt erhaltenes Ozonid wird, üblicherweise ohne isoliert zu werden, reduktiv zu einer Verbindung der Formel XII gespalten, wobei man katalytisch aktivierten Wasserstoff, z.B. Wasserstoff in Gegenwart eines Schwermetallhydrierkatalysators, wie eines Nickel- ferner Palladiumkatalysators, vorzugsweise auf einem geeigneten Trägermaterial, wie Calciumcarbonat oder Kohle, oder chemische Reduktionsmittel, wie reduzierende Schwermetalle, inkl. Schwermetallegierungen oder -amalgame, z.B. Zink, in Gegenwart eines Wasserstoffdonators, wie einer Säure, z.B. Essigsäure, oder eines Alkohols, z.B. Niederalkanols, reduzierende anorganische Salze, wie Alkalimetalljodide, z.B. Natriumjodid, oder Alkalimetallhydrogensulfite, z.B. Natriumhydrogensulfit,

in Gegenwart eines Wasserstoffdonators, wie einer Säure, z.B. Essigsäure, oder Wasser, oder reduzierende organische Verbindungen, wie Ameisensäure, verwendet. Als Reduktionsmittel kommen auch Verbindungen zum Einsatz, die leicht in entsprechende Epoxiverbindungen oder Oxide umgewandelt werden können, wobei die Epoxidbildung aufgrund einer C,C-Doppelbindung und die Oxidbildung aufgrund eines vorhandenen Oxidbildenden Hetero-, wie Schwefel-, Phosphor- oder Stickstoffatoms erfolgen kann. Solche Verbindungen sind z.B. geeignet substituierte Aethenverbindungen (die in der Reaktion in Aethylenoxidverbindungen umgewandelt werden), wie Tetracyanäthylen, dann insbesondere geeignete Sulfidverbindungen (die in der Reaktion in Sulfoxidverbindungen umgewandelt werden), wie Diniederalkylsulfide, in erster Linie Dimethylsulfid, geeignete organische Phosphorverbindungen, wie ein Phosphin, das gegebenenfalls substituierte aliphatische oder aromatische Kohlenwasserstoffreste als Substituenten enthält (und das in der Reaktion in ein Phosphinoxid umgewandelt wird), wie Triniederalkyl-phosphine, z.B. Tri-n-butylphosphin, oder Triarylphosphine, z.B. Triphenylphosphin, ferner Phosphite, welche gegebenenfalls substituierte aliphatische Kohlenwasserstoffreste als Substituenten enthalten (und in der Reaktion in Phosphorsäuretriester übergeführt werden), wie Triniederalkyl-phosphite, üblicherweise in der Form von entsprechenden Alkoholadduktverbindungen, wie Trimethylphosphit, oder Phosphorigsäure-triamide, welche gegebenenfalls substituierte aliphatische Kohlenwasserstoffreste als Substituenten enthalten, wie Hexaniederalkylphosphorigsäuretriamide, z.B. Hexamethylphosphorigsäuretriamid, letzteres vorzugsweise in der Form eines Methanoladdukts, ferner geeignete Stickstoffbasen (die in der Reaktion in die entsprechenden N-Oxide umgewandelt werden), wie heterocyclische Stickstoffbasen aromatischen Charakters, z.B. Basen vom Pyridintyp und insbesondere Pyridin selber. Die Spaltung des üblicherweise nicht isolierten Ozonids erfolgt normalerweise unter den Bedingungen, die man zu seiner Herstellung anwendet, d.h. in Gegenwart eines geeigneten Lösungsmittels oder Lösungsmittelgemisches, sowie unter Kühlen oder leichtem Erwärmen, wobei man vorzugsweise bei Temperaturen von etwa —10°C bis etwa +25°C arbeitet und die Reaktion üblicherweise bei Raumtemperatur abschliesst.

*Stufe 2.6:* Eine Verbindung der Formel IVa wird erhalten, indem man eine Verbindung der Formel XII solvolysiert, und wenn erwünscht in einer erhaltenen Verbindung eine Gruppe $R_a$ oder $R_1$ in eine andere Gruppe $R_a$ oder $R_1$ überführt, und/oder gewünschtenfalls eine gegebenenfalls substituierte Methylidengruppe Z' in eine Oxogruppe Z überführt.

Die Solvolyse kann als Hydrolyse, als Alkoholyse oder auch als Hydrazinolyse durchgeführt werden. Die Hydrolyse wird mit Wasser, gegebenenfalls in einem mit Wasser mischbaren Lösungsmittel, durchgeführt. Die Alkoholyse wird üblicherweise mit einem Niederalkanol, z.B. Methanol oder Aethanol, vorzugsweise in Gegenwart von Wasser und eines organischen Lösungsmittels, wie eines Niederalkancarbonsäure-niederalkylesters, z.B. Essigsäureäthylester, vorzugsweise bei Raumtemperatur, wenn notwendig unter Kühlen oder Erwärmen durchgeführt. Die Hydrazinolyse wird auf konventionelle Weise mit einem substituierten Hydrazin, z.B. mit Phenyl- oder einem Nitrophenylhydrazin, wie 2-Nitrophenylhydrazin, 4-Nitrophenylhydrazin, oder 2,4-Dinitrophenylhydrazin, das bevorzugt in etwa äquimolarer Menge eingesetzt wird, in einem organischen Lösungsmittel, wie einem Aether, z.B. Tetrahydrofuran, Dioxan, Diäthyläther, einem aromatischen Kohlenwasserstoff, wie Benzol oder Toluol, einem halogenierten Kohlenwasserstoff, wie Methylenchlorid, Chlorbenzol oder Dichlorbenzol, einem Ester, wie Aethylacetat, und dergleichen, bei Temperaturen zwischen etwa Raumtemperatur und etwa 65°C durchgeführt. Die $\alpha$-Ketoverbindung der Formel XII braucht nicht notwendigerweise isoliert zu werden. Führt man z.B. die Spaltung des Ozonids in Gegenwart eines Solvolysemittels, wie z.B. Wasser, durch, so kann direkt eine Verbindung der Formel IVa erhalten werden.

Optisch aktive cis-, trans- und cis, trans-Verbindungen der Formel IIIa können auch nach dem folgenden Reaktionsschema erhalten werden:

REAKTIONSSCHEMA 3

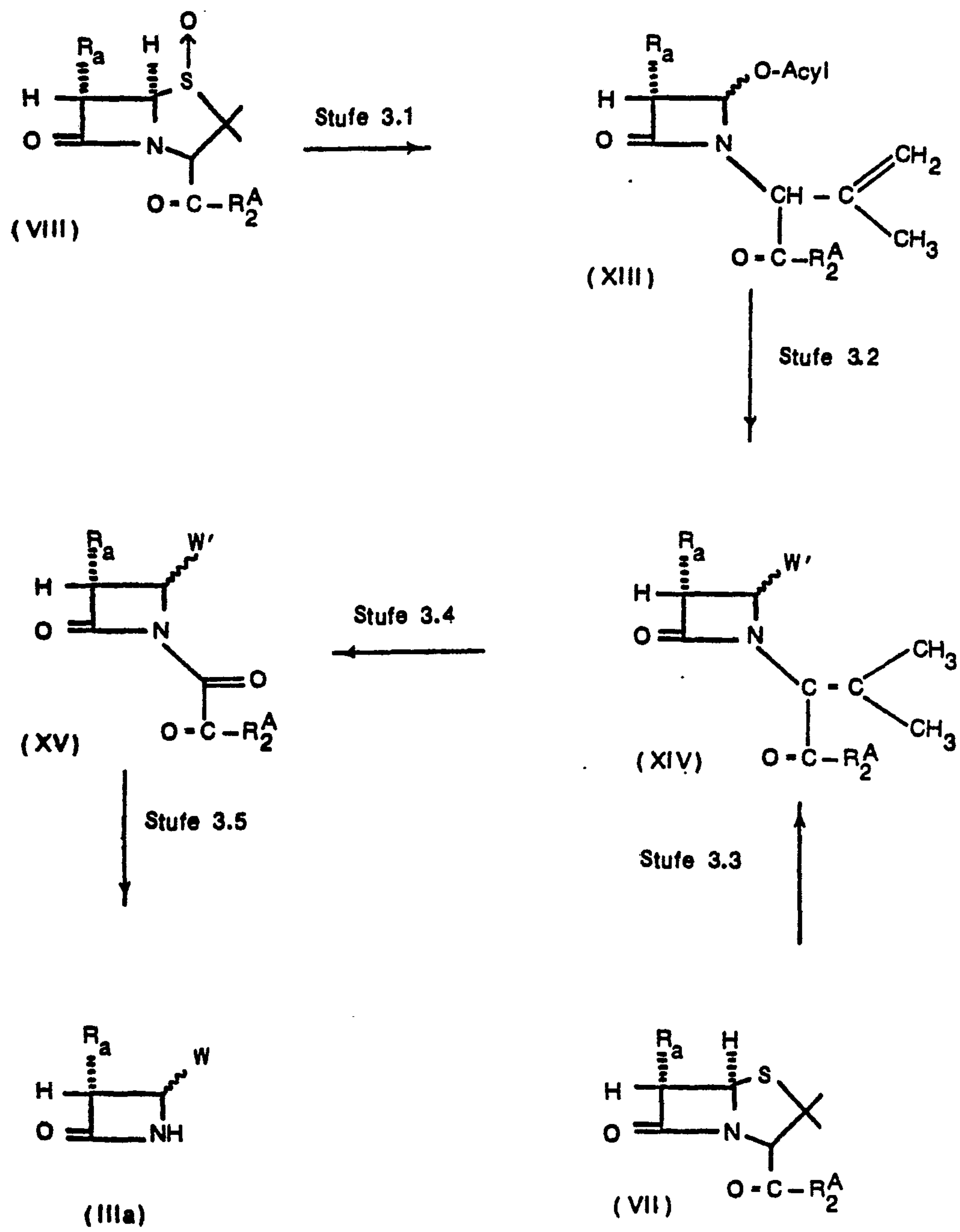


W' = Acyloxy oder Halogen

*Stufe 3.1:* Eine 3-Methylenbuttersäureverbindung der Formel XIII wird erhalten, indem man ein 1-Oxid einer Penicillansäureverbindung der Formel VIII in Gegenwart eines Triniederalkylphosphits mit einer organischen Carbonsäure Acyl-OH behandelt, gewünschtenfalls in einer erhaltenen Verbindung eine Gruppe $R_a$ in eine andere Gruppe $R_a$ umwandelt, und/oder aus einer erhaltenen cis, trans-Verbindung die cis- und/oder die trans-Verbindung isoliert.

Als Triniederalkylphosphit ist beispielsweise Trimethylphosphit geeignet. Als organische Carbonsäure Acyl-OH kommt beispielsweise eine Carbonsäure $R_1$—COOH in Frage, worin $R_1$ Wasserstoff oder einen der genannten, über ein Kohlenstoffatom gebundenen organischen Reste, z.B. einen der genannten Niederalkyl-, Cycloalkyl-, Cycloalkylniederalkyl-, Phenyl- oder Phenylniederalkylreste, darstellt. Bevorzugt sind Niederalkancarbonsäuren, inklusive Ameisensäure, insbesondere Essigsäure.

Die Reaktion findet, analog A. Suarato et al., Tetrahedeon Letters, *42*, 4059—4062, 1978, in einem inerten organischen Lösungsmittel, z.B. einem Kohlenwasserstoff, wie Benzol, Toluol oder Xylol, oder einem ätherartigen Lösungsmittel, wie Dioxan oder Tetrahydrofuran, oder einem Lösungsmittelgemisch, bei erhöhter Temperatur, etwa bis zur Rückflusstemperatur des verwendeten Lösungsmittels, etwa bei 50 bis 150°C, bevorzugt bei etwa 80° bis etwa 100°C, statt.

Bei der Reaktion entsteht ein Gemisch der cis- und trans-Verbindungen. Durch übliche Trennmethoden, wie Kristallisieren oder Chromatographieren, kann die cis- und/oder die trans-Verbindung in reiner Form erhalten werden.

*Stufe 3.2:* Eine 3-Methylcrotonsäureverbindung der Formel XIV worin W' Acyloxy bedeutet, wird erhalten, indem man eine 3-Methylenbuttersäureverbindung der Formel XIII durch Behandeln mit einem geeigneten basischen Mittel isomerisiert, und gewünschtenfalls in einer erhaltenen Verbindung eine Gruppe $R_a$ in eine andere Gruppe $R_a$ umwandelt, und/oder gewünschtenfalls aus einer erhaltenen cis, trans-Verbindung die cis- und/oder die trans-Verbindung isoliert.

Die basische Isomerisierung wird wie in der Stufe 2.3 beschrieben durchgeführt. Die gewünschtenfalls durchzuführende nachträgliche Auftrennung in reine Verbindungen wird wie unter Stufe 3.1 beschrieben durchgeführt.

*Stufe 3.3:* Eine Verbindung der Formel XIV, worin W' Halogen bedeutet, wird erhalten, indem man eine Penicillansäureverbindung der Formel VII mit einem positive Halogenionen liefernden Halogenierungsmittel und, falls erforderlich, ein gegebenenfalls erhaltenes Zwischenprodukt mit einer Base behandelt, und gewünschtenfalls in einer erhaltenen Verbindung der Formel XIV eine Gruppe $R_a$ in eine andere Gruppe $R_a$ überführt und/oder aus einer erhaltenen cis, trans-Verbindung die cis- und/oder die trans-Verbindung isoliert.

Positive Halogenionen liefernde Halogenierungsmittel sind beispielsweise elementare Halogene, wie Chlor, Brom oder Jod, gemischte Halogene, wie BrCl,Cl J oder Br J, Sulfurylhalogenide, wie Sulfurylchlorid oder Sulfurylbromid, N-Halogenamide oder N-Halogenimide, wie N-Chloracetamid, N-Cromacetamid, N-Chlorsuccinimid, N-Bromsuccinimid oder N,N'-Dibromhydantoine, oder organische Hypohalogenite, insbesondere Niederalkanoylhypohalogenite, wie Acetylhypochlorit, Propionylhypochlorit, Butyrylhypochlorit, Acetylhypobromit, Propionylhypobromit, Butyrylhypobromit, und dergleichen.

Die Reaktion wird analog USP 3.920.696 oder St. Kukolja, Journ. Am. Chem. Soc. *93*, 6267 (1971), in einem inerten, aprotischen Lösungsmittel, insbesondere in einem halogenierten Kohlenwasserstoff, wie Methylenchlorid oder Tetrachlorkohlenstoff, bei Temperaturen zwischen etwa −80° bis etwa +80°C, bevorzugt bei etwa −76°C bis etwa Raumtemperatur, durchgeführt.

Das Molverhältnis von Halogenierungsmittel zur Verbindung der Formel VII liegt zwischen 1:1 bis 3:1 oder auch höher. Wenn das Molverhältnis etwa 1:1 ist, wird als Zwischenprodukt eine Verbindung der Formel

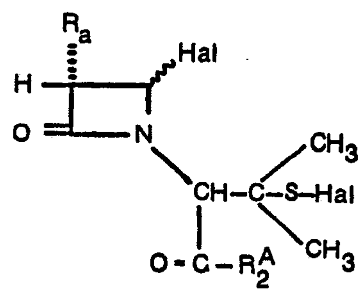

(XIV')

erhalten, die durch Behandeln mit einer Base, wie einem tertiären Amin, z.B. Triäthylamin, in die Verbindung der Formel XIV übergeführt werden kann. Bei Verwendung von mindestens 2 Mol Halogenierungsmittel oder mehr pro Mol Penicillansäureverbindung wird die gewünschte Verbindung der Formel XIV auch ohne nachträgliche Behandlung mit Base erhalten.

Bei der Ringöffnungsreaktion entsteht ein Gemisch der cis- und trans-Verbindung, wobei die trans-Verbindung bevorzugt gebildet wird. Durch übliche Trennmethoden, wie Kristallisieren oder Chromatographieren, können die cis- und/oder die trans-Verbindungen in reiner Form erhalten werden.

*Stufe 3.4:* Eine 2-Oxoessigsäureverbindung der Formel XV worin W' Acyloxy oder Halogen bedeutet, wird erhalten, indem man eine Verbindung der Formel XIV ozonisiert und das gebildete Ozonid reduktiv zur Oxogruppe spaltet, und gewünschtenfalls in einer erhaltenen Verbindung eine Gruppe $R_a$ in eine andere Gruppe $R_a$ umwandelt, und/oder gewünschtenfalls aus einer erhaltenen cis, trans-Verbindung die cis- und/oder die trans-Verbindung isoliert.

Die Ozonisierung und die Reduktion des gebildeten Ozonids wird wie in der Stufe 2.5 beschrieben durchgeführt. Die gewünschtenfalls nachträgliche Auftrennung in reine Verbindungen wird wie unter Stufe 3.1 beschrieben durchgeführt.

*Stufe 3.5:* Eine Verbindung der Formel IIIa, worin W die unter Formel III angegebene Bedeutung hat, wird erhalten, indem man eine Verbindung der Formel XV, worin W' Acyloxy oder Halogen bedeutet, solvolysiert, und gewünschtenfalls in einer erhaltenen Verbindung eine Gruppe $R_a$ in eine andere

Gruppe $R_8$ umwandelt, und/oder gewünschtenfalls eine Gruppe W' in eine andere Gruppe W' oder W überfuhrt, und/oder gewünschtenfalls eine erhaltene cis-Verbindung zur entsprechenden trans-Verbindung isomerisiert, und/oder aus einer erhaltenen cis, trans-Verbindung die cis- und/oder die trans-Verbindung isoliert.

Die Solvolyse wird wie in Stufe 2.6 beschrieben durchgeführt. Wenn W Halogen ist, wird bevorzugt die Hydrazinolyse angewendet. Auch hier ist es nicht nötig das Zwischenprodukt der Formel XV nach der Ozonisierungs- und Reduktionsreaktion zu isolieren, sondern man kann es in situ herstellen und unmittelbar solvolysieren.

In einer erhaltenen Verbindung der Formel IIIa, worin W Acyloxy oder Halogen bedeutet, kann diese Gruppe durch nucleophilen Austausch in eine andere Gruppe W übergeführt werden, wobei die eintretende Gruppe W nucleophiler sein muss als die austretende. Dieser Austausch kann analog der Stufe 1.1, z.B. durch Behandeln mit einem Alkalimetallsalz, wie einem Natrium- oder Kaliumsalz einer Säure H—W, durchgeführt werden.

Bei diesem Austausch von W gegen ein anderes W, wie auch bei dem nachfolgenden Austausch gegen eine Gruppe $R_1$—C(=Z')—S— gemäss Stufe 1.1, werden die optisch aktiven trans-Verbindungen der Formel IIIa bzw. IVa im Ueberschuss erhalten, gleichgültig, ob man von einer cis- oder trans- Verbindung ausgeht.

Durch Isomerisierung, z.B. durch Behandeln mit einer milden Lewissäure in katalytischen Mengen, kann eine erhaltene cis- in eine trans-Verbindung übergeführt werden. Die Isomerisierung mit einer Lewissäure findet in einem inerten Lösungsmittel bei erhöhter Temperatur, etwa bei 50° bis 150°C, z.B. unter Rückfluss in Benzol, statt.

In den Verbindungen II, IV bis XV, IIIa und IVa kann eine Gruppe $R_8$, $R_1$ bzw. $R_2^A$ nach an sich bekannten Methoden in eine andere Gruppe $R_8$, $R_1$ bzw. $R_2^A$ übergeführt werden, wobei unter Berücksichtigung der verschiedenen funktionellen Gruppen, die gleichen Methoden zur Anwendung gelangen können, wie zur Umwandlung dieser Substituenten in den Verbindungen der Formel I angegeben ist.

In den Verbindungen IV (incl. IVa) bis VI und II kann eine gegebenenfalls substituierte Methylidengruppe Z' durch Ozonisierung und anschliessende Reduktion des gebildeten Ozonids, gemäss dem in der Stufe 2.5 beschriebenen Verfahren, in eine Oxogruppe Z übergeführt werden.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zu enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die wenn erwünscht, weitere pharmakologische wertvolle Stoffe enthalten können, werden in an sich bekannter Weise, z.B. mittels konventioneller Misch-, Granulier-, Dragier-, Lösungs- oder Lyophilisierungsverfahren hergestellt und enthalten von etwa 0,1% bis 100%, insbesondere von etwa 1% bis etwa 50%, Lyophilisate bis zu 100% des Aktivstoffes.

Im Zusammenhang mit der vorliegenden Beschreibung enthalten mit "nieder" bezeichnete organische Reste, sofern nicht ausdrücklich definiert, bis zu 7, vorzugsweise bis zu 4 Kohlenstoffatome; Acylreste enthalten bis zu 20, vorzugsweise bis zu 12 und in erster Linie bis zu 7 Kohlenstoffatomen.

Die folgenden Beispiele dienen zur Illustration der Erfindung; Temperaturen werden in Celsiusgraden angegeben. Folgende Abkürzung wird benutzt: DC = Dünnschichtchromatogramm auf Silicagel.

## Beispiel 1

4-Acetylthio-3-methyl-2-oxo-azetidin (racemisches cis- und trans-Verbindung

Eine Lösung von 438 mg (3.06 mM) 4-Acetoxy-3-methyl-azetidin-2-on (hergestellt gemäss K. Clauss et al., Lieb. Ann. Chem., *1974*, 539; racemische Mischung von cis- und trans-Isomer im Verhältnis 3:1; F. 53—65°) in 1.13 ml Wasser und 0,27 ml Aceton wird bei Raumtemperatur unter Stickstoffatmosphäre tropfenweise mit einer Lösung von 0,33 ml Thioessigsäure in 4,5 ml 1N

Natriumhydroxidlösung versetzt und bei der gleichen Temperatur 3 Stunden gerührt. Die Reaktionsmischung wird erschöpfend mit Methylenchlorid extrahiert. Die kombinatierten organischen Phasen werden über Natriumsulfat getrocknet und im Vakuum eingedampft. Der Rückstand wird an Silicagel mit Toluol-Aethylacetat 4:1 bis 3:2 chromatographiert und ergibt zunächst die reine trans-Verbindung, dann eine Mischung der cis und trans-Isomeren der Titelverbindung und anschliessend die reine cis-Verbindung. DC: Rf = 0,31 (cis-Isomer); 0,36 (trans-Isomer) (Toluol-Aethylacetat 2:3); IR-Spektrum ($CH_2Cl_2$): Absorptionsbanden bei 2,95; 5,6; 5,87; 8,65; 8,85; 10,45 $\mu$. NMR-Spektrum (in $CDCl_3$/100 Mc; in ppm): *cis-Verbindung*: 6,2, 1H, breit (Austausch mit $D_2O$); 5,45, 1H, d(J = 5,5 Hz; 3,5—3,9, 1H, m; 2,4, 3H,s; 1,3, 3H,d; *trans-Verbindung:* 6,5, 1H, breit (Austausch mit $D_2O$); 4,93; 1H, d (J ~ 2,5 Hz); 3,0—3,4, 1H, m; 2,4, 3H,s; 1,42, 3H, d.

Beispiel 2

2-(4-Acetylthio-3-methyl-2-oxo-1-azetidinyl)-2-hydroxyessigsäure-p-nitrobenzylester (racemisches cis, trans-Gemisch)

Bei Raumtemperatur werden 129 mg (0,81 mM) 4-Acetylthio-3-methyl-2-oxo-azetidin (racemisches cis-, trans-Gemisch) mit einer Lösung von 500 mg 2 - Aethoxy - 2 - hydroxy - essigsäure - p - nitrobenzylester in einer Mischung von 10 ml Toluol und 2,5 ml Dimethylformamid versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an Silikagel mit Toluol-Aethylacetat 9:1 bis 8:2 chromatographiert. Nach Elution von nicht umgesetztem 2-Aethoxy-2-hydroxy-essigsäure-p-nitrobenzylester wird eine Mischung der cis-trans Isomeren der Titelverbindung mit den folgenden physikochemischen Eigenschaften eluiert: DC: Rf = 0,38 (Toluol-Aethylacetat 2:3); IR-Spektrum ($CH_2Cl_2$): Absorptionsbanden bei 2,85; 5,62; 5,7; 5,9; 6,2; 6,55; 7,4 und 8,25 $\mu$.

Beispiel 3

2-(4-Acetylthio-3-methyl-2-oxo-1-azetidinyl)-2-triphenyl-phosphoranyliden-essigsäure-p-nitrobenzylester (racemisches cis, trans-Gemisch)

a) Eine Lösung von 225 mg 2 - (4 - Acetylthio - 3 - methyl - 2 - oxo - 1 - azetidinyl) - 2 - hydroxyessigsäure - p - nitrobenzylester (racemisches cis, trans-Gemisch) in 5 ml absolutem Dioxan wird zu einer bereits 30 Minuten gerührten Lösung von 1 g Poly-Hünigbase in 2,5 ml absolutem Dioxan gegeben. Nach Zugabe einer Lösung von 0,175 ml Thionylchlorid in 1,5 ml absolutem Dioxan wird die Reaktionsmischung 100 Minuten bei Raumtemperatur und unter Stickstoff gerührt. Die Poly-Hünigbase wird abfiltriert, mit Dioxan gewaschen und das Filtrat im Vakkum eingedampft. DC des rohen 2 - (4 - Acetyl - thio - 3 - methyl - 2 - oxo - 1 - azetidinyl) - 2 - chloressigsäure - p - nitrobenzylesters (racemisches cis, trans-Gemsich): Rf = 0,62 (Toluol-Aethylacetat 2:3).

b) Der erhaltene rohe 2 - (4 - Acetylthio - 3 - methyl - 2 - oxo - 1 - azetidinyl) - 2 - chloressigsäure - p - nitrobenzylester wird in 12 ml absolutem Dioxan gelöst, mit 1 g Poly-Hünigbase versetzt, 30 Minuten gerührt, dann mit 31 mg Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vacuum eingedampft. Der Rückstand wird an Silikagel mit Toluol-Aethylacetate chromatographiert und ergibt ein cis-trans-Gemisch der Titelverbindung mit den folgenden physiko-chemischen Eigenschaften; DC: Rf = 0,28 (Toluol-Aethylacetat 2:3); IR-Spektrum ($CH_2Cl_2$): Absorptionsbanden bei 5,67; 5,9; 6,15; 6,55; 6,95; 7,4; 9,0 und 9,25 $\mu$.

Beispiel 4

2,6-Dimethyl-2-penem-3-carbonsäure-p-nitrobenzylester (racemisches cis, trans-Gemisch)

Eine Lösung von 118 mg 2 - [4 - Acetylthio - 3 - methyl - 2 - oxo - 1 - azetidinyl] - 2 - triphenylphosphoranyliden - essigsäure - p - nitrobenzylester (racemisches cis, trans-Gemisch) in 50 ml absolutem Toluol wird mit einer katalytischen Menge p-Hydroxychinon versetzt und unter Stickstoff 48 Stunden bei 90° gerührt. Das Lösungsmittel wird im Vakuum abgedampft und der Rückstand an Silikagel mit Toluol- Aethylacetat 19:1 chromatographiert. Man erhält ein cis- trans Gemisch (1:4) der Titelverbindung in Form eines gelblichen Oeles mit folgenden physikochemischen Eigenschaften: DC: Rf = 0,59 (Toluol-Aethylacetate 2:3); IR-Spektrum ($CH_2Cl_2$): Absorptionsbanden bei 5,6; 5,85; 6,3; 6,55; 7,4; 7,6; 8,3; 9,25 $\mu$; NMR-Spektrum (in $CDCl_3$/100 Mc; in ppm): 8,4—8,2, 2H, 7,75—7,76, 2H, m; 5,7—5,2, 3H, m; 4,1—3,6, 1H, m; 2,4, 2,43, 3H, 2s; 1,6—1,4, 3H, 2d.

Beispiel 5

2,6-Dimethyl-2-penem-3-carbonsäure (racemisches cis, trans-Gemisch)

Eine Lösung von 47 mg (0,14 mM) 2,6-Dimethyl-2-penem-3-carbonsäure-p-nitrobenzylester (racemisches cis, trans-Gemisch 1:4) in 3 ml absolutem Aethylacetat wird mit 2 ml 0,2 M wässriger Natriumhydrogencarbonatlösung und 100 mg 10%-igem Palladium/Kohl Katalysator versetzt und bei Normaldruck während 40 Minuten unter Wasserstoff gerührt. Die Hydriermischung wird über Diatomeenerde vom Katalysator abfiltriert, mit 0,2 M Natriumhydrogencarbonatlösung und mehrere

Male mit Aethylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung (cis, trans-Gemisch ~ 1:4) hat folgende physikochemischen Eigenschaften: DC: RF = 0,28 (Toluol-Aethylacetat-Essigsäure 60:40:5; IR-Spektrum ($CH_2CL_2$): Absorptionsbanden bei 3,5, 5,6, 5,95, 6,3 $\mu$; NMR-Spektrum (DMSO d6/100 Mc; in ppm): 5,65, 1H, q; 3,3—3,9, 2H, m (+$H_2O$); 2,28, 3H, s, Schmelzpunkt 119°.

Beispiel 6

2,6-Dimethyl-2-penem-3-carbonsäure Natriumsalz (racemisches cis, trans-Gemisch)

Eine Lösung von 50 mg 2,6-Dimethyl-2-penem-3-carbonsäure in der äquivalenten Menge wässriger Natriumhydrogencarbonatlösung wird im Vakuum eingedampft und am Hochvakuum getrocknet.

Beispiel 7

4-Acetylthio-3-methyl-2-oxo-azetidin (racemische trans-Verbindung)

Eine Lösung von 2 g 4-Acetoxy-3-methyl-azetidin-2-on (hergestellt gemäss K. Clauss et al., Lieb. Ann. Chem., *1974*, 539; racemische Mischung von cis- und trans-Isomer im Verhältnis 3:1; F. 53—65°) in 5,16 ml Wasser und 1,25 ml Aceton wird bei Raumtemperatur unter Stickstoffatmosphäre tropfenweise mit einer Lösung von 1,5 ml Thioessigsäure in 20,5 ml 1N Natriumhydroxidlösung versetzt und bei gleichen Temperatur 3 Stunden gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 150 g Silicagel mit Toluol-Aethylacetat 9:1 chromatographiert und ergibt das fast reine trans-Isomere der Titelverbindung mit den folgenden physikochemischen Eigenschaften: DC: 0,38 (Toluol-Aethylacetat 2:3); IR-Spektrum ($CH_2Cl_2$): Absorptionsbanden bei 2,95; 5,6; 5,87; 7,37; 7,45; 8,62; 8,82 $\mu$. NMR-Spektrum (in $CDCl_3$/100 Mc; in ppm): 6,55, 1H, m (Austausch mit $D_2O$); 4,9, 1H, d, J = 2 Hz, 3,35—3,05, 1H,m; 2,38, 3H, s; 1,4, 3H,d, J = 7Hz. Nachfolgend wird ein Gemisch des cis- und trans-Isomeren isoliert.

Beispiel 8

2-(4-Acetylthio-3-methyl-2-oxo-1-azetidinyl)-2-hydroxyessigsäure-p-nitrobenzylester (racemische trans-Verbindung)

Bei Raumtemperatur werden 1,35 g (8,49 mM) 4-Acetylthio-3-methyl-2-oxo-azetidin (racemische trans-Verbindung) mit einer Lösung von 5 g 2-Aethoxy-2-hydroxyessigsäure-p-nitrobenzylester in einer Mischung von 100 ml Toluol und 25 ml Dimethylformamid versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an 100 g Silikagel mit Toluol-Aethylacetat 9:1 chromatographiert. Nach Elution von nicht umgesetztem 2-Aethoxy-2-hydroxy-essigsäure-p-nitrobenzylester wird die Titelverbindung mit den folgenden physikochemischen Eigenschaften eluiert: DC: Rf = 0,33 (Toluol-Aethylacetat 2:3); IR-Spektrum ($CH_2Cl_2$): Absorptionsbanden bei 2,85; 5,6; 5,7; 5,87; 6,2; 6,52; 7,4, 8,28; 9—9,2 $\mu$.

Beispiel 9

2-(4-Acetylthio-3-methyl-2-oxo-1-azetidinyl)-2-triphenylphosphoranyliden-essigsäure-p-nitrobenzylester (racemische trans-Verbindung)

a) Eine Lösung von 3 g 2-(4-Acetylthio-3-methyl-2-oxo-1-azetidinyl)-2-hydroxyessigsäure-p-nitrobenzylester (racemische trans-Verbindung) in 75 ml absolutem Dioxan wird zu einer bereits 30 Minuten gerührten Lösung von 13,5 g Poly-Hünigbase in 35 ml absolutem Dioxan gegeben. Nach Zugabe einer Lösung von 2,4 ml Thionylchlorid in 22,4 ml absolutem Dioxan wird die Reaktionsmischung 100 Minuten bei Raumtemperatur und unter Stickstoff gerührt. Die Poly-Hünigbase wird abfiltriert, mit Dioxan gewaschen und das Filtrat im Vakuum eingedampft. DC des rohen 2-(4-Acetylthio-3-methyl-2-oxo-1-azetidinyl)-2-chloressigsäure-p-nitrobenzylesters (racemische trans-Verbindung): Rf = 0,59 (Toluol-Aethylacetat 2:3).

b) Der erhaltene rohe 2-(4-Acetylthio-3-methyl-2-oxo-1-azetidinyl)-2-chloressigsäure-p-nitrobenzylester wird in 175 ml absolutem Dioxan gelöst, mit 13,5 g Poly-Hünigbase versetzt, dann mit 4,2 mg Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an Silikagel mit Toluol-Aethylacetat chromatographiert und ergibt die trans-Titelverbindung mit den folgenden physiko-chemischen Eigenschaften: DC: Rf = 0,24 (Toluol-Aethylacetat 2:3); IR-Spektrum ($CH_2Cl_2$): Absorptionsbanden bei 5,67; 5,9; 6,15; 6,55; 7,4; 9.0 $\mu$.

Beispiel 10

2,6-Dimethyl-2-penem-3-carbonsäure-p-nitrobenzylester (racemische trans-Verbindung)

Eine Lösung von 363 mg 2-[4-Acetylthio-3-methyl-2-oxo-1-azetidinyl]-3-triphenylphosphorayliden-essigsäure-p-nitrobenzylester (racemische trans-Verbindung) in 180 ml absolutem Toluol wird

mit einer katalytischen Menge p-Hydroxychinon versetzt und unter Stickstoff 48 Stunden bei 90° gerührt. Das Lösungsmittel wird im Vakuum abgedampft und der Rückstand an 20 g Silikagel mit Toluol-Aethylacetat 19:1 chromatographiert. Man erhält die trans-Titelverbindung in Form gelblicher Kristalle vom Schmelzpunkt 141—143° und mit folgenden physikochemischen Eigenschaften: DC: Rf = 0,6 (Toluol-Aethylacetat 2:3); IR-Spektrum ($CH_2Cl_2$): Absorptionsbanden bei 3,4; 5,57; 5,82; 6,27; 6,55; 7,4; 7,6; 8,3; 9,22 $\mu$; NMR-Spektrum (in $CDCl_3$/100 Mc; in ppm): 8,25—8,15, 2H, m; 7,65—7,56, 2H, m; 5,55—5,12, 3H, m + d (J = 1,5 Hz); 3,9—3,6, 1H, m; 2,36, 3H, s; 1,5, 3H, d.

Beispiel 11

2,6-Dimethyl-2-penem-3-carbonsäure (racemische trans-Verbindung)

Eine Lösung von 80 mg (0,24 mM) 2,3-Dimethyl-2-penem-3-carbonsäure-p-nitrobenzylester (racemische trans-Verbindung) in 5 ml absolutem Aethylacetat wird mit 3 ml 0,2 N wässriger Natriumhydrogencarbonatlösung und 150 mg 10%-igem Palladium/Kohle Katalysator versetzt und bei Normaldruck während 50 Minuten unter Wasserstoff gerührt. Die Hydriermischung wird über Diatomeenerde vom Katalysator abfiltriert, mit 0,2 N Natriumhydrogencarbonatlösung und mehrere Male mit Aethylacetat nachgewaschen. Die wässrigen Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: Schmelzpunkt 119° (Zersetzung); DC: Rf = 0,3 (Toluol-Aethylacetat-Essigsäure 60:40:5); IR-Spektrum (KBr): Absorptionsbanden bei 3,3—3,5; 5,62; 6,0; 6,35; 6,95; 7,55; 7,85 $\mu$; NMR—Spektrum (DMSO d6/100 Mc; in ppm): 5,38, 1H, d, (J=1,5 Hz); 3,7, 1H, m; 3,4, 1H, m (Austausch mit $D_2O$); 2,28, 3H, s; 1,34, 3H, d.

Beispiel 12

4-Acetylthio-3-isopropyl-2-oxo-azetidin (racemische trans-Verbindung)

Eine Lösung von 750 mg (4,38 mM) 4-Acetoxy-3-isopropyl-azetidin-2-on (racemische Mischung von cis- und trans-Isomer im Verhältnis 1:3) in 3,6 ml Wasser und 0,9 ml Aceton wird bei Raumtemperatur unter Stickstoffatmosphäre tropfenweise mit einer Lösung von 0,52 ml Thioessigsäure in 7 ml 1N Natriumhydroxidlösung versetzt und bei der gleichen Temperatur 75 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 40 g Silicagel mit Toluol-Aethylacetat 4:1 chromatographiert und ergibt die trans-Titelverbindung. DC: Rf = 0,4 (Toluol-Aethylacetat 2:3); IR-Spektrum ($CH_2Cl_2$): Absorptionsbanden bei 2,95; 3,37; 5,62; 5,87; 8,8 $\mu$. NMR-Spektrum (in $CDCl_3$/100 Mc; in ppm): 6,35, 1H, m (Austaisch mit $D_2O$); 5,04, 1H, d (J = 2,5 Hz); 3,0, 1H, m; 2,37, 3H, s; 2,1, 1H, m; 1,05, 3H, m.

Das Ausgangsmaterial wird wie folgt hergestellt:

a) Eine Mischung von 172,28 g (216,5 ml; 2 M) Isovaleraldehyd, 306 g (283 ml) Acetanhydrid und 24 g frisch geschmolzenes Kaliumacetat wird während 17 Stunden unter Rückfluss gekocht. Die abgekühlte Mischung wird mit 5%-iger Natriumcarbonatlösung gewaschen, bis die organische Phase neutral reagiert. Nach waschen mit Wasser und trocknen über Magnesiumsulfat wird das erhaltene Oel destilliert. Man erhält das 3-Methyl-1-butenylacetat (cis, trans-Gemisch 1:4) vom Siedepunkt 135—140°/760 mmHg.

b) Eine Lösung von 12,8 g (0,1 M) 3-Methyl-1-butenyl-acetate (cis,trans-Gemisch 1:4) in 40 ml absolutem Methylenchlorid wird bei Raumtemperatur und unter Stickstoff tropfenweise mit einer Lösung von 8,72 ml N-Chlorsulfonyl-isocyanat in 10 ml absolutem Methylenchlorid versetzt. Nach 4 Stunden wird die Reaktionsmischung langsam auf eine Mischung von 10 ml Wasser, 45 g Eis, 24 g Natriumbicarbonat und 8,3 g Natriumsulfit gegossen, wobei die Temperatur durch gelegentliche Zugabe von Eis zwischen 0° und 5° gehalten wird. Nach etwa 30 Minuten reagiert die organische Phase neutral, worauf sie abgetrennt wird. Die wässrige Phase wird mit Methylenchlorid extrahiert. Die organischen Phasen werden vereinigt, über Natriumsulfat getrocknet und im Vakuum eingedampft. Der Rückstand wird an Silikagel mit Toluol/Aethylacetat chromatographiert und ergibt ein cis,trans-Gemisch von 4-Acetoxy-3-isopropyl-azetidin-2-on im Verhältnis von ca. 1:3. DC: Rf = 0,3 (Toluol-Aethylacetat 2:3); IR-Spektrum (in Methylenchlorid): Absorptionsbanden bei 2,95; 3,37; 5,6; 5,72; 7,32; 8,1; 9,7; 10,2 $\mu$; NMR-Spektrum ($CDCl_3$/100 Mc; in ppm): 6,75, 1H, m (Austausch mit $D_2O$); 5,85, d, J = 4,5 Hz (cis) und 5,6 d, J = 1,5 Hz (trans), 1H; 3,03, 1H, m; 2,1, 3H, 2s; 2,3—1,8, 1H, m; 1,1, 6H, m.

Beispiel 13

2-(4-Acetylthio-3-isopropyl-2-oxo-1-azetidinyl)-2-hydroxy-essigsäure-p-nitrobenzylester (racemische trans-Verbindung)

Bei Raumtemperatur wird eine Lösung von 616 mg (3,3 mM) 4-Acetylthio-3-isopropyl-2-oxo-azetidin (racemische trans-Verbindung) in 48 ml Toluol und 10,5 ml Dimethylformamid mit 1,9 g 2-Aethoxy-2-hydroxy-essigsäure-p-nitrobenzylester versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum eingedampft. Der Rückstand wird im Hochvakuum getrocknet und an 60 g Silikagel mit Toluol-Aethylacetat 9:1 chromatographiert. Man erhält die beiden mit nicht umgesetztem 2-Aethoxy-2-hydroxy-essigsäure-p-nitrobenzylester etwas verunreinigten trans-Isomeren der Titelverbindung mit den folgenden physikochemiscnen Eigenschaften: DC: Rf = 0,4 und 0,37 (Toluol-Aethylacetat 2:3); IR-Spektrum ($CH_2Cl_2$); Absorptionsbanden bei 5,62; 5,68; 6,55; 7,42 $\mu$.

Beispiel 14

2-(4-Acetylthio-3-isopropyl-2-oxo-1-azetidinyl)-2-triphenylphosphoranyliden-essigsäure-p-nitrobenzylester (racemische trans-Verbindung)

a) Eine Lösung von 1,175 g 2-(4-Acetylthio-3-isopropyl-2-oxo-1-azetidinyl)-2-hydroxyessigsäure-p-nitrobenzylester (racemische trans-Verbindung) in 21 ml absolutem Dioxan wird zu einer bereits 30 Minuten gerührten Lösung von 3,8 g Poly-Hünigbase in 10 ml absolutem Dioxan gegeben. Nach tropfenweiser Zugabe einer Lösung von 0,67 ml Thionylchlorid in 6,3 ml absolutem Dioxan wird die Reaktionsmischung 90 Minuten bei Raumptemperatur und unter Stickstoff gerührt. Die Poly-Hünigbase wird abfiltriert, mit Dioxan gewaschen und das Filtrat im Vakuum eingedampft. Der erhaltene rohe 2-(4-Acetylthio-3-isopropyl-2-oxo-1-azetidinyl)-2-chloressigsäure-p-nitrobenzylester (racemische, trans-Verbindung) kann ohne weitere Reinigung in die nächste Stufe eingesetzt werden.

b) Der erhaltene rohe 2-(4-Acetylthio-3-isopropyl-2-oxo-1-azetidinyl)-2-chloressigsäure-p-nitrobenzylester wird in 50 ml absolutem Dioxan gelöst, mit 3,8 g Poly-Hünigbase versetzt, 30 Minuten gerührt, dann mit 1,18 g Triphenylphosphin versetzt und 15 Stunden bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an 60 g Silikagel mit Toluol-Aethylacetat 7:3 chromatographiert und ergibt die trans-Titelverbindung mit den folgenden physiko-chemischen Eigenschaften: DC: Rf = 0,25 (Toluol-Aethylacetat 2:3); IR-Spektrum ($CH_2Cl_2$): Absorptionsbanden bei 5,7; 5,9; 6,17; 6,55; 7,42; 9.05 $\mu$.

Beispiel 15

2-Methyl-6-isopropyl-2-penem-3-carbonsäure-p-nitrobenzylester (racemisch trans-Verbindung)

Eine Lösung von 660 mg 2-[4-Acetylthio-3-isopropyl-2-oxo-1-azetidinyl]-2-triphenylphosphoranylidenessigsäure-p-nitrobenzylester (racemische trans-Verbindung in 300 ml absolutem Toluol wird mit einer katalytischen Menge p-Hydroxychinon versetzt und unter Stickstoff 48 Stunden bei 90° gerührt. Das Lösungsmittel wird im Vakuum abgedampft und der Rückstand an 30 g Silikagel mit Toluol-Aethylacetat 19:1 chromatographiert. Man erhält die trans-Titelverbindung nach Kristallisation aus Diäthyläther-Methylenchlorid in Form farbloser Kristalle mit folgenden physikochemischen Eigenschaften: Schmelzpunkt: 138—139°; DC: Rf = 0,59 (Toluol-Aethylacetat 2:3): IR-Spektrum ($CH_2Cl_2$): Absorptionsbanden bei 5,57; 5,82; 6,27; 6,55; 7,4; 7,6 $\mu$; NMR-Spektrum (in $CDCl_3$/100 Mc; in ppm): 8,3—8,2, 2H, m; 7,5—7,4, 2H, m; 5,75—5.1, 3H, m; 3,6—3,5, 1H, dd, J = 8 und 1,5 Hz; 2,35, 3H, s; 1,07, 6H, m.

Beispiel 16

2-Methyl-6-isopropyl-2-penem-3-carbonsäure (racemische trans-Verbindung)

Eine Lösung von 100 mg 2-Methyl-6-isopropyl-2-penem-3-carbonsäure-p-nitrobenzylester (racemische trans-Verbindung) in 7 ml absolutem Aethylacetat wird mit 4 ml 0,2 N wässriger Natriumhydrogencarbonatlösung und 50 mg 10%-igem Palladium/Kohle Katalysator versetzt und bei Normaldruck während 30 Minuten unter Wasserstoff gerührt. Die Hydriermischung wird über Diatomeenerde vom Katalysator abfiltriert, mit 0,2 N Natriumhydrogencarbonatlösung und mehrere Male mit Aethylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: Schmelzpunkt 140—143° (Zersetzung); DC:Rf = 0,37 (Toluol-Aethylacetat-Essigsäure 60:40:5; IR-Spektrum (KBr): Absorptionsbanden bei 3,5, 5,62, 6,0, 6,35, 6,9, 7,52, 7,8, 8,0 $\mu$; NMR-Spektrum (DMSO d6/100 Mc; in ppm): 5,52, 1H, d, J = 1,5 Hz; 3,56, 1H + $2H_2O$, dd, J=1,5 und 7,5 Hz, 2,26, 3H, s; 2,04, 1H, m; 1—0,9, 6H, m.

Beispiel 17

4-Acetylthio-3-benzyl-2-oxo-azetidin (racemische trans-Verbindung)

Eine Lösung von 2,19 g (10 mM) 4-Acetoxy-3-benzyl-azetidin-2-on (racemische Mischung von cis- und trans-Isomer im Verhältnis 9:13) in 10 ml Dioxan wird bei Raumtemperatur unter Stickstoffatmosphäre tropfenweise mit einer Lösung von 0,76 g (10 mM) Thioessigsäure in 10 ml 1N Natriumhydroxidlösung versetzt und bei der gleichen Temperatur 3 Stunden gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an Silicagel mit Toluol-Aethylacetat 9:1 chromatographiert und ergibt ein cis-trans-Gemisch der Titelverbindung im Verhältnis 2:10. Durch Umkristallisation aus Methylenchlorid/Hexan bei —10° erhält man die reine trans-Verbindung vom Schmelzpunkt 42—43°. DC: Rf = 0,52 (Toluol-Aethylacetat 1:1); IR-Spektrum ($CH_2Cl_2$): Absorptionsbanden bei 2,95; 5,65; 5,95; 7,40; 8,8; 10,5 μ. NMR-Spektrum (in $CDCl_3$/100 Mc; in ppm): 7,24; 5H, m; 6,60, 1H, b; 4,99, 1H, d, J = 2Hz; 3,45, 1H, dq, $J_B$ = 8 Hz; $J_C$ = 6 Hz, $J_D$ = 2 Hz; 3,18, 1H, q, $J_A$ = 15 Hz, $J_C$ = 6 Hz; 3,00, 1H, q, $J_A$ = 15 Hz, $J_B$ = 8 Hz; 2,30, 3H, s.

Das Ausgangsmaterial wird wie folgt hergestellt:

a) Eine Mischung von 25 g (0,186 M) 3-Phenylpropionaldehyd, 50 ml Acetanhydrid und 50 ml Pyridin wird während 15 Stunden bei 100° gerührt und dann im Wasserstrahlvakuum eingedampft. Der Rückstand wird im Methylenchlorid gelöst, mit 5%-iger wässriger Natriumhydrogencarbonatlösung und Zitronensäurelösung gewaschen, über Natriumsulfat getrocknet und im Vakuum vom Lösungsmittel befreit. Der Rückstand wird im Vakuum destilliert. Man erhält das 3-Phenyl-1-propenylacetat (cis,trans-Gemisch 1:1) vom Siedepunkt 61—65°/1 mm Hg.

b) Eine bei 0° bereitete Mischung von 17,6 g (0,1 M) 3-Phenyl-1-propenylacetate (cis,trans-Gemisch 1:1) und 14,15 g (0.1 mM) N-Chlorsulfonyl-isocyanat wird 6 Stunden bei 10—15° gerührt. Die Reaktionsmischung wird mit 100 ml kaltem Methylenchlorid verdünnt und langsam auf eine Mischung von 10 ml Wasser, 45 g Eis, 24 g Natriumbicarbonat und 17 g Natriumsulfit gegossen. Nach Filtration wird die organische Phase abgetrennt. Die wässrige Phase wird mit Methylenchlorid extrahiert. Die organischen Phasen werden vereinigt, über Natriumsulfat getrocknet und im Vakuum eingedampft. Der Rückstand wird an Silikagel mit Toluol/Aethylacetat 9:1 bis 8:2 chromatographiert und ergibt ein cis-trans-Gemisch von 4-Acetoxy-3-benzyl-azetidin-2-on im Verhältnis von 9:13. DC: Rf = 0,5 (Toluol-Aethylacetat 1:1); IR-Spektrum (in Methylenchlorid): Absorptionsbanden bei 2,95; 5,6; 5,75; 7,35; 8,15; 8,65; 9,6; 10,25 μ; NMR-Spektrum ($CDCl_3$/100 Mc; in ppm): 2,04, s, und 2,08, s, 3H, 2,95—3,15, 2H, m; 3,35—3,8, 1H, m; 5,50, 0,6 H, d, J = 2Hz (trans); 5,86, 0,4 H, d, J = 4Hz (cis); weitere Signale bei 6,80—7,45.

## Beispiel 18

2-(4-Acetylthio-3-benzyl-2-oxo-1-azetidinyl)-2-hydroxy-esigsäure-p-nitrobenzylester (racemische trans-Verbindung)

Bei Raumtemperatur wird eine Lösung von 0,73 g (3,1 mM) 4-Acetylthio-3-benzyl-3-oxo-azetidin (racemische trans-Verbindung) in 50 ml Toluol und 20 ml Dimethylformamid mit 2 g 2-Aethoxy-2-hydroxy-essigsäure-p-nitrobenzylester versetzt. Nach Zugabe von frisch getrockneten Molekularsieben wird die Mischung unter Stickstoff über Nicht bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an Silikagel mit Toluol-Aethylacetat 9:1 bis 4:1 chromatographiert. Man erhält die beiden mit nicht umgesetztem 2-Aethoxy-2-hydroxyessigsäure-p-nitrobenzylester etwas verunreinigten trans-Isomeren der Titelverbindung mit den folgenden physikochemischen Eigenschaften: DC: Rf = 0,57 (Toluol-Aethylacetat 1:1); IR-Spektrum ($CH_2Cl_2$): Absorptionsbanden bei 2,85; 5,60; 5,70; 6,00; 6,20; 6,55; 7,40; 8,25; 9,00; 11,75 μ.

## Beispiel 19

2-(4-Acetylthio-3-benzyl-2-oxo-1-azetidinyl)-2-triphenyl-phosphoranyliden-essigsäure-p-nitrobenzylester (racemische trans-Verbindung)

a) Eine Lösung von 1,5 g 2-(4-Acetylthio-3-benzyl-2-oxo-1-azetidinyl)-2-hydroxyessigsäure-p-nitrobenzylester (racemische trans-Verbindung) in 20 ml trockenem Dioxan wird mit 6 g Poly-Hünigbase versetzt. Nach tropfenweiser Zugabe einer Lösung von 1,5 ml Thionylchlorid in 10 ml Dioxan wird die Reaktionsmischung 60 Minuten bei Raumtemperatur unter Stickstoff gerührt. Die Poly-Hünigbase wird abfiltriert, mit Dioxan gewaschen und das Filtrat im Vakuum eingedampft. Der erhaltene rohe 2-(4-Acetylthio-3-benzyl-2-oxo-1-azetidinyl)-2-chloressigsäure-p-nitrobenzylester (racemische, trans-Verbindung) kann ohne weitere Reinigung in die nächste Stufe eingesetzt werden.

b) Der erhaltene rohe 2-(4-Acetylthio-3-isopropyl-2-oxo-1-azetidinyl)-2-chloressigsäure-p-nitrobenzylester wird in 20 ml trockenem Dioxan gelöst, mit 6 g Poly-Hünigbase versetzt, 30 Minuten gerührt, dann mit 1,5 g Triphenylphosphin versetzt und über Nacht bei 50° unter Stickstoff gerührt. Die Poly-Hünigbase wird abfiltriert, mit Dioxan gewaschen und Filtrat und Waschflüssigkeit zusammen im Vakuum eingedampft. Der Rückstand wird an Silikagel mit Toluol-Aethylacetat 9:1 bis 1:1 chromatographiert und ergibt die trans-Titelverbindung mit den folgenden physiko-chemischen Eigenschaften: DC: Rf = 0,50 (Toluol-Aethylacetat 1:1); IR-Spektrum ($CH_2Cl_2$): Absorptionsbanden bei 5,7; 5,9; 6,2; 6,55; 7,00; 7,42; 9,05; 11,75 μ.

## Beispiel 20

2-Methyl-6-benzyl-2-penem-3-carbonsäure-p-nitrobenzylester (racemische trans-Verbindung)

Eine Lösung von 0,90 g (1,3 mM) 2-[4-Acetylthio-3-benzyl-2-oxo-1-azetidinyl]-2-triphenylphosphoranyliden-essigsäure-p-nitrobenzylester (racemische trans-Verbindung) in 50 ml trockenem Toluol wird mit einer katalytischen Menge p-Hydroxychinon versetzt und unter Stickstoff zwei Tage bei 90° gerührt. Das Lösungsmittel wird im Vakuum abgedampft und der Rückstand an Silikagel mit Toluol-Aethylacetat 9:1 chromatographiert. Man erhält die trans-Titelverbindung nach Kristallisation aus

Methylenchlorid-Diäthyläther mit folgenden physikochemischen Eigenschaften: Schmelzpunkt: 182—183°; DC: Rf = 0,85 (Toluol-Aethylacetat 1:1): IR-Spektrum ($CH_2Cl_2$): Absorptionsbanden bei 5,60; 5,85; 6,30; 6,55; 7,4; 7,6; 8.25; 8,55; 9,25; 11,70 $\mu$; NMR-Spektrum (in $CDCl_3$/100 Mc; in ppm): 2,36, 3H, s; 3,12; 1H, dd, $J_A$ = 14 Hz, $J_B$ = 9 Hz; 3,34, 1H, dd, $J_A$ = 14 Hz, $J_C$ = 6 Hz; 4,03, 1H, dq, $J_B$ = 9 Hz, $J_C$ = 6 Hz, $J_D$ = 2 Hz; 5,40, 1H, d, $J_D$ = 2 Hz; 5,25, 1H, d, J = 14 Hz; 5,45, 1H, d, J = 14 Hz; 7,30, 5H, m; 7,66, 2H, d, J = 9 Hz; 8,27, 2H, d, J = 9 Hz.

Beispiel 21

2-Methyl-6-benzyl-2-penem-3-carbonsäure (racemische trans-Verbindung)

Eine Lösung von 200 mg 2-Methyl-6-benzyl-2-penem-3-carbonsäure-p-nitrobenzylester (racemische trans-Verbindung) in 12 ml absolutem Aethylacetat wird mit 8 ml 0,2 M wässriger Natriumhydrogencarbonatlösung und 400 mg 10%-igem Palladium/Kohle Katalysator versetzt und bei Normaldruck während 60 Minuten unter Wasserstoff gerührt. Die Hydriermischung wird über Diatomeenerde vom Katalysator abfiltriert. Die wässrige Phase wird abgetrennt, mit 5%-iger wässriger Zitronensäurelösung angesäuert und mit Methylenchlorid erschöpfend extrahiert. Die vereinigten organischen Phasen werden über Natriumsulfat getrocknet, filtriert, im Vakuum eingedampft und im Hochvakuum getrocknet. Die erhaltene Titelverbindung hat folgende physikochemischen Eigenschaften: DC: Rf = 0,31 (Toluol-Aethylacetat-Essigsäure 60:40:5, IR-Spektrum (KBr): Absorptionsbanden bei 3,20—4,30 b; 5,65; 6,0; 6,35; 6,9; 7,5; 7,9; 8.2 $\mu$.

Beispiel 22

4-Aethylthio-thiocarbonylthio-3-isopropyl-2-oxoazetidin (racemische trans-Verbindung)

Eine Lösung von 195 mg (1,14 mM) 4-Acetoxy-3-isopropyl-azetidin-2-on (racemische Mischung von cis- und trans-Isomer im Verhältnis 1:3) in 1 ml Wasser und 0,2 ml Aceton wird bei Raumtemperatur unter Stickstoffatmosphäre tropfenweise mit einer Lösung von 230 mg Kaliumäthyltrithiocarbonat in 1,5 ml Wasser versetzt und bei der gleichen Temperatur 120 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an 12 g Silicagel mit Toluol-Aethylacetat 9:1 chromatographiert und ergibt die trans-Titelverbindung. Schmelzpunkt: 65—66°. DC: Rf = 0,5 (Toluol-Aethylacetat 2:3); IR-Spektrum ($CH_2Cl_2$): Absorptionsbanden bei 2,95; 3,37; 5,62; 9,25 $\mu$. NMR-Spektrum (in $CDCl_3$/100 Mc; in ppm): 6,65, 1H, m (Austausch mit $D_2O$); 5,4, 1H, d (J = 2,5 Hz); 3,39, 2H, q; 3,05, 1H, m; 2,15, 1H, m; 1,38, 3H, t; 1,1, 6H, m;

Beispiel 23

2-(4-Aethylthiothiocarbonylthio-3-isopropyl-2-oxo-1-azetidinyl)-2-hydroxy-essigsäure-p-nitrobenzylester (racemische trans-Verbindung)

Bei Raumtemperatur wird eine Lösung von 137 mg (0,55 mM) 4-Aethylthiothiocarbonylthio-3-isopropyl-2-oxoazetidin (racemische trans-Verbindung) in 8 ml Toluol und 2 ml Dimethylformamid mit 311 mg 2-Aethoxy-2-hydroxy-essigsäure-p-nitrobenzylester versetzt. Nach Zugabe von frisch getrockneten Molekularsieben wird die Mischung unter Stickstoff 15 Stunden bei Raumtemperatur und anschliessend während 2 Stunden bei 50° gerührt. Die Molekularsiebe werden abfiltriert, mit Toluol gewaschen und Filtrat und Waschflüssigkeit zusammen im Vakuum eingedampft. Der Rückstand wird im Hochvakuum getrocknet und an 80 g Silikagel mit Toluol-Aethylacetat 9:1 chromatographiert. Man erhält die beiden mit nicht umgesetztem 2-Aethoxy-2-hydroxy-essigsäure-p-nitrobenzylester etwas verunreinigten trans-Isomeren der Titelverbindung mit den folgenden physikochemischen Eigenschaften: DC: Rf = 0,4 (Toluol-Aethylacetat 2:3); IR-Spektrum ($CH_2Cl_2$): Absorptionsbanden bei 5,62; 5,7; 6,55; 7,42; 8,2; 9,2 $\mu$.

Beispiel 24

2-(4-Aethylthiothiocarbonylthio-3-isopropyl-2-oxo-1-azetidinyl)-2-triphenylphosphoranylidenessigsäure-p-nitrobenzylester (racemische trans-Verbindung)

Eine Lösung von 606 mg 2-(4-Aethylthio-thiocarbonylthio-3-isopropyl-2-oxo-1-azetidinyl)-2-hydroxyessigsäure-p-nitrobenzylester (racemische trans-Verbindung) in 6 ml absolutem Tetrahydrofuran wird auf —15° gekühlt, unter Rühren mit 0,16 ml (2,23 mM) Thionylchlorid und anschliessend langsam mit einer Lösung von 0,31 ml Triäthylamin in 0,3 ml absolutem Tetrahydrofuran versetzt. Das Reaktionsgemisch wird eine Stunde bei 0° gerührt, mit 30 ml kaltem Methylenchlorid versetzt und mit eiskalter 2N Salzsäure gewaschen. Die organische Phase wird mit Wasser bis zur neutralen Reaktion gewaschen, mit Natriumsulfat getrocknet und im Vakuum eingedampft. Der erhaltene rohe 2-(4-Aethylthiothiocarbonylthio-3-isopropyl-2-oxo-1-azetidinyl)-2-chlor-essigsäure-p-nitrobenzylester wird in 1,5 ml trockenem Tetrahydrofuran gelöst, mit 0,71 g Triphenylphosphin versetzt und in einer Stickstoffatmosphäre bei Raumtemperatur über Nacht gerührt. Die Reaktionsmischung wird mit Methylenchlorid verdünnt, nacheinander mit gesättigter wässriger Natriumhydrogencarbonatlösung und Wasser gewaschen, über Natriumsulfat getrocknet und im Vakuum eingedampft. Der Rückstand ergibt nach Chromatographie an Silikagel mit Toluol-Aethylacetat 9:1 die Titelverbindung. DC: Rf = 0,5 (Toluol-

Aethylacetat 2:3); Ir-Spektrum ($CH_2Cl_2$): Absorptionsbanden bei 3,4; 5,7; 6,15; 6,55; 7,45; 9.05; 9,25 μ.

Beispiel 25

2-Aethylthio-6-isopropyl-2-penem-3-carbonsäure-p-nitrobenzylester (racemische trans-Verbindung)

Eine Lösung von 600 mg (0,855 mM) 2-[4-Aethylthiothiocarbonylthio-3-isopropyl-2-oxo-1-azetidinyl]-2-triphenylphosphoranyliden-essigsäure-p-nitrobenzylester (racemische trans-Verbindung) in 250 ml absolutem o-Xylol wird mit einer katalytischen Menge p-Hydroxychinon versetzt und unter Stickstoff 48 Stunden unter Rückfluss gerührt. Das Lösungsmittel wird im Vakuum abgedampft und der Rückstand an 35 g Silikagel mit Toluol-Aethylacetat 19:1 chromatographiert. Man erhält die trans-Titelverbindung nach Kristallisation aus Diäthyläther-Methylenchlorid in Form farbloser Kristalle; DC: Rf = 0,62 (Toluol-Aethylacetat 2:3): IR-Spektrum ($CH_2Cl_2$): Absorptionsbanden bei 5,57; 5,9; 6,55; 7,4; 7.52 μ.

Beispiel 26

2-Aethylthio-6-isopropyl-2-penem-3-carbonsäure (racemische trans-Verbindung)

Eine Lösung von 100 mg 2-Aethylthio-6-isopropyl-2-penem-3-carbonsäure-p-nitrobenzylester (racemische trans-Verbindung) in 6 ml absolutem Aethylacetat wird mit 4 ml 0,2 N wässriger Natriumhydrogencarbonatlösung und 150 mg 10%-igem Palladium/Kohle Katalysator versetzt und bei Normaldruck während 240 Minuten unter Wasserstoff gerührt. Die Hydriermischung wird über Diatomeenerde vom Katalysator abfiltriert, mit 0,2 N Natriumhydrogencarbonatlösung und mehrere Male mit Aethylacetat nachgewaschen. Die wässrige Phase wird mit Methylenchlorid gewaschen, mit 5%-iger wässriger Zitronensäurelösung angesäuert und mit Methylenchlorid erschöpfend extrahiert. Die vereinigten organischen Phasen werden über Natriumsulfat getrocknet; filtriert, im Vakuum eingedampft und im Hochvakuum getrocknet. Die erhaltene Titelverbindung hat folgende physikochemischen Eigenschaften: DC: Rf = 0,35 (Toluol-Aethylacetat-Essigsäure 60:40:5); IR-Spektrum (KBr): Absorptionsbanden bei 3,5, 5,62, 6,0, 6,75, 6,9, 7,52, 7,9, 8,15, 8,9 μ.

Beispiel 27

6-Diazo-penicillansäure-methylester

Analog der Deutschen Offenlegungsschrift Nr. 2.305.972 werden 1,01 g roher 6β-(N-Nitroso)-phenoxyacetamido-penicillansäure-methylester (hergestellt gemäss USP 3.880.837) bei Zimmertemperatur in 75 ml absolutem Chloroform gelöst und nach Zugabe von 200 ml gesättigter wässriger Natriumbicarbonatlösung während 9 Stunden zwischen 10 und 20° gerührt. Die Chloroformlösung wird abgetrennt, mit Wasser gewaschen und über Natriumsulfat getrocknet. Nach Abdampfen des Lösungsmittels im Vakuum bei Raumtemperatur wird die rohe Diazoverbindung in Form eines Oeles erhalten. Sie kann ohne weitere Reinigung in die nächste Reaktion eingesetzt werden. IR-Spektrum (in Methylenchlorid: charakteristische Absorptionsbanden bei 3,40; 4,80; 5,55; 5,70; 6,23; 6,50; 6,68; 7,75; 8,22; 8,85; 10,6; 11,42 μ.

Beispiel 28

6α-Methoxy-penicillansäure-methylester

Eine Lösung von 2 g rohem 6-Diazo-penicillansäure-methylester in 15 ml absolutem Methylenchlorid wird mit 5 ml Methanol und wenigen Tropfen 30%-iger wässriger Perchlorsäure versetzt und 15 Minuten während Raumtemperatur gerührt. Die Reaktionsmischung wird mit 30 ml Methylenchlorid verdünnt und nacheinander mit wässriger Natriumbicarbonatlösung. Wasser und Kochsalzlösung gewaschen, über Natriumsulfat getrocknet und im vakuum eingedampft. Der Rückstand wird an Silicagel mit Toluol-Aethylacetat 9:1 und 4:1 chromatographiert und ergibt die leicht verunreinigte Titelverbindung. IR-Spektrum (in Methylenchlorid): charakterische Banden bei 3,40; 5,63; 5,70; 6,90; 7,30; 7,68; 8,25; 8,47; 8,90; 9,15; 9,70; 9,86 μ.

Beispiel 29

6α-Methoxy-penicillansäure-methylester-1-oxid

Eine Lösung von 473 mg 6α-Methoxy-penicillansäure-methylester in 10 ml Methylenchlorid wird auf 0° gekühlt, mit 334 mg m-Chlorperbenzoesäure versetzt und die erhaltene Suspension während 1 Stunde bei der gleichen Temperatur weitergerührt. Die Reaktionsmischung wird mit 50 ml Methylenchlorid verdünnt, zweimal mit wässriger Natriumbicarbonatlösung und mit Wasser gewaschen und über Natriumsulfat getrocknet. Das Lösungsmittel wird im Vakuum abgedampft und der Rückstand an Silicagel chromatographiert. Mit Toluol-Aethylacetat 9:1 und 4:1 wird die Titelverbindung in Form eines weissen Pulvers gewonnen. Eine analytische Probe wird aus Methylenchlorid-Diäthyläther-Pentan umkristallisiert und hat folgende physiko-chemischen Eigenschaften: F. 121°; $\alpha_D = +281° \pm 1°$; IR-Spektrum (in Methylenchlorid): charakterische Absorptionsbanden bei 3,40; 5,58; 5,70, 6,85; 6,97; 7,75; 8,20; 8,90; 9,45 μ.

Beispiel 30

2-[(3S,4R)-4-(Benzthiazol-2-yldithio)-3-methoxy-2-oxoazetidin-1-yl]-3-methylenbuttersäure-methylester.

Eine Lösung von 307 mg 6α-Methoxy-penicillansäure-methylester-1-oxid wird in 10 ml Toluol gelöst, mit 196,57 mg 2-Mercaptobenzthiazol versetzt und während 90 Minuten am Rückfluss erhitzt. Das Lösungsmittel wird im Vakuum abdestilliert und der Rückstand an Silicagel chromatographiert. Durch Elution mit Toluol-Aethylacetat 9:1 wird die Titelverbindung in Form eines farblosen Oeles gewonnen. IR-Spektrum (in Methylenchlorid): charakteristische Banden bei 3,40; 5,62; 5,72; 6,68; 6,85; 7,02; 7,25; 7,50; 8,10; 8,20; 8,60; 8,95; 9,55; 9,92; 10,92 μ.

Beispiel 31

2-[(3S,4R)-4-(Benzthiazol-2-yldithio)-3-methoxy-2-oxoazetidin-1-yl]-3-methylcrotonsäure-methylester

Eine Lösung von 432 mg 2-[(3S,4R)-4-(Benzothiazol-2-yldithio)-3-methoxy-2-oxoazetidin-1-yl]-3-methylenbuttersäure-methylester in 25 ml Methylenchlorid wird mit 0,1 ml Triäthylamin versetzt und bei Raumtemperatur 100 Minuten gerührt. Die Reaktionsmischung wird mit Methylenchlorid verdünnt, zweimal mit wässriger Zitronensäurelösung und Wasser gewaschen, über Natriumsulfat getrocknet und im Vakuum vom Lösungsmittel befreit. Der Rückstand wird durch Chromatographie an Silicagel mit Toluol-Aethylacetat 9:1 und 4:1 gereinigt und ergibt die Titelverbindung in Form eines Oeles. IR-Spektrum (in Methylenchlorid): charakteristische Absorptionsbanden bei 3,40; 5,63; 5,78; 6,85; 7,03; 7,23; 7,32; 7,0; 8,15; 8,87; 9,00; 9,25; 9,92 μ.

Beispiel 32

2-[(3S,4R)-4-Acetylthio-3-methoxy-2-oxoazetidin-1-yl]-3-methylcrotonsäure-methylester

Eine Lösung von 372 mg 2-[(3S,4R)-4-(Benzthiazol-2-yldithio)-3-methoxy-2-oxoazetidin-1-yl]-3-methylcrotonsäure-methylester in 10 ml Dimethylformamid wird auf —20° gekühlt, mit 10 ml einer Lösung von 2 g Natriumborhydrid in 200 ml Dimethylformamid versetzt und bei der gleichen Temperatur 30 Minuten gerührt. Die Reaktionsmischung wird mit 5 ml frisch destilliertem Acetylbromid versetzt und bei 0° während 110 Minuten weiter gerührt. Nach Zugabe von 150 ml Benzol wird die Reaktionsmischung nacheinander mit Natriumbicarbonatlösung und Wasser gewaschen, über Natriumsulfat getrocknet und im Vakuum eingedampft. Der Rückstand ergibt nach Chromatographie an Silicagel mit Toluol-Aethylacetat 9:1 die Titelverbindung in Form eines leicht gelblichen Oeles. IR-Spektrum (in Methylenchlorid): charakteristische Absorptionsbanden bei 3,40; 5,63; 5,77; 5,83; 6,10; 6,95; 7,20; 7,30; 7,70; 8,12; 8,90; 9,20; 9,90; 10,20; 10,50; 11,83 μ.

Beispiel 33

2-[(3S,4R)-4-Acetylthio-3-methoxy-2-oxoazetidin-1-yl]-2-oxoessigsäure-methylester

Durch eine auf —30° gekühlte Lösung von 87 mg (0,31 mM) 2-[(3S,4R)-4-Acetylthio-3-methoxy-2-oxoazetidin-1-yl]-3-methylcrotonsäure-methylester in 5 ml Aethylacetat werden 3 Aequivalente Ozon geleitet. Die Reaktionsmischung wird mit 30ml Methylenchlorid verdünnt und während 2 Minuten mit einer 10%-igen wässrigen Natriumbisulfitlösung geschüttelt. Die organische Phase wird abgetrennt, mit gesättigter Natriumchloridlösung gewaschen, über Natriumsulfat getrocknet und im Vakuum eingedampft. IR-Spektrum der erhaltenen öligen Titelverbindung (in Methylenchlorid): charakteristische Banden bei 3,40; 5,47; 5,67; 5,82; 6,97; 7,33; 8,10; 8,92; 9,88; 10,40 μ. Das erhaltene Produkt kann ohne weitere Reinigung in die nächste Stufe eingesetzt werden.

Beispiel 34

(3S,4R)-4-Acetylthio-3-methoxy-2-oxoazetidin

Eine Lösung von 71,20 mg 2-[(3S,4R)-4-Acetylthio-3-methoxy-2-oxazetidin-1-yl]-2-oxoessigsäure-methylester (Rohprodukt) in 10 ml 1%-igem wässrigem Methanol wird über Nacht bei Raumtemperatur gerührt. Die Reaktionsmischung wird mit Methylenchlorid verdünnt, mit Wasser gewaschen, über Natriumsulfat getrocknet und im Vakuum eingedampft. Der Rückstand wird an Silicagel mit Toluol-Aethylacetat 9:1 chromatographiert und ergibt die Titelverbindung. IR-Spektrum (in Methylenchlorid):charakteristische Absorptionsbanden bei 2,95; 3,40; 5,60; 5,88; 7,00; 7,37; 7,52; 8,25; 8,70; 8,85; 10,5; 12,15 μ.

Beispiel 35

2-[(3S,4R)-4-Acetylthio-3-methoxy-2-oxoazetidin-1-yl]-2-hydroxyessigsäure-p-nitrobenzylester

Eine Lösung von 245 mg (3S,4R)-4-Acetylthio-3-methoxy-2-oxoazetidin in einer Mischung von 8 ml Toluol und 2 ml Dimethylformamid wird mit 714 mg 2-Aethoxy-2-hydroxy-essigsäure-p-nitrobenzylester und 4 g Molekularsieb A4 versetzt und bei Raumtemperatur über Nacht gerührt. Von den Molekularsieben wird abfiltriert und das Filtrat im Vakuum eingedampft. Der Rückstand wird an Silicagel chromatographiert, wobei durch Elution mit Toluol-Aethylacetat 9:1 und 4:1 die Titelverbindung, verunreinigt mit etwas Glyoxylat, erhalten wird.

Beispiel 36

2-[(3S,4R)-4-Acetylthio-3-methoxy-2-oxoazetidin-1-yl]-2-triphenylphosphoranyliden-essigsäure-p-nitrobenzylester

a) Eine Suspension von 2 g Poly-Hünigbase in 8 ml Dioxan wird 30 Minuten bei Raumtemperatur gerührt, mit 832 mg 2-[(3S,4R)-4-Acetylthio-3-methoxy-2-oxoazetidin-1-yl]-2-hydroxyessigsäure-p-nitrobenzylester gelöst in 12 ml Dioxan und anschliessend langsam mit einer Lösung von 0,54 ml Thionylchlorid in 10 ml Dioxan versetzt. Die Mischung wird 2 Stunden bei Raumtemperatur gerührt, von der Poly-Hünigbase abfiltriert und das Filtrat im Vakuum eingedampft. Der Rückstand wird durch Chromatographie an Silicagel mit Toluol-Aethylacetat 1:1 gereinigt und ergibt den 2-[(3S,4R)-4-Acetylthio-3-methoxy-2-oxoazetidin-1-yl]-2-chloressigsäure-p-nitrobenzylester in roher Form.

b) Eine Lösung von 833 mg 2-[(3S,4R)-4-Acetylthio-3-methoxy-2-oxoazetidin-1-yl]-2-chloressigsäure-p-nitrobenzylester in 50 ml Dioxan wird mit 812 mg Triphenylphosphin und 3 g Poly-Hünigbase versetzt und über Nacht bei 50° gerührt. Die Poly-Hünigbase wird durch Filtration entfernt und das Filtrat im Vakuum eingedampft. Der Rückstand wird an Silicagel mit Toluol-Aethylacetat 9:1, 4:1 und 1:1, chromatographiert und ergibt die Titelverbindung. IR-Spektrum (in Methylenchlorid): charakteristische Absorptionsbanden bei 3,40; 5,67; 5,85; 6,15; 6,55; 6,97; 7,42; 8,0; 9,03 $\mu$.

Beispiel 37

(5R6S)-2-Methyl-6-methoxy-2-penem-3-carbonsäure-p-nitrobenzylester

Eine Lösung von 244 mg 2-[(3S,4R)-4-Acetylthio-3-methoxy-2-oxoazetidin-1-yl]-2-triphenylphosphoranylidenessigsäure-p-nitrobenzylester in 100 ml absolutem Toluol wird mit einer katalytischen Menge Hydrochinon versetzt und 32 Stunden bei 90° unter Stickstoff gerührt. Das Toluol wird im Vakuum abgedampft und der Rückstand an Silicagel mit Toluol-Aethylacetat 19:1 chromatographiert. Die Titelverbindung wird in Form einer weissen festen Substanz erhalten. IR-Spektrum (in Methylenchlorid): charakteristische Absorptionsbanden bei 3,40; 5,55; 6,30; 6,55; 7,03; 7,42; 7,60; 8,20; 8,42; 8,60; 8,90; 9,20; 9,60; 11,7 $\mu$; NMR-Spektrum ($CDCl_3$/100 Mc; in ppm): 8,22, 2H, d, J = 8 Hz; 7,64, 2H, d, J = 8 Hz; 1H, d, J = 2 Hz; 5,35, 2H, AB; 4,91, 1H, d, J = 2 Hz; 3,57, 3H, s; 2,37, 3H, s.

Beispiel 38

(5R,6S)-2-Methyl-6-methoxy-2-penem-3-carbonsäure

Eine Lösung von 34 mg (5R,6S)-2-Methyl-6-methoxy-2-penem-3-carbonsäure-p-nitrobenzylester in einer Mischung von 2 ml Aethylacetat und 2 ml 2M-Natriumbicarbonatlösung wird mit 75 mg 10% Palladium/Kohle-Katalysator versetzt und unter Atmosphärendruck während 1,5 Stunden bei Raumtemperatur hydriert. Die Hydriermischung wird durch Diatomeenerde filtriert, der Filterrückstand mit 1 ml 2 M wässriger Natriumbicarbonatlösung und Aethylacetat gewaschen. Vom Filtrat wird die wässrige Phase abgetrennt, mit 0,1 M wässriger Zitronensäure angesäuert und mehrere Male mit Methylenchlorid extrahiert. Die vereinigten Methylenchloridextrakte werden über Natriumsulfat getrocknet und im Vakuum eingedampft. IR-Spektrum (in Methylenchlorid) der erhaltenen rohen Titelverbindung: charakterische Absorptionsbanden bei 3,40; 5,57; 5,80; 5,95; 6,30; 7,00; 8,20; 9,90 $\mu$m.

Beispiel 39

6$\alpha$-Phenoxyacetoxy-penicillansäure-methylester

Analog D. Hauser und H. P. Sigg, Helv. Chim. Acta *50*, 1327 (1967) wird eine Lösung von 7.4 g (20.3 mM) 6$\beta$-(N-Nitroso)-phenoxyacetamido-penicillansäure-methylester (Rohprodukt gemäss USP 3.880.837) in 100 ml Benzol 3 Stunden bei 50° unter Stickstoffatmosphäre gerührt. Das Lösungsmittel wird im Vakuum abgedampft und der Rückstand an Silicagel mit Toluol-Aethylacetat 9:1 chromatographiert. Das erhaltene ölige Produkt wird aus Diäthyläther-Hexan umkristallisiert und ergibt die Titelverbindung vom Schmelzpunkt 71°; $\alpha_D = 114 \pm 1°$ ($CHCl_3$); IR-Spektrum (in Methylenchlorid): charakteristische Absorptionsbanden bei 3,4; 5,6; 5,7; 6,25; 6,69; 7,17; 8,26; 8,55; 9,05; 9,18 $\mu$.

Beispiel 40

6$\alpha$-Phenoxyacetoxy-penicillansäure-methylester-1-oxid

Eine Lösung von 1,16 g (3,18 mM) 6$\alpha$-Phenoxyacetoxy-penicillansäure-methylester in 30 ml absolutem Methylenchlorid wird bei 0° portionenweise mit 1,1 g (1 Aequivalent) 50%-iger m-Chlorperbenzoesäure versetzt. Nach Beendigung der Zugabe wird die Reaktionsmischung 30 Minuten bei 0° gerührt, dann mit Methylenchlorid verdünnt, nacheinander mit wässriger Natriumbircarbonatlösung, Wasser und Kochsalzlösung gewaschen und über Natriumsulfat getrocknet. Nach Abdampfen des Lösungsmittels wird der Rückstand an Silicagel mit Toluol-Aethylacetat 4:1 chromatographiert. Man erhält die Titelverbindung in Form eines Schaumes; DC: Rf = 0,24 (Toluol-Aethylacetat 1:1), IR-Spektrum (in Methylenchlorid): charakteristische Absorptionsbanden bei 3,33; 3,41; 5,57; 5,72; 6,27; 6,72; 7,0; 8,25; 8,6; 9,21; 9,46 $\mu$.

## Beispiel 41

2-[(3S,4R)-4-(Benzthiazol-2-yldithio)-3-phenoxyacetoxy-2-oxoazetidin-1-yl]-3-methylenbuttersäure-methylester

Eine Lösung von 1,01 g (2,65 mM) 6α-Phenoxyacetoxy-penicillansäure-methylester-1-oxid wird in 30 ml Toluol gelöst, mit 445 mg (2,65 mM) 2-Mercaptobenzthiazol versetzt und während 60 Minuten in einer Stickstoffatmosphäre am Rückfluss erhitzt. Das Lösungsmittel wird im Vakuum abdestilliert und der Rückstand an Silicagel chromatographiert. Durch Elution mit Toluol-Aethylacetat 19:1 wird die Titelverbindung in Form eines schwach-braunen Oeles gewonnen. IR-Spektrum (in Methylenchlorid):charakteristische Banden bei 3,45; 5,62; 5,75; 6,27; 6,71; 6,89; 7,05; 7,30; 7,54; 7,68; 8,15; 8,55; 9,15; 9,35; 9,95 μ; DC: Rf = 0,63 (Toluol-Aethylacetat 1:1).

## Beispiel 42

2-[(3S,4R)-4-(Benzthiazol-2-yldithio)-3-phenoxyacetoxy-2-oxoazetidin-1-yl]-4-(Benzthiazol-2-yldithio)-3-phenoxyacetoxy-2-oxoazetidin-1-yl]-3-methylcrotonsäure-methylester

Eine Lösung vo 1,28 g (2,41 mM) 2-[(3S,4R)-4-(Benzthiazol-2-yldithio)-3-phenoxyacetoxy-2-oxoazetidin-1-yl]-3-methylenbuttersäure-methylester in 30 ml Methylenchlorid wird mit 0,4 ml Triäthylamin versetzt und bei Raumtemperatur 30 Minuten gerührt. Die Reaktionsmischung wird mit 50 ml Methylenchlorid verdünnt, nacheinander mit 2N Salzsäure, Wasser und Kochsalzlösung gewaschen, über Natriumsulfat getrocknet und im Vakuum vom Lösungsmittel befreit. Der Rückstand wird durch Chromatographie an Silicagel mit Toluol-Aethylacetat 19:1 gereinigt und ergibt die Titelverbindung in Form eines schwachgelben Oeles. IR-Spektrum (in Methylenchlorid): charakteristische Absorptionsbanden bei 3,46; 5,69; 5,82; 5,90; 6,28; 6,73; 6,90; 7,06; 7,28; 7,38; 7,75; 8,20; 8,60; 9,27; 9,96 μ; DC:Rf = 0,61 (Toluol-Aethylacetat 1:1).

## Beispiel 43

2-[(3S,4R)-4-Acetylthio-3-phenoxyacetoxy-2-oxoazetidin-1-yl]-3-methylcrotonsäure-methylester

Eine Lösung von 687 mg (1,29 mM) 2-[(3S,4R)-4-(Benzthiazol-2-yldithio)-3-phenoxyacetoxy-2-oxoazetidin-1-yl]-3-methylcrotonsäure-methylester in 14 ml Dimethylformamid wird zu einer auf —20° gekühlten Lösung von 76 mg (2 mM) Natriumborhydrid in 10 ml Dimethylformamid gegeben und bei der gleichen Temperatur 10 Minuten gerührt. Die Reaktionsmischung wird mit 7 ml frisch destilliertem Acetylbromid versetzt und bei 0° während 40 Minuten weiter gerührt. Nach Zugabe von 400 ml Benzol wird die Reaktionsmischung nacheinander mit wässriger Natriumbicarbonatlösung, Wasser und Kochsalzlösung gewaschen, über Natriumsulfat getrocknet und im Vakuum eingedampft. Der Rückstand ergibt nach Chromatographie an Silicagel mit Toluol-Aethylacetat 19:1 die Titelverbindung in Form eines Oeles, das auf Silicagelplatten mit Toluol-Aethylacetat 4:1 weiter gereinigt wird. Man erhält die Titelverbindung in öliger Form; IR-Spektrum (in Methylenchlorid): charakteristische Absorptionsbanden bei 3,45; 5,63; 5,83; 6,27; 6,70; 7,00; 7,25; 7,35; 8,15; 8,58; 8,93; 9,20 μ; DC:Rf= 0,54 (Toluol-Aethylacetat 1:1).

## Beispiel 44

2-[3S,4R)-4-Acetylthio-3-phenoxyacetoxy-2-oxoazetidin-1-yl]-2-oxoessigsäure-methylester

Durch eine auf —20° gekühlte Lösung von 170 mg (0,42 mM) 2-[(3S,4R)-4-Acetylthio-3-phenoxyacetoxy-2-oxoazetidin-1-yl]-3-methylcrotonsäure-methylester in 5 ml Aethylacetat werden 4 Aequivalente Ozon geleitet. Die Reaktionsmischung wird mit 5 ml Aethylacetat verdünnt und intsensiv mit einer 10%-igen wässrigen Natriumbisulfitlösung geschüttelt. Die organische Phase wird abgetrennt, mit Wasser und gesättigter Natriumchloridlösung gewaschen, über Natriumsulfat getrocknet und im Vakuum eingedampft. IR-Spektrum der erhaltenen öligen Titelverbindung (in Methylenchlorid): charakteristische Banden bei 3,38; 5,48; 5,63; 5,70; 5,83; 6,27; 6,70; 7,00; 7,40; 8,07; 8,25; 8,63; 8,95 μ. Das erhaltene Produkt kann ohne weitere Reinigung in die nächste Stufe eingesetzt werden.

## Beispiel 45

(3S,4R)-4-Acetylthio-3-phenoxyacetoxy-2-oxoazetidin

Eine Lösung von 129 mg (0,34 mM) (3S,4R)-2-(4-Acetylthio-3-phenoxyacetoxy-2-oxoazetidin-1-yl)-2-oxoessigsäure-methylester (Rohprodukt) in 10 ml 1%-igem wässrigem Methanol wird 4 Stunden bei Raumtemperatur gerührt. Die Reaktionsmischung wird mit 50 ml Methylenchlorid verdünnt, nacheinander mit Wasser und gesättigter Kochsalzlösung gewaschen, über Natriumsulfat getrocknet und im Vakuum eingedampft. Der Rückstand wird an Silicagel mit Toluol-Aethylacetat 9:1 chromatographiert und ergibt die Titelverbindung. IR-Spektrum (in Methylenchlorid): charakteristische Banden bei 2,95; 3,45; 5,55; 5,60; 5,88; 6,25; 6,68; 8,33; 8,85 μ; DC:Fr = 0,36 (Toluol-Aethylacetat 1:1).

## Beispiel 46

2-[(3S,4R)-4-Acetylthio-3-phenoxyacetoxy-2-oxoazetidin-1-yl]-2-hydroxyessigsäure-p-nitrobenzylester

Eine Lösung von 283 mg (3S,4R)-4-Acetylthio-3-methoxy-2-oxoazetidin in einer Mischung von 8 ml Toluol und 2 ml Dimethylformamid wird mit 760 mg 2-Aethoxy-2-hydroxy-essigsäure-p-nitrobenzylester und 4 g Molekularsieb A4 versetzt und bei Raumtemperatur über Nacht gerührt. Von den Molekularsieben wird abfiltriert und das Filtrat im Vakuum eingedampft. Der Rückstand wird an Silicagel chromatographiert, wobei durch Elution mit Toluol-Aethylacetat 9:1 und 4:1 die Titelverbindung, verunreinigt mit etwas Glyoxylat, erhalten wird.

Beispiel 47

2-[(3S,4R)-4-Acetylthio-3-phenoxyacetoxy-2-oxoazetidin-1-yl]-2-triphenylphosphoranyliden-essigsäure-p-nitrobenzylester

a) Eine Suspension von 2 g Poly-Hünigbase in 8 ml Dioxan wird 30 Minuten bei Raumtemperatur gerührt, mit 962 mg 2-[(3S,4R)-4-Acetylthio-3-phenoxyacetoxy-2-oxoazetidin-1-yl]-2-hydroxyessigsäure-p-nitrobenzylester gelöst in 10 ml Dioxan und anschliessend langsam mit einer Lösung von 0,38 ml Thionylchlorid in 8 ml Dioxan versetzt. Die Mischung wird 2 Stunden bei Raumtemperatur gerührt, von der Poly-Hünigbase abfiltriert und das Filtrat im Vakuum eingedampft. Der Rückstand wird durch Chromatographie an Silicagel mit Toluol-Aethylacetat 1:1 gereinigt und ergibt den 2-[(3S,4R)-4-Acetylthio-3-methoxy-2-oxoazetidin-1-yl]-2-chloressigsäure-p-nitrobenzylester in roher Form.

b) Eine Lösung von 960 mg 2-[(3S,4R)-4-Acetylthio-3-phenoxyacetoxy-2-oxoazetidin-1-yl]-2-chloressigsäure-p-nitrobenzylester in 40 ml Dioxan wird mit 786 mg Triphenylphosphin und 3 g Poly-Hünigbase versetzt und über Nacht bei 50° unter Stickstoff gerührt. Die Poly-Hünigbase wird durch Filtration entfernt und das Filtrat im Vakuum eingedampft. Der Rückstand wird an Silicagel mit Toluol-Aethylacetat 9:1, 4:1 und 1:1, chromatographiert und ergibt die Titelverbindung. IR-Spektrum (in Methylenchlorid): charakteristische Absorptionsbanden bei 5,7; 5,9; 6,17; 6,55; 7,45 $\mu$.

Beispiel 48

(5R,6S)-2-Methyl-6-phenoxyacetoxy-2-penem-3-carbonsäure-p-nitrobenzylester

Eine Lösung von 285 mg 2-[(3S,4R)-4-Acetylthio-3-phenoxyacetoxy-2-oxoazetidin-1-yl]-2-triphenylphosphoranyliden-essigsäure-p-nitrobenzylester in 100 ml absolutem Toluol wird mit einer katalytischen Menge Hydrochinon versetzt und 35 Stunden bei 90° unter Stickstoff gerührt. Das Toluol wird im Vakuum abgedampft und der Rückstand an Silicagel mit Toluol-Aethylacetat 19:1 chromatographiert. Die Titelverbindung wird in Form eines Oeles erhalten. IR-Spektrum (in Methylenchlorid): charakteristische Absorptionsbanden bei 5,55; 6,30; 6,55; 7,42 $\mu$.

Beispiel 49

(5R,6S)-2-Methyl-6-phenoxyacetoxy-2-penem-3-carbonsäure

Eine Lösung von 45 mg (5R,6S)-2-Methyl-6-phenoxyacetoxy-2-penem-3-carbonsäure-p-nitrobenzylester in einer Mischung von 2 ml Aethylacetat und 2 ml 2M-Natriumbicarbonatlösung wird mit 75 mg 10% Palladium/Kohle-Katalysator versetzt und unter Atmosphärendruck während 1,5 Stunden bei Raumtemperatur hydriert. Die Hydriermischung wird durch Diatomeenerde filtriert, der Filterrückstand mit 1 ml 2M wässriger Natriumbicarbonatlösung und Methylacetat gewaschen. Vom Filtrat wird die wässrige Phase abgetrennt, mit 0,1 M wässriger Zitronensäure angesäuert und mehrere Male mit Methylenchlorid extrahiert. Die vereinigten Methylenchloridextrakte werden über Natriumsulfat getrocknet und im Vakuum eingedampft, IR-Spektrum (in Aethanol) der erhaltenen rohen Titelverbindung: charakteristische Absorptionsbande bei 5,6 $\mu$; UV-Spektrum (in Aethanol): $\lambda_{max} = 305$ nm.

Beispiel 50

6$\alpha$-Methoxy-penicillansäure-2,2,2-trichloräthylester-1-oxid.

Eine Lösung von 2 g 6$\alpha$-Methoxy-penicillansäure-2,2,2-trichloräthylester (hergestellt nach P. J. Giddins, D. I. Johns, E. J. Thomas; T. L. *11*, 995, 1978) in 100 ml Methylenchlorid und 0,3 ml Aceton wird auf −15°C gekühlt, innert 5 Minuten mit 1 ml 40 prozentiger Peressigsäure versetzt und 15 Minuten bei der gleichen Temperatur gerührt. Anschliessend wird die Reaktionsmischung mit 15 ml einer 0,1 N Natriumthiosulfatlösung versetzt. Die organische Lösung wird abgetrennt und zweimal mit Eiswasser gewaschen. Das Lösungsmittel wird nach Trocknen über Natriumsulfat im Vakuum eingedampft und der Rückstand aus Aether-Petroläther umkristallisiert. Die erhaltene Verbindung hat folgende physiko-chemischen Eigenschaften: F = 127—128°. IR-Spektrum (in Methylenchlorid): charakteristische Absorptionsbanden bei 3,41; 5,58; 5,65; 8,33; 8,47; 8,70; 9,48 $\mu$.

Beispiel 51

2-[(3S,4R)-4-(Benzthiazol-2-yldithio)-3-methoxy-2-oxo-azetidin-1-yl]-3-methylenbuttersäure-2,2,2-trichloräthylester

Eine Lösung von 3 g 6$\alpha$-Methoxy-penicillansäure-2,2,2-trichloräthylester-1-oxid in 40 ml absolutem Toluol wird mit 1,39 g 2-Mercaptobenzthiazol versetzt und während 105 Minuten unter Stickstoff am Rückfluss erhitzt. Das Lösungsmittel wird im Vakuum abdestilliert und liefert die Titelverbindung in Form eines gelblichen Oeles. IR-Spektrum (in Methylenchlorid): charakteristische Absorptionsbanden bei 3,39; 5,60; 5,65; 6,85; 8,20; 8,62; 8,97; 9,85; 9,95 $\mu$.

Das erhaltene Produkt kann ohne weiter Reinigung in die nächste Stufe eingesetzt werden.

Beispiel 52

2-[(3S,4R)-4-(Benzthiazol-2-yldithio)-3-methoxy-2-oxoazetidin-1-yl]-3-methylcrotonsäure-2,2,2-trichloräthylester

Eine Lösung von 4,17 g 2-[(3S,4R)-4-(Benzthiazol-2-yldithio)-3-methoxv-2-oxoazetidin-1-yl]-3-methylen-buttersäure-2,2,2-trichloräthylester in 75 ml absolutem Methylenchlorid wird bei 0° mit 0,78 ml Triäthylamin versetzt und bei dieser Temperatur 15 Minuten gerührt.

Das Reaktionsgemisch wird nacheinander mit 4N Phosphorsäure, gesättigter wässriger Natriumbicarbonatlösung und Sole gewaschen und über Natriumsulfat getrocknet. Das Lösungsmittel wird abgedampft und der Rückstand durch Chromatographie an Silicagel mit Toluol und Toluol-Aethylacetat 19:1 gereinigt. Die Titelverbindung wird in Form eines Oeles erhalten. IR-Spektrum (in Methylenchlorid): charakteristische Absorptionsbanden bei 3,39; 5,62; 5,76; 6,85; 7,04; 7,25; 6,85; 9,01; 9,48; 9,85; 9,95 $\mu$.

Beispiel 53

2-[(3S,4R)-4-Acetylthio-3-methoxy-2-oxoazetidin-1-yl]-3-methylcrotonsäure-2,2,2-trichloräthylester

Eine Lösung von 3,26 g 2-[(3S,4R)-4-(Benzthiazol-2-yldithio)-3-methoxy-2-oxoazetidin-1-yl]-3-methylcrotonsäure-2,2,2-trichloräthylester in 36,3 ml Acetanhydrid und 12,4 ml Essigsäure wird auf —15° gekühlt und mit 1,7 g Triphenylphosphin versetzt. Nach 75 Minuten Rühren unter Stickstoff bei der gleichen Temperatur gibt man dem Gemisch 24,8 ml Pyuridin zu. Nach weiteren 3 Stunden Rühren bei 0° wird das Reaktionsgemisch unter vermindertem Druck eingedampft und der erhaltene Rückstand durch Chromatographie an Silicagel mit Toluol und Toluol-Aethylacetat 19:1 gereinigt. IR-Spektrum (in Methylenchlorid): charakteristische Absorptionsbanden bei 3,40; 5,63; 5,77; 5,80; 6,13; 7,25; 7,35; 8,26; 9,0; 9,52; 11,90 $\mu$.

Beispiel 54

2-[(3S,4R)-4-Acetylthio-3-methoxy-2-oxoazetidin-1-yl]-2-oxoessigsäure-2,2,2-trichloräthylester

Durch eine auf —30° gekühlte Lösung von 8,4 g 2-[(3S,4R)-4-Acetylthio-3-methoxy-2-oxoazetidin-1-yl]-3-methylcrotonsäure-2,2,2-trichloräthylester in 765 ml Methylacetat werden 3 Aequivalente ozon geleitet. Nach der Ozon-Behandlung wird das Reaktionsgemisch 15 Minuten bei gleicher Temperatur stehengelassen und anschliessend das uberschüssige Ozon durch einen Stickstoffstrom entfernt. Das Reaktionsgemisch wird bei 0° mit einer 10%-igen wässrigen Natriumbisulfitlösung und danach mit Sole gewaschen. Nach Abtrennen werden die vereinten wässrigen Phasen noch 4 mal mit Methylacetat extrahiert. Die vereinigten Methylesterlösungen werden über Natriumsulfat getrocknet und im Vakuum eingedampft. IR-Spektrum der erhaltenen öligen Titelverbindung (in Methylenchlorid): charakteristische Absorptionsbanden bei 3,39; 5,48; 5,63; 6,09; 6,94; 7,25; 7,38; 7,46; 8,23; 8,93; 9,90; 11,83 $\mu$.

Beispiel 55

(3S,4R)-4-Acetylthio-3-methoxy-2-oxoazetidin

a) Eine Lösung von 1,52 g 2-[(3S,4R)-4-Acetylthio-3-methoxy-2-oxoazetidin-1-yl]-2-oxoessigsäure-2,2,2-trichloräthylester (Rohprodukt) in 290 ml Methanol, 40 ml Methylacetat und 5,9 ml Wasser wird während 20 Minuten unter Stickstoff am Rückfluss erhitzt. Das Lösungsmittel wird im Vakuum eingedampft. Der Rückstand ergibt nach Chromatographie an Silicagel mit Toluol/Aethylacetat 3:1 die Titelverbindung. IR-Spektrum (in Methylenchlorid): charakteristische Absorptionsbanden bei 2,95; 3,40; 5,60; 5,88; 7,37; 7,52; 8,25; 8,70; 8,85; 10,5; 12,12 $\mu$.

Die gleiche Verbindung kann auch wie folgt erhalten werden:

b) Eine Lösung von 40 mg (3S,4S)-4-Acetoxy-3-methoxy-2-oxoazetidin (Herstellung siehe unten) in 1,5 ml Phosphatpuffer pH 7 und 0,1 ml Dioxan wird mit 1,5 Aequivalenten einer wässrigen Natriumthioacetat-Lösung versetzt und bei Raumtemperatur 30 Minuten gerührt. Das Reaktionsgemisch wird mit Methylenchlorid extrahiert und die abgetrennte organische Lösung wird anschliessend über Natriumsulfat getrocknet. Das Lösungsmittel wird im Vakuum eingedampft und der Rückstand durch Chromatographie an Silicagel mit Toluol/Aethylacetat 3:1 gereinigt. Das IR-Spektrum der so erhaltenen Titelverbindung (in Methylenchlorid) ist identisch mit demjenigen des gemäss a) erhaltenen Produktes.

Beispiel 56

2-[(3S,4R)-4-Acetylthio-3-methoxy-2-oxoazetidin-1-yl]-2-hydroxyessigsäure-p-nitrobenzylester

Eine Lösung von 350 mg (3S,4R)-4-Acetylthio-3-methoxy-2-oxoazetidin in einer Mischung von 24 ml absolutem Toluol und 6 ml absolutem Dimethylformamid wird mit 1,15 g 2-Aethoxy-2-hydroxy-essigsäure-p-nitrobenzylester und 4 g Molekularsieben A4 versetzt und bei Raumtemperatur unter Stickstoff über Nacht gerührt. Von den Molekularsieben wird abfiltriert und das Filtrat im Vakuum eingedampft. Der Rückstand wird an Silicagel chromatographiert wobei durch Elution mit Toluol und Toluol-Aethylacetat 19:1 die Titelverbindung erhalten wird. IR-Spektrum (in Methylenchlorid): charakteristische Absorptionsbanden bei 2,86; 3,39; 5,60; 5,68; 5,88; 6,21; 6,56; 7,41; 8,26; 9,01; 11,76 $\mu$.

Beispiel 57

2-[(3S,4R)-4-Acetylthio-3-methoxy-2-oxoazetidin-1-yl]-2-chloressigsäure-p-nitrobenzylester

Eine Lösung von 0,6 g 2-[(3S,4R)-4-Acetylthio-3-methoxy-2-oxo-azetidin-1-yl]-2-hydroxyessigsäure-p-nitrobenzylester in 7 ml trockenem Tetrahydrofuran wird auf —15° gekühlt und mit 0,19 ml Thionylchlorid versetzt.

Danach gibt man 0,37 ml Triäthylamin in 0,4 ml trockenem Tetrahydrofuran bei gleicher Temperatur tropfenweise dazu. Das Reaktionsgemisch wird eine Stunde bei 0° gerührt,, mit kaltem Methylenchlorid verdünnt und mit einer eiskalten 2N HCl-Lösung gewaschen. Nach mehrmaligen Ausschütteln mit Wasser wird die Methylenchloridlösung über Natriumsulfat getrocknet und eingedampft. IR-Spektrum (in Methylenchlorid): charakteristische Absorptionsbanden bei 3,41; 5,59; 5,65; 5,88; 6,21; 6,56; 7,41; 8,23; 8,55; 9,05; 10,5; 11,76 $\mu$.

Beispiel 58

2-[(3S,4R)-4-Acetylthio-3-methoxy-2-oxo-azetidin-1-yl]-2-triphenylphosphoranyliden-essigsäure-p-nitrobenzylester.

Eine Lösung von 0,63 2-[3S,4R)-4-Acetylthio-3-methoxy-2-oxoazetidin-1-yl]-2-chloressigsäure-p-nitrobenzylester in 1,8 ml trockenem Tetrahydrofuran wird mit 0,84 g Triphenylphosphin versetzt und über Nacht unter Stickstoff bei Raumtemperatur gerührt. Das Gemisch wird mit Methylenchlorid verdünnt und mit einer kalten, gesättigten, wässrigen Natriumbicarbonatlösung gewaschen. Zusätzliches Waschen mit Wasser, Trocknen über Natriumsulfat und Eindampfen im Vakuum gibt die rohe Titelverbindung die durch Chromatographie an Silicagel mit Toluol-Aethylacetat 19:1 bis 3:1 gereinigt wird. IR-Spektrum (in Methylenchlorid): charakteristische Absorptionsbanden bei 3,40; 5,67; 5,90; 6,20; 6,58; 7,46; 9,05 $\mu$.

Beispiel 59

(5R,6S)-2-Methyl-6-methoxy-2-penem-3-carbonsäure-p-nitrobenzylester

Eine Lösung von 74 mg 2-[(3S,4R)-4-Acetylthio-3-methoxy-2-oxoazetidin-1-yl]-2-triphenylphosphoranyliden-essigsäure-p-nitrobenzylester in 30 ml absolutem Toluol wird mit einer katalytischen Menge 3,5-Di-tert.butyl-4-hydroxytoluol versetzt und 3 Stunden unter Stickstoff am Rückfluss erhitzt. Das Toluol wird im Vakuum abgedampft und der Rückstand an Silicagel mit Toluol-Aethylacetat 19:1 chromatographiert. Die Titelverbindung wird in festem Zustand erhalten. IR-Spektrum (in Methylenchlorid): charakteristische Absorptionsbanden bei 3,41; 5,60; 5,85; 6,63; 6,58; 7,41; 7,60; 8,23; 9,26; 11,76 $\mu$.

Beispiel 60

3-Aethyl-4-(2-acetylaminoäthylthio-thiocarbonylthio)-2-oxoazetidin (racemische cis, trans-Verbindung)

a) Eine Lösung von 0,78 g (5 mM) 4-Acetoxy-3-äthylazetidin-2-on (racemische Mischung von cis- und trans-Isomer im Verhältnis 6:4) in 2 ml Dioxan wird unter Stickstoffatmosphäre tropfenweise zu einer Lösung von 1,175 g Kalium-(2-acetylaminoäthyl)-trithiocarbonat in 20 ml vorgekühltem pH 7-Phosphatpuffer gegeben und 60 Minuten gerührt. Die Reaktionsmischung wird zentrifugiert, die überstehende klare Lösung abdekantiert und der ölige Rückstand in Methylenchlorid aufgenommen. Die organische Phase wird über Natriumsulfat getrocknet und im Vakuum eingedampft. Der rückstand wird einmal mit Diäthyläther trituriert und in dieser Form weiterverarbeitet. DC: Rf = 0,16 (Aethylacetat); IR-Spektrum ($CH_2Cl_2$): Absorptionsbanden bei 2,92; 2,97; 5,63; 5,97; 6,62; 9,35 und 12,34 $\mu$.

Die beiden Ausgangsmaterialien können wir folgt hergestellt werden:

b) Zu einer gerührten Lösung von 34,5 g (0,302 Mol) 1-Butenylacetat in 35 ml trockenem Methylenchlorid werden bei —10° innerhalb 30 Minuten 42,7 g (26,3 ml; 0,302 Mol) N-Chlorsulfonylisocyanat getropft. Nach weiteren 4 Stunden Rühren bei 0° wird die Reaktionsmischung mit 50 ml vorgekühltem Methylenchlorid verdünnt und zu einer hydrolysierenden Mischung von 32 ml Wasser, 144 g Eis, 113 g Natriumhydrogencarbonat und 38,2 g wasserfreiem Natriumsulfit getropft. Während der Hydrolyse wird die Temperatur durch äussere Kühlung bei 0° gehalten. Wenn die organische Phase nicht mehr sauer reagiert, wird die Reaktionsmischung mit 100 ml Diäthyläther verdünnt und durch Celite filtriert. Die organische Phase wird abgetrennt, die wässrige Phase mit dreimal 400 ml Diäthyläther extrahiert, die organischen Phasen vereinigt, getrocknet und im Vakuum eingedampft. Der Rückstand wird an Silicagel mit Toluol/Aethylacetat 2:1 chromatographiert und ergibt eine racemische Mischung von cis- und trans-4-Acetoxy-3-äthylazetidin-2-on im Verhältnis 6:4 in öliger Form. IR-Spektrum ($CH_2Cl_2$): Absorptionsbanden bei 2,94:; 5,60, 5,75; 7,35; 8,06 und 8,85 $\mu$.

c) Eine Lösung von 1,708 g (14,35 mM) 2-Acetylaminoäthylmercaptan in 2 ml absolutem Aethanol wird während 0,5 Stunden unter Rühren und Kühlen auf 10—15° zu einer Lösung von 0,80 g (14,35 mM) Kaliumhydroxid in 5 ml absolutem Aethanol getropft. Nach einer weiteren halben Stunde wird eine Lösung von 1,09 g (14,35 mM) Schwefelkohlenstoff in 3 ml absolutem Aethanol zugefügt, wobei die Temperatur bei 10—15° gehalten wird. Die Reaktionsmischung wird während 3 Stunden bei Raumtemperatur weitergerührt und während 20 Minuten im Eisbad gekühlt. Der gelbe kristalline Niederschlag wird abfiltriert, einmal mit absolutem Aethanol gewaschen und ergibt das Kalium-(2-

acetylaminoäthyl)-trithiocarbonat vom Schmelzpunkt 171—174°. IR-Spektrum (KBr): Absorptionsbanden bei 2,95; 6,18; 6,50; 7,00; 7,32; 7,43; 7,79; 8,33; 9,09 und 11,83 $\mu$.

Beispiel 61

2-[3-Aethyl-4-(2-acetylaminoiäthylthio-thiocarbonylthio)-2-oxo-1-azetidinyl]-2-hydroxy-essigsäure-p-nitrobenzylester (racemische cis, trans-Verbindung)

Bei Raumtemperatur wird eine Lösung von 3,30 g (11 mM) 3-Aethyl-4-(2-acetylaminoäthylthio-thiocarbonylthio-2-oxo-azetidin (racemische cis, trans-Verbindung) in 120 ml Toluol und 32 ml Dimethylformamid mit 4,20 g (16,5 mM) 2-Aethoxy-2-hydroxy-essigsäure-p-nitrobenzylester versetzt. Nach Zugabe von frisch getrockneten Molekularsieben wird die Mischung unter Stockstoff 3 Stunden bei Raumtemperatur gerührt. Die Molekularsiebe werden abfiltriert, mit 20 ml Toluol gewaschen und Filtrat und Waschflüssigkeit zusammen im Vakuum eingedampft. Der Rückstand wird im Hochvakuum getrocknet und dann mit Diäthyläther trituriert zur Entfernung von nicht umgesetztem 2-Aethoxy-2-hydroxy-essigsäure-p-nitrobenzylester. Man erhält die Titelverbindung mit den folgenden physikochemischen Eigenschaften: DC: Rf = 0,16 (Aethylacetat); IR-Spektrum ($CH_2Cl_2$): Absorptionsbanden bei 2,86; 2,92; 3,03; 5,65; 5,71; 5,97; 6,58; 7,41 und 8,37 $\mu$.

Beispiel 62

2-[3-Aethyl-4-(2-acetylaminoäthylthio-thiocarbonylthio)-2-oxo-1-azetidinyl]-2-triphenylphosphoranyliden-essigsäure-p-nitrobenzylester (racemische cis, trans-Verbindung)

Eine Lösung von 5,52 g (11 mM) 2-[3-Aethyl-4-(2-acetylaminoäthylthio-thiocarbonylthio)-2-oxo-1-azetidinyl]-2-hydroxyessigsäure-p-nitrobenzylester (racemische cis, trans-Verbindung) in 30 ml absolutem Tetrahydrofuran wird auf —15° gekühlt, unter Rühren mit 1,02 ml (14 mM) Thionylchlorid und anschliessend langsam mit 1,95 ml (14 mM) Triäthylamin versetzt. Das Reaktionsgemisch wird 20 Minuten bei 0° gerührt, mit 150 ml Methylenchlorid versetzt und mit eiskalter 1N Salzsäure gewaschen. Die organische Phase wird mit Natriumsulfat getrocknet und im Vakuum eingedampft. Der erhaltene rohe 2-[3-Aethyl-4-(2-acetylaminoäthylthiothiocarbonylthio)-2-oxo-1-azetidinyl)-2-chloressigsäure-p-nitrobenzylester wird in 3 ml trockenem Tetrahydrofuran gelöst, mit 6 g Triphenylphosphin versetzt und 24 Stunden bei Raumtemperatur gerührt. Die Reaktionsmischung wird mit 200 ml Methylenchlorid verdünnt, mit gesättigter wässriger Natriumhydrogencarbonatlösung gewaschen, über Natriumsulfat getrocknet und im Vakuum eingedampft. Der Rückstand ergibt nach Chromatographie an Silikagel mit Aethylacetat die Titelverbindung. DC: Rf = 0,19 (Aethylacetat); IR-Spektrum ($CH_2Cl_2$): Absorptionsbanden bei 2,93; 5,70; 5,97; 6,17; 6,58; 6,99; 7,00; 8,07; 8,33 und 9,39 $\mu$.

Beispiel 63

2-(2-Acetylaminoäthylthio)-6-äthyl-2-penem-3-carbonsäure-p-nitrobenzylester (racemische cis- und trans-Verbindung)

Eine Lösung von 1,75 g (2,34 mM) 2-[3-Aethyl-4-(2-acetylaminoäthylthio-thiocarbonylthio)-2-oxo-1-azetidinyl]-2-triphenylphosphoranyliden-essigsäure-p-nitrobenzylester (racemische cis, trans-Verbindung) in 1500 ml trockenem o-Xylol wird mit einer katalytischen Menge Hydrochinon versetzt und unter Stickstoff 7 Stunden unter Rückfluss gerührt. Das Lösungsmittel wird im Vakuum abgedampft und der Rückstand an Silikagel mit Aethylacetat chromatographiert. Man erhält ein Gemisch der cis- und trans-Titelverbindung.

Durch eine Kombination von präparativer Dünnschichtchromatographie (Silicagel mit Methyl-Isobutylketon) und Säulenchromatographie (Silikagel mit Aethylacetat) können die cis- und die trans-Titelverbindung erhalten werden.

*cis-Verbindung*: Schmelzpunkt 141—142° (nach Kristallisation aus Methylenchlorid/Diäthyläther); DC: Rf = 0,62 (Methyl-isobutylketon); IR-Spektrum ($CH_2Cl_2$): Absorptionsbanden bei 2,93; 5,62; 5,98; 6,60; 7,46; 7,57; 8,44 und 9,09 $\mu$.

*trans-Verbindung*: Schmelzpunkt 132—133° (nach Kristallisation aus Methylenchlorid/Diäthyläther); DC: Rf = 0,56 (Methyl-isobutylketon); IR-Spektrum ($CH_2Cl_2$): Absorptionsbanden bei 2,92; 5,62; 5,96; 6,58; 7,44; 7,58; 8,40 und 9,01 $\mu$.

Beispiel 64

2-(2-Acetylaminoäthylthio)-6-äthyl-2-penem-3-carbonsäure (racemische cis- und trans-Verbindung)

a) Eine Lösung von 100 mg (0,22 mM) 2-(2-Acetylaminoäthylthio)-6-äthyl-2-penem-3-carbonsäure-p-nitrobenzylester (racemische cis-Verbindung) in 6 ml absolutem Aethylacetat wird mit 4 ml 0,2 N wässriger Natriumhydrogencarbonatlösung und 200 mg 10%-igem Palladium/Kohle Katalysator versetzt und bei Normaldruck während 60 Minuten unter Wasserstoff gerührt. Die Hydriermischung wird über Diatomeenerde vom Katalysator abfiltriert. Die wässrige Phase wird abgetrennt, mit Diäthyläther gewaschen, mit 5%-iger wässriger Zitronensäurelosung angesäuert und mit Methylenchlorid erschöpfend extrahiert. Die vereinigten Methylenchlorid-Phasen werden über Natriumsulfat getrocknet, filtriert, im Vakuum eingedampft und im Hochvakuum getrocknet. Die erhaltene cis-Titelverbindung hat folgende physikochemischen Eigenschaften: Schmelzpunkt 153—154° (Methylenchlorid/Diäthyläther); IR-Spektrum (KBr): Absorptionsbanden bei 3,08; 3,22; 3,39; 3,42; 3,50; 3,77; 4,08; 5,67; 6,06; 6,21; 6,35; 6,75; 7,04; 7,69; 7,93; 8,27; 9,01; 9,62 und 14,38 $\mu$.

b) Auf die gleiche Weise wird ausgehend von 100 mg 2-(2-Acetylaminoäthylthio)-6-äthyl-2-penem-3-carbonsäure-p-nitrobenzylester (racemische trans-Verbindung) die trans-Titelverbindung erhalten. IR-Spektrum (KBr). Absorptionsbanden bei 3,01; 3,39; 3,44; 5,68; 6,10; 6,85; 7,75; 8,16; 8,47 und 8,93 $\mu$.

Beispiel 65

3-Aethyl-4-(4-p-nitrobenzyloxycarbonylaminobutyrylthio)-2-oxo-azetidin (racemische cis, trans-Verbindung im Verhältnis 1:4)

Eine vorgekühlte Lösung von 3,24 g (20 mM) 3-Aethyl-4-acetoxyazetidin-2-on (racemische cis, trans-Verbindung im Verhältnis 6:4) in 50 ml Dioxan wird tropfenweise mit einer in der Kälte bereiteten Lösung von 7,95 g (26,7 mM) 4-p-Nitrobenzyloxycarbonylamino-thiobuttersäure in 26,7 ml 1N Natriumhydroxidlösung versetzt und bei Raumtemperatur 2 Stunden gerührt. Die Reaktionsmischung wird erschöpfend mit Methylenchlorid extrahiert. Die vereinigten organischen Phasen werden über Natriumsulfat getrocknet und im Vakuum eingedampft. Der Rückstand wird an Silicagel mit Toluol-Aethylacetat 9:1, 4:1 und 1:1 chromatographiert und ergibt die Titelverbindung mit den folgenden physikochemischen Eigenschaften. DC: Rf = 0,10 (Toluol- Aethylacetat 1:1); IR-Spektrum ($CH_2Cl_2$): Absorptionsbanden bei 2,81; 2,92; 5,66; 5,81; 5,94; 6,58; 7,52 und 8,20 $\mu$.

Die als Ausgangsmaterial verwendete Thiocarbonsäure wird wie folgt erhalten:

a) Eine Lösung von 10,30 g (0,1 mM) 4-Aminobuttersäure wird in 300 ml 1N Natriumhydroxidlösung in einem Eisbad tropfenweise innerhalb 20 Minuten mit einer Lösung von 25,87 g (0,12 mM) p-Nitrobenzyl-chloroformat in 100 ml trockenem Dioxan versetzt. Die Reaktionsmischung wird 3 Stunden bei Raumtemperatur gerührt, mit Aethylacetat gewaschen und mit 2N Salzsäure angesäuert. Die ausfallende 4-p-Nitrobenzyloxycarbonylaminobuttersäure wird abfiltriert und aus Aethylacetat umkristallisiert; Schmelzpunkt 145—146°.

b) Zu einer auf —10° gekühlten Lösung von 2,82 g (10 mM) 4-p-Nitrobenzyloxycarbonylaminobuttersäure in 50 ml trockenem Methylenchlorid werden nacheinander 2,2 g (20 mM) Triäthylamin und eine Lösung von 1,4 ml (10 mM) Isobutylchloroformat in 20 ml trockenem Methylenchlorid zugetropft. Die Reaktionsmischung wird eine Stunde gerührt und anschliessend während 2 Stunden ein starker Schwefelwasserstoffstrom durchgeleitet. Nach Ansäuren mit 2N Schwefelsäure wird die organische Phase abgetrennt, getrocknet und im Vakuum eingedampft. Die erhaltene 4-p-Nitrobenzyloxycarbonylamino-thiobuttersäure kann ohne weitere Reinigung weiterverarbeitet werden.

Beispiel 66

2-[3-Aethyl-4-(4-p-Nitrobenzyloxycarbonylaminobutyrylthio)-2-oxo-1-azetidinyl]-2-hydroxyessigsäure-p-nitrobenzylester (racemische cis, trans-Verbindung)

Bei Raumtemperatur werden 6,50 g (16,45 mM) 3-Aethyl-4-(4-p-Nitrobenzyloxycarbonylaminobutyrylthio)-2-oxo-azetidin (racemische cis, trans-Verbindung) und 8,41 g 2-Aethoxy-2-hydroxy-essigsäure-p-nitrobenzylester in 160 ml Toluol und 40 ml Dimethylformamid gelöst. Nach Zugabe von etwa 15 g frisch getrockneten Molekularsieben wird die Mischung unter Stockstoff 3 Stunden bei Raumtemperatur gerührt. Die Molekularsiebe werden abfiltriert und mit Dimethylformamid/Toluol (1:4) gewaschen. Das Filtrat wird im Vakuum eingedampft, am Hochvakuum getrocknet und der Rückstand mit Diäthyläther zur Entfernung von nicht umgesetztem 2-Aethozy-2-hydroxy-essigsäure-p-nitrobenzylester digeriert. Die rohe Titelverbindung hat folgende physikochemischen Eigenschaften: DC: Rf = 0,1 (Toluol-Aethylacetat 1:1), IR-Spektrum ($CH_2Cl_2$): Absorptionsbanden bei 2,83; 2,90; 5,67; 5,73; 5,80; 5,99; 6,58; 7,52; 8,26 und 9,52 $\mu$.

Beispiel 67

2-[3-Aethyl-4-(4-p-nitrobenzyloxycarbonylaminobutyrylthio)-2-oxo-2-azetidinyl]-2-triphenylphosphoranyliden-essigsäure-p-nitrobenzylester (racemische cis, trans-Verbindung)

a) Zu einer Mischung von 10,40 g (17,2 mM) 2-[3-Aethyl-4-(4-p-nitrobenzyloxycarbonylaminobutyrylthio)-2-oxo-1-azetidinyl]-2-hydroxyessigsäure-p-nitrobenzylester in 40 ml absolutem Dioxan werden bei —15° nacheinander 3,06 ml (42 mM) Thionylchlorid und 5,85 ml (42 mM) Triäthylamin getropft. Die Reaktionsmischung wird 20 Minuten bei 0° und unter Stockstoff gerührt, mit 200 ml Methylenchlorid verdünnt und mit gekühlter 1N Salzsäure gewaschen. Die organische Phase wird getrocknet und im Vakuum eingedampft.

b) Der erhaltene rohe 2-[3-Aethyl-4-(4-p-nitrobenzyloxycarbonylaminobutyrylthio)-2-oxo-1-azetidinyl]-2-chloressigsäure-p-nitrobenzylester wird in der minimalen Menge Tetrahydrofuran gelöst, mit 9 g Triphenylphosphin versetzt und über Nacht bei Raumtemperatur unter Stickstoff gerührt. Die Reaktionsmischung wird mit 250 ml Methylenchlorid verdünnt, mit gesättigter wässriger Natriumhydrogencarbonatlösung gewaschen, getrocknet und im Vakuum eingedampft. Der Rückstand wird an Silikagel mit Toluol-Aethylacetat 1:1 chromatographiert und ergibt die Titelverbindung mit den folgenden physiko-chemischen Eigenschaften: DC: Rf 0,05 (Toluol:Aethylacetat 1:1), IR-Spektrum ($CH_2Cl_2$): Absorptionsbanden bei 2,90; 5,73; 5,80; 5,94; 6,58; 7,52; 8,20 und 9,35 $\mu$.

## Beispiel 68

6-Aethyl-2-(3-p-nitrobenzyloxycarbonylaminopropyl)-2-penem-3-carbonsäure-p-nitrobenzylester (racemische cis, trans-Verbindung)

Eine Lösung von 5,40 g (6,36 mM) 2-[3-Aethyl-4-(4-p-nitrobenzyloxycarbonylaminobutyrylthio)-2-oxo-1-azetidinyl]-2-triphenylphosphoranyliden-essigsäure-p-nitrobenzylester (racemische cis, trans-Verbindung in 1500 ml trockenem Toluol wird mit einer katalytischen Menge Hydrochinon versetzt und unter Stickstoff 20 Stunden bei 100° gerührt. Das Lösungsmittel wird im Vakuum abgedampft und der Rückstand an Silikagel mit Toluol-Aethylacetat 4:1 chromatographiert. Man erhält ein Gemisch der cis- und trans-Titelverbindung im Verhältnis 1:10 mit folgenden physikochemischen Eigenschaften: DC: Rf = 0,22 (Toluol-Aethylacetat 1:1); IR-Spektrum ($CH_2Cl_2$): Absorptionsbanden bei 2,90; 5,62; 5,81; 5,85; 6,58; 7,52; 7,87 und 8,20 $\mu$. Durch wiederholte Chromatographie können die cis- und die trans-Verbindung in reiner Form erhalten werden.

## Beispiel 69

6-Aethyl-2-(3-aminopropyl)-2-penem-3-carbonsäure (racemische cis, trans-Verbindung)

Eine Lösung von 2 g (3,5 mM) 6-Aethyl-2-(3-p-nitrobenzyloxycarbonylaminopropyl)-2-penem-3-carbonsäure-p-nitrobenzylester (racemische cis, trans-Verbindung) in 600 ml Dioxan, 330 ml Aethanol und 600 ml Wasser wird mit 2 g Dinatriumhydrogenphosphat und 4 g 10%-igem Palladium/Kohle Katalysator versetzt und bei Normaldruck während einer Stunde unter Wasserstoff gerührt. Die Hydriermischung wird über Diatomeenerde vom Katalysator abfiltriert. Das Filtrat wird mit 3 ml 1500 ml Aethylacetat gewaschen und lyophilisiert. Das Lyophilisat wird zweimal an silyliertem Silikagel (Dünnschichtplatten ANTEC-GEL, OPTI®- UP-$C_{12}$) mit Wasser-Acetonitril 9:1 chromatographiert und ergibt die Titelverbindung (cis:trans etwa 1:10) mit den folgenden physikochemischen Eigenschaften: DC (ANTEC-GEL, UP-$C_{12}$): Rf 0,55 (Wasser-Acetonitril 9:1); IR-Spektrum (KBr): Absorptionsbanden bei 2,94; 3,39; 5,68; 6,41; 7,33; 7,81; 8,93; 12,82 und 14,28 $\mu$.

Ausgehend von der reinen cis- oder trans-Vorstufe können die reinen cis- und trans-Titelverbindungen erhalten werden.

## Beispiel 70

2-[(3S,4S)- und (3S,4R)-4-Acetoxy-3-methoxy-2-oxoazetidin-1-yl]-3-methylenbuttersäure-$\beta,\beta,\beta$-trichloräthylester

200 mg (6S)-6-Methoxy-penicillansäure-$\beta,\beta,\beta$-trichloräthylester-1-oxid werden in 13 ml absolutem Benzol mit 0,114 ml Eisessig und 0,35 ml Trimethylphosphit versetzt und 7 Stunden am Rückfluss erhitzt. Das Lösungsmittel wird am Vakuum abgedampft und der Rückstand durch Chromatographie an Silicagel mit Toluol-Aethylacetat 19:1 und 9:1 gereinigt.

Die Titelverbindungen können so getrennt werden. IR-Spektrum (in Methylenchlorid): charakteristische Absorptionsbanden für (3S,4R)-Isomer (trans-Verbindung): 3,42; 5,62; 5,68; 7,25; 7,35; 8,26; 9,01; 10,93; 11,83 $\mu$; (3S,4S)- Isomer (cis-Verbindung): 3,42; 5,61; 5,7; 7,25; 7,35; 8,21; 9,61; 10,93 $\mu$.

Das Verhältnis von cis-: trans-Verbindung ist etwa 1:1.

## Beispiel 71

2-[(3S,4S)- und (3S,4R)-4-Acetoxy-3-methoxy-2-oxoazetidin-1-yl]-3-methylcrotonsäure-$\beta,\beta,\beta$-trichloräthylester

Eine Lösung von 0,93 g 2-[(3S,4S)- und (3S,4R)-4-Acetoxy-3-methoxy-2-oxo-azetidin-1-yl]-3-methylenbuttersäure-$\beta,\beta,\beta$-trichloräthylester in 60 ml absolutem Methylenchlorid wird auf 0° gekühlt und 10 Minuten mit 0,33 ml Triäthylamin gerührt. Das Reaktionsgemisch wird dann nacheinander mit 4N Phosphorsäure, gesättigter wässriger Natriumbicarbonatlösung und Wassser gewaschen und über Natriumsulfat getrocknet. Das Lösungsmittel wird im Vakuum abgedampft und der Rückstand durch Chromatographie an Silicagel gereinigt. IR-Spektrum (in Methylenchlorid): charakteristische Absorptionsbanden bei: 3,41; 5,60; 5,73; 6,13; 7,19; 7,33; 8,26; 9,09; 9,57; 10,64; 10,87; 12,19 $\mu$.

## Beispiel 72

2-[(3S,4S)- und (3S,4R)-4-Acetoxy-3-methoxy-2-oxoazetidin-1-yl]-2-oxossigsäure-$\beta,\beta,\beta$-trichloräthylester

Durch eine auf −30° gekühlte Lösung von 0,91 g 2-[(3S,4S)- und (3S,4R)-4-Acetoxy-3-methoxy-2-oxoazetidin-1-yl]-3-methylcrotonsäure-$\beta,\beta,\beta$-trichloräthylester in 130 ml Essigsäuremethylester werden 3 Aequivalente Ozon geleitet. Nach der Ozon-Behandlung wird das Gemisch 15 Minuten bei gleicher Temperatur stehengelassen und anschliessend das überschüssige Ozon durch einen Stickstoffstrom entfernt. Das Reaktionsgemisch wird bei 0° mit einer 10%igen wässrigen Natriumsulfitlösung und danach mit Sole gewaschen. Die vereinten wässrigen Lösungen werden noch dreimal mit Essigsäuremethylester rückextrahiert. Die vereinten organische Extrakte werden über Natriumsulfat getrocknet und im Vakuum eingedampft. IR-Spektrum (in Methylenchlorid): charakteristische Absorptionsbanden bei 3,41; 5,46; 5,68; 5,81; 7,27; 7,43; 8,23; 8,40; 9,52; 9,90 $\mu$.

Beispiel 73

(3S,4S)- und (3S,4R)-4-Acetoxy-3-methoxy-2-oxoazetidin

Eine Lösung von 120 mg 2-[(3S,4S)- und (3S,4R)-4-Acetoxy-3-methoxy-2-oxoazetidin-1-yl]-2-oxoessigsäure-β,β,β-tri-chloräthylester in 25 ml Methanol, 3,5 ml Essigsäuremethylester und 0,5 ml Wasser wird während 20 Minuten am Rückfluss erhitzt. Das Lösungsmittel wird im Vakuum abgedampft und der Rückstand ergab nach Chromatographie an Silicagel mit Toluol/Aethylacetat 9:1 das reine (3S,4R)-4-Acetoxy-3-methoxy-2-oxoazetidin, IR-Spektrum (in Methylenchlorid): charakteristische Absorptionsbanden bei 2,96; 3,42; 5,57; 5,73; 7,30; 8,23; 8,70; 8,85; 9,62; 10,0; 10,20 μ, und bei weiterem Eluieren das reine (3S,4S)-4-Acetoxy-3-methoxy-2-oxoazetidin, IR-Spektrum (in Methylenchlorid): charakteristische Absorptionsbanden bei 2,94; 3,41; 5,56; 5,73; 7,35; 7,49; 8,20; 9,52 μ.

Beispiel 74

(3S,4R)-4-(2-p-Nitrobenzyloxycarbonylaminoäthylthio-thiocarbonylthio)-3-methoxy-2-oxoazetidin

Eine Lösung von 159 mg (1 mM) (3S,4S)-4-Acetoxy-3-methoxy-2-oxoazetidin in 3 ml Phosphatpuffer pH 7 und 0,2 ml Dioxan wird bei Raumtemperatur unter Stickstoffatmosphäre tropfenweise mit einer Lösung von 422 mg Kalium-2-p-nitrobenzyloxycarbonylaminoäthyl-trithiocarbonat in 1 ml Wasser versetzt und bei der gleichen Temperatur 30 Minuten gerührt. Die Reaktionsmischung wird erschöpfend mit Methylenchlorid extrahiert. Die kombinierten organischen Phasen werden über Natriumsulfat getrocknet und im Vakuum eingedampft. Der Rückstand wird an Silicagel chromatographiert und liefert die Titelverbindung mit folgendem IR-Spektrum (in Methylenchlorid): charakteristische Absoptionsbanden bei 2,95; 5,62; 5,78; 6,21; 6,56; 7,41; 8,26; 9,25 μ.

Beispiel 75

2-[(3S,4R)-4-(2-p-Nitrobenzyloxycarbonylaminoäthylthio-thiocarbonylthio)-3-methoxy-2-oxo-1-azetidinyl]-2-hydroxy-essigsäure-p-nitrobenzylester

Analog Beispiel 23 werden 646 mg (1,5 mM) (3S,4R)-4-(2-p-Nitrobenzyloxycarbonylaminoäthylthio-thiocarbonylthio)-3-methoxy-2-oxoazetidin in 22 ml absolutem Toluol und 5,5 ml absolutem Dimethylformamid mit 848 mg 2-Aethoxy-2-hydroxyessigsäure-p-nitrobenzylester in Gegenwart von frisch getrockneten Molekularsieben umgesetzt. Nach Aufarbeiten und Chromatographie an Silikagel erhält man die Titelverbindung. IR-Spektrum (in Methylenchlorid): charakteristische Absorptionsbanden bei 5,62; 5,7; 5,78; 6,56; 7,41; 8,26 μ.

Beispiel 76

2-[(3S,4R)-4-(2-p-Nitrobenzyloxycarbonylaminoäthylthio-thiocarbonylthio)-3-methoxy-2-oxo-1-azetidinyl]-2-triphenylphosphoranyliden-essigsäure-p-nitrobenzylester

Analog Beispiel 24 wird eine Lösung von 640 mg 2-[(3S,4R)-4-(2-p-Nitrobenzyloxycarbonylaminoäthylthio - thiocarbonylthio) - 3 - methoxy - 2 - oxo - 1 - azetidinyl] - 2 - hydroxyessigsäure - p - nitrobenzylester in 4,5 ml absolutem Tetrahydrofuran mit 0,12 ml Thionylchlorid und 0,23 ml Triäthylamin in 0,23 ml absolutem Tetrahydrofuran versetzt. Nach Reaktion und Aufarbeiten wird der als Zwischenprodukt erhaltene rohe 2-[(3S,4R)-4-(2-p-Nitrobenzyloxycarbonylaminoäthylthio-thiocarbonylthio)-3-methoxy-2-oxo-1-azetidinyl]-2-chlor-essigsäure-p-nitrobenzylester in 1,15 ml absolutem Tetrahydrofuran mit 0,54 g Triphenylphosphin versetzt. Nach Aufarbeiten und Chromatographie an Silikagel erhält man die Titelverbindung. IR-Spektrum (in Methylenchlorid): charakteristische Absorptionsbanden bei 3,4; 5,7; 5,78; 6,15; 6,55; 7,45; 8,26 μ.

Beispiel 77

(6S,5R)-2-(2-p-Nitrobenzyloxycarbonylaminoäthylthio)-6-methoxy-2-penem-3-carbonsäure-p-nitrobenzylester

Analog Beispiel 25 wird eine Lösung von 500 mg 2-[(3S,4R)-4-(2-p-Nitrobenzyloxycarbonylaminoäthylthio-thiocarbonylthio)-3-methoxy-2-oxo-1-azetidinyl]-2-triphenylphosphoranyliden-essigsäure-p-nitrobenzylester in 165 ml absolutem o-Xylol am Rückfluss gerührt.

Nach Aufarbeiten und Chromatographie an Silikagel mit Toluol-Aethylacetat 19:1 bis 9:1 erhält man die Titelverbindung. IR-Spektrum ($CH_2Cl_2$): Absorptionsbanden bei 5,57; 5,78; 5,9; 6,55; 7,45 und 8,26 μ.

Beispiel 78

(6S,5R)-2-(2-Aminoäthylthio)-6-methoxy-2-penem-3-carbonsäure

Analog Beispiel 69 wird eine Lösung von 295 mg (6S,5R)-2-(2-p-Nitrobenzyloxycarbonylaminoäthylthio)-6-methoxy-2-penem-3-carbonsäure-p-nitrobenzylester in 85 ml Dioxan, 47 ml Aethanol und 85 ml Wasser mit 286 mg Dinatriumhydrogenphosphat und 570 mg eines 10%-igen Palladium-auf-Kohle-Katalysators versetzt und bei Normaldruck unter Wasserstoff gerührt. Nach Reaktion und Aufarbeiten erhält man die Titelverbindung mit folgendem IR-Spektrum (KBr): Absorptionsbanden bei 2,8—4,16; 5,68; 6,41 und 8,26 μ.

Beispiel 79

(3S,4R)-4-(4-p-Nitrobenzyloxycarbonylaminobutyrylthio)-3-methoxy-2-oxoazetidin

Analog Beispiel 55b) werden 159 mg (3S,4S)-4-Acetoxy-3-methoxy-2-oxoazetidin in 6 ml Phosphatpuffer pH 7 und 0,4 ml Dioxan mit einer wässrigen Lösung von 480 mg 4-p-Nitrobenzyloxycarbonylamino-thiobuttersäure-natriumsalz versetzt. Nach Aufarbeiten und Chromatographie an Silikagel erhält man die Titelverbindung mit folgendem IR-Spektrum ($CH_2Cl_2$): charakteristische Absorptionsbanden bei 2,95; 5,6; 5,78; 5,87; 6,56; 7,41 und 8,26 $\mu$.

Beispiel 80

2-[(3S,4R)-4-(4-p-Nitrobenzyloxycarbonylaminobutyrylthio)-3-methoxy-2-oxo-1-azetidinyl]-2-hydroxy-essigsäure-p-nitrobenzylester

Analog Beispiel 23 werden 400 mg (3S,4R)-4-(4-p-Nitrobenzyloxycarbonylaminobutyrylthio)-3-methoxy-2-oxoazetidin in 15 ml absolutem Toluol und 3,7 ml absolutem Dimethylformamid mit 565 mg 2-Aethoxy-2-hydroxy-essigsäure-p-nitrobenzylester im Gegenwart von frisch getrockneten Molekularsieben umgesetzt. Nach Aufarbeiten und Chromatographie an Silikagel erhält man die Titelverbindung mit folgendem IR-Spektrum ($CH_2Cl_2$): charakteristische Absorptionsbanden bei 5,6; 5,7; 5,78; 5,87; 6,56; 7,41 und 8,26 $\mu$.

Beispiel 81

2-[(3S,4R)-4-(4-p-Nitrobenzyloxycarbonylaminobutyrylthio)-3-methoxy-2-oxo-1-azetidinyl]-2-triphenylphosphoranyliden-essigsäure-p-nitrobenzylester

Analog zu Beispiel 24 wird eine Lösung von 606 mg 2-[(3S,4R)-4-p-Nitrobenzyloxycarbonylaminobutyrylthio)-3-methoxy-2-oxo-1-azetidinyl]-2-hydroxy-essigsäure-p-nitrobenzylester in 4,5 ml absolutem Tetrahydrofuran mit 0,12 ml Thionylchlorid und anschliessend 0,23 ml Triäthylamin in 0,23 ml absolutem Tetrahydrofuran versetzt. Nach Reaktion und Aufarbeiten wird der erhaltene rohe 2-[(3S,4R) - 4 - (4 - p - Nitrobenzyloxycarbonylaminobutyrylthio) - 3 - methoxy - 2 - oxo - 1 - azetidinyl] - 2-chloressigsäure - p - nitrobenzylester in 1,15 ml absolutem Tetrahydrofuran mit 0,54 g Triphenylphosphin versetzt. Aufarbeiten und Chromatographie an Silikagel geben die Titelverbindung mit folgendem IR-Spektrum ($CH_2Cl_2$): charakteristische Absorptionsbanden bei 5,7; 5,78; 5,9; 6,15; 6,55; 7,45 und 8,26 $\mu$.

Beispiel 82

(6S,5R)-2-(3-p-Nitrobenzyloxycarbonylaminopropyl)-6-methoxy-2-penem-3-carbonsäure-p-nitrobenzylester

Analog Beispiel 68 wird eine Lösung von 400 mg 2-[(3S,4R)-4-(4-p-Nitrobenzyloxycarbonylaminobutyrylthio - 3 - methoxy - 2 - oxo - 1 - azetidinyl] - 2 - triphenylphosphoranyliden - essigsäure-p - nitrobenzylester in 160 ml absolutem Toluol am Rückfluss gerührt. Nach Aufarbeiten und Chromatographie an Silikagel mit Toluol/Aethylacetat von 19:1 bis 9:1 erhält man die Titelverbindung. IR-Spektrum ($CH_2Cl_2$): charakteristische Absorptionsbanden bei 5,57; 5,78; 5,85; 6,55; 7,45 und 8,26 $\mu$.

Beispiel 83

(6S,5R)-2-(3-Aminopropyl)-6-methoxy-2-penem-3-carbonsäure

Analog Beispiel 69 wird eine Lösung von 572 mg (6S,5R)-2-(3-p-Nitrobenzyloxycarbonylaminopropyl-6-methoxy-2-penem-3-carbonsäure-p-nitrobenzylester in 170 ml Dioxan, 94 ml Aethanol und 170 ml Wasser und mit 571 mg Dinatriumhydrogenphosphat und 1.14 g eines 10%-igen Palladium-auf-Kohle-Katalysators versetzt und bei Normaldruck unter Wasserstoff gerührt. Nach Reaktion und Aufarbeiten erhält man die Titelverbindung mit folgendem IR-Spektrum (KBr): Absorptionsbanden bei 2,75—4,15; 5,67; 6,42 und 8,25 $\mu$.

Beispiel 84

2-[(3S,4S)- und (3S,4R)-4-Chlor-3-methoxy-2-oxoazetidin-1-yl]-3-methylcrotonsäure-$\beta,\beta,\beta$-trichloräthylester

612 mg (6S)-6-Methoxy-penicillansäure-$\beta,\beta,\beta$-trichloräthylester werden in 9 ml absolutem Methylenchlorid bei —80° tropfenweise mit 3,25 ml einer in $CCl_4$ 1,1 M Chlorlösung versetzt. Nach 2 Stunden Rühren bei —80° lässt man das Reaktionsgemisch innert einer Stunde auf Raumtemperatur anwärmen. Das Lösungsmittel wird im Vakuum eingedampft und der Rückstand an Silikagel 10% Wasser chromatographiert. Die Titelverbindungen haben folgendes IR-Spektrum in $CH_2Cl_2$): charakteristische Absorptionsbanden bei 3,41; 5,60; 5,76; 6,15; 7,22; 7,35; 8,33; 9,09; 9,52 und 12,20 $\mu$. In dem erhaltenen Gemisch ist das Verhältnis (3S,4S)-: (3S,4R)-Verbindung 1:10.

Beispiel 85

2-[(3S,4S)- und (3S,4R)-4-Chlor-3-methoxy-2-oxoazetidin-1-yl]-2-oxoessigsäure-$\beta,\beta,\beta$-trichloräthylester

Durch eine auf —35° C gekühlte Lösung von 210 mg 2-[(3S,4S)- und (3S,4R)-4-Chlor-3-methoxy-2-oxoazetidin-1-yl]-3-methylcrotonsäure-$\beta,\beta,\beta$-trichloräthylester in 30 ml Essigsäuremethylester werden 3 Aequivalente Ozon geleitet. Nach der Ozon-Behandlung wird das Gemisch 15 Minuten bei gleicher Temperatur stehengelassen und anschliessend das überschüssige Ozon durch einen Stickstoffstrom entfernt. Das Reaktionsgemisch wird bei 0° mit einer 10%-igen wässrigen Natriumbisulfitlösung und danach mit Sole gewaschen. Die vereinten wässrigen Lösungen werden noch dreimal mit Essigsäuremethylester rückextrahiert. Die organischen Extrakte werden über Natriumsulfat getrocknet und im Vakuum eingedampft. Die rohe Titelverbindung hat folgendes IR-Spektrum (in $CH_2Cl_2$): charakteristische Absorptionsbanden bei 3,41; 5,46; 5,65; 5,80; 7,46; 8,23; 8,47; 8,89; 9,57; 9,95 und 11,90 $\mu$.

Beispiel 86

(3S,4S)- und (3S,4R)-4-Chlor-3-methoxy-2-oxoazetidin

Eine Lösung von 339 mg 2-[(3S,4S)- und (3S,4R)-4-Chlor-3-methoxy-2-oxoazetidin-1-yl]-2-oxo-essigsäure-$\beta,\beta,\beta$-trichloräthylester und 197 mg 2,4-Dinitrophenylhydrazin in 9 ml Tetrahydrofuran wird 30 Minuten am Rückfluss erhitzt. Das Lösungsmittel wir abgedampft und der Rückstand an Silikagel chromatographiert. Die Titelverbindung hat folgendes IR-Spektrum ($CH_2Cl_2$): charakteristische Absorptionsbanden bei 2,94; 5,56; 8,26 und 9,09 $\mu$.

Beispiel 87

(3S,4R)-4-(4-p-Nitrobenzyloxycarbonylaminobutyrylthio)-3-methoxy-2-oxoazetidin

Eine Lösung von 135 mg (3S,4R)-4-Chlor-3-methoxy-2-oxoazetidin in 6 ml Phosphatpuffer pH 7 und 0,4 ml Dioxan wird in Gegenwart von 150 mg Natriumjodid tropfenweise mit einer Lösung von 350 mg 4-p-Nitrobenzyloxycarbonylamino-thiobuttersäurenatriumsalz in 4 ml Wasser versetzt.

Nach 30 Minuten Rühren bei Raumtemperatur wird erschöpfend mit Methylenchlorid extrahiert. Nach Abtrennen und Trocknen der organischen Phase über Natriumsulfat wird das Lösungsmittel im Vakuum abgedampft und der Rückstand an Silikagel chromatographiert. Die Titelverbindung hat folgendes IR-Spektrum ($CH_2Cl_2$): Absorptionsbanden bei 2,95; 5,6; 5,78; 5,87; 6,56; 7,41 und 8,26 $\mu$.

Beispiel 88

Ausgehend von entsprechenden Ausgangsmaterialien und über entsprechende Zwischenprodukte werden analog den voranstehenden Beispielen die folgenden Verbindungen erhalten:

6-Aethyl-2-(2-aminoäthylthio)-2-penem-3-carbonsäure,
6-Hydroxymethyl-2-penem-3-carbonsäure,
6-Hydroxymethyl-2-methyl-2-penem-3-carbonsäure,
6-Hydroxymethyl-2-(3-aminopropyl)-2-penem-3-carbonsäure,
6-Hydroxymethyl-2-(3-acetylaminopropyl)-2-penem-3-carbonsäure,
6-Hydroxymethyl-2-äthylthio-2-penem-3-carbonsäure,
6-Hydroxymethyl-2-(2-aminoäthylthio)-2-penem-3-carbonsäure,
6-Hydroxymethyl-2-(2-acetylaminoäthylthio)-2-penem-3-carbonsäure,
6-(1-Hydroxyäthyl)-2-penem-3-carbonsäure,
6-(1-Hydroxyäthyl)-2-methyl-2-penem-3-carbonsäure,
6-(1-Hydroxyäthyl)-2-(3-aminopropyl)-2-penem-3-carbonsäure,
6-(1-Hydroxyäthyl)-2-(3-acetylaminopropyl)-2-penem-3-carbonsäure,
6-(1-Hydroxyäthyl)-2-äthylthio-2-penem-3-carbonsäure,
6-(1-Hydroxyäthyl)-2-(2-aminoäthylthio)-2-penem-3-carbonsäure,
6-(1-Hydroxyäthyl)-2-(2-acetylaminoäthylthio)-2-penem-3-carbonsäure,
6-Methoxy-2-penem-3-carbonsäure,
6-Methoxy-2-(3-acetylaminopropyl)-2-penem-3-carbonsäure,
6-Methoxy-2-äthylthio-2-penem-3-carbonsäure,
6-Methoxy-2-(2-acetylaminoäthylthio)-2-penem-3-carbonsäure,
6-Methoxy-2-(1,3,4-thiadiazol-2-ylthio)-2-penem-3-carbonsäure,
6-(2-Hydroxyprop-2-yl)-2-penem-3-carbonsäure,
6-(2-Hydroxyprop-2-yl)-2-methyl-2-penem-3-carbonsäure,
6-(2-Hydroxyprop-2-yl)-2-(3-aminopropyl)-2-penem-3-carbonsäure,
6-(2-Hydroxyprop-2-yl)-2-(3-acetylaminopropyl)-2-penem-3-carbonsäure,
6-(2-Hydroxyprop-2-yl)-2-äthylthio-2-penem-3-carbonsäure,
6-(2-Hydroxyprop-2-yl)-2-(2-aminoäthylthio-2-penem-3-carbonsäure,
6-(2-Hydroxyprop-2-yl)-2-(2-acetylaminoäthylthio-2-penem-3-carbonsäure,

sowohl in racemischer als auch in optisch aktiver Form und ihre Salze.

Beispiel 89

Trockenampullen oder Vials, enthaltend 0,5 g 6-Aethyl-2-(3-aminopropyl)-2-penem-3-carbonsäure als Wirksubstanz werden wie folgt hergestellt:

*Zusammensetzung* (für 1 Ampulle oder Vial):

| | |
|---|---|
| Wirksubstanz | 0,5 g |
| Mannit | 0,05 g |

Eine sterile wässrige Lösung der Wirksubstanz und des Mannits wird unter aseptischen Bedingungen in 5 ml.-Ampullen oder 5 ml-Vials der Gefriertrocknung unterworden und die Ampullen bzw. Vials verschlossen und geprüft.

Beispiel 90

Trockenampullen oder Vials, enthaltend 0,25 g 6-Aethyl-2-(3-aminopropyl)-2-penem-3-carbonsäure als Wirksubstanz werden wie folgt hergestellt:

*Zusammensetzung* (für 1 Ampulle oder Vial):

| | |
|---|---|
| Wirksubstanz | 0,25 g |
| Mannit | 0,025 g |

Eine sterile wässrige Lösung der Wirksubstanz und des Mannits wird unter aseptischen Bedingungen in 5 ml.-Ampullen oder 5 ml-Vials der Gefriertrocknung unterworfen und die Ampullen bzw. Vials verschlossen und geprüft.

**Patentansprüche**

1. 2-Penem-3-carbonsäureverbindungen der Formel

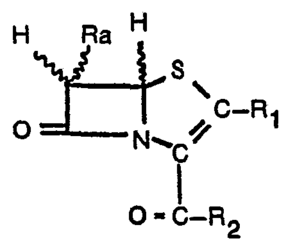

(I)

worin $R_a$ ein gesättigter oder ungesättigter, gegebenenfalls substituierter, aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen,

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel,

in welchen Resten $R_a$ die gegebenenfalls vorhandenen Substituenten Hydroxy, Niederalkoxy, Niederalkanoyloxy, in Salzform vorliegendes Hydroxysulfonyloxy, Halogen, Mercapto, Niederalkylmercapto, Carboxyl, Niederalkoxycarbonyl, Carbamoyl, Cyan, Nitro, in Salzform vorliegendes Sulfo, oder gegebenenfalls durch Niederalkyl oder $C_1$—$C_{20}$-Acyl mono- oder disubstituiertes oder durch Niederalkylen disubstituiertes Amino sind,

gegebenenfalls geschütztes Carboxyl,

Hydroxy,

Mercapto,

durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen veräthertes Hydroxy oder Mercapto, worin

die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$—$C_{20}$-Acyl mono- oder disubstituiertes oder durch Niederalkylen disubstituiertes Amino sind,

durch einen Acylrest einer gegebenenfalls substituierten aliphatischen, cycloaliphatischen, cycloaliphatisch-aliphatischen, aromatischen oder araliphatischen Carbonsäure mit bis zu 18 Kohlenstoffatomen verestertes Hydroxy oder Mercapto, worin

die gegebenenfalls vorhandenen Substituenten Hydroxy, Niederalkoxy, Phenyloxy, Niederalkanoyloxy, Halogen, Mercapto, Niederalkylmercapto, Carboxyl, Niederalkoxycarbonyl, Carbamoyl, Cyan, Nitro, oder gegebenenfalls durch Niederalkyl oder $C_1$—$C_{20}$-Acyl mono- oder disubstituiertes oder durch Niederalkylen disubstituiertes Amino sind, oder

Halogen ist,

$R_1$ Wasserstoff, ein gesättigter oder ungesättigter, gegebenenfalls substituierter aliphatischer, cycloaliphatischer, cycloaliphatisch-aliphatischer, aromatischer oder araliphatischer Kohlenwasserstoffrest mit bis zu 18, bevorzugt bis zu 10 Kohlenstoffatomen, oder

ein gegebenenfalls substituierter Heterocyclyl- oder Heterocyclylniederalkylrest mit bis zu 10 Kohlenstoffatomen und bis zu 4 Ringheteroatomen aus der Gruppe Stickstoff, Sauerstoff und/oder Schwefel ist,

in welchen Resten $R_1$ die gegebenenfalls vorhandenen Substituenten Hydroxy, Niederalkoxy, Niederalkanoyloxy, Halogen, Mercapto, Niederalkylmercapto, gegebenenfalls durch Niederalkyl, Hydroxyniederalkyl, Carboxyniederalkyl, Aminoniederalkyl, Diniederalkylaminoniederalkyl, in Salzform vorliegendes Sulfoniederalkyl, Cycloalkyl, Aryl, Arylniederalkyl, Halogen, Amino, Mono- oder Di-niederalkylamino, Nitro, Hydroxy, Niederalkoxy, Carboxyl, Niederalkoxycarbonyl, Carbamoyl, N-Mono- oder N,N-Diniederalkylcarbamoyl, Cyan, Oxo oder Oxido substituiertes Heterocyclylmercapto, Carboxyl, Niederalkoxycarbonyl, Carbamoyl, Cyan, Nitro, Amino, Niederalkylamino, Diniederalkylamino, $C_1$—$C_{20}$-Acylamino, Di-$C_1$—$C_{20}$-acylamino oder Niederalkylenamino sind, oder worin $R_1$ eine durch einen gegebenenfalls substituierten aliphatischen, cycloaliphatischen, cycloaliphatisch-aliphatischen, aromatischen oder araliphatischen Kohlenwasserstoffrest mit bis zu 18, insbesondere bis zu 10 Kohlenstoffatomen, verätherte Mercaptogruppe ist.

worin die gegebenenfalls vorhandenen Substituenten Hydroxy, Niederalkoxy, Niederalkanoyloxy, Halogen, Mercapto, Niederalkylmercapto, Carboxyl, Niederalkoxycarbonyl, Carbamoyl, Cyan, Nitro, gegebenenfalls durch Niederalkyl oder $C_1$—$C_{20}$-Acyl mono- oder disubstituiertes oder durch Niederalkylen disubstituiertes Amino, Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$—$C_{20}$-Acylamino, oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl oder Oxazolyl sind, oder worin

$R_1$ eine durch einen gegebenenfalls substituierten heterocyclischen Rest verätherte Mercaptogruppe ist, worin der Heterocyclylrest über ein Ringkohlenstoffatom an die Mercaptogruppe gebunden ist und 1 bis 4 Ringstoffatome und gegebenenfalls ein weiteres Ringheteroatom der Gruppe Sauerstoff und Schwefel enthält und worin die gegebenenfalls vorhandenen Substituenten Niederalkyl, durch Hydroxy, Niederalkanoyloxy, Halogen, Carboxy, Niederalkoxycarbonyl, Sulfo, amidiertes Sulfo, Amino, Mono- oder Diniederalkylamino, $C_1$—$C_{20}$-Acylamino oder durch Carboxy oder Halogen substituiertes Niederalkanoylamino substituiertes Niederalkyl, Cycloalkyl, gegebenenfalls durch Halogen oder Nitro substituiertes Phenyl, Benzyl, Furyl, Thienyl, Oxazolyl, Halogen, gegebenenfalls durch Niederalkyl mono- oder disubstituiertes Amino, $C_1$—$C_{20}$-Acylamino, Nitro, Hydroxy, Niederalkoxy, Carboxy, Niederalkoxycarbonyl, gegebenenfalls N-mono- oder N,N-diniederalkyliertes Carbamoyl, Cyan, Oxo oder Oxido sind,

wobei die mit "Nieder" bezeichneten Gruppen bis zu 7, insbesondere bis zu 4 Kohlenstoffatome enthalten, und

$R_2$ Hydroxy oder einen zusammen mit der Carbonylgruppierung —C(=O)— eine geschützte Carboxylgruppe bildenden Rest $R_2^A$ bedeutet, sowie Salzen von solchen Verbindungen mit salzbildenden Gruppen.

2. Verbindungen der Formel I, gemäss Patentanspruch 1, worin —C(=O)—$R_2$ eine unter physiologischen Bedingungen spaltbare, veresterte Carboxylgruppe ist.

3. Verbindungen der Formel I, nach Patentanspruch 1, worin $R_a$ Niederalkyl, Hydroxyniederalkyl, Niederalkoxyniederalkyl, Niederalkanoyloxyniederalkyl, in Salzform vorliegendes Hydroxysulfonyloxyniederalkyl, Hydroxy, Niederalkoxy, Niederalkanoyloxy, Niederalkanoyloxy substituiert durch Phenyloxy, Halogen, Amino oder Cyan, Phenylniederalkanoyloxy, oder durch Hydroxy oder Amino substituiertes Phenylniederalkanoyloxy ist, $R_1$ Wasserstoff, Niederalkyl, Hydroxyniederalkyl, Niederalkoxyniederalkyl, Niederalkanoyloxyniederalkyl, Niederalkylthioniederalkyl, Heterocyclylthioniederalkyl, worin Heterocyclyl einen gegebenenfalls durch Niederalkyl, Carboxyniederalkyl, Diniederalkylaminoniederalkyl oder in Salzform vorliegendes Sulfoniederalkyl substituierten fünfgliedrigen aromatischen diaza-, triaza-, tetraza-, thiaza-, thiadiaza-, thiatriaza, oxaza- oder oxadiazacyclischen Rest darstellt, Aminoniederalkyl, Acylaminoniederalkyl, worin Acyl Niederalkanoyl oder eine als Aminoschutzgruppe gebräuchliche substituierte Oxycarbonylgruppe, z.B. eine tert.-Butyl-, 2,2,2-Trichloräthyl-, Diphenylmethyl- oder p-Nitrobenzyl-oxycarbonylgruppe, ist, Carboxyniederalkyl, Niederalkoxycarbonylniederalkyl, Phenylniederalkyl, Phenyl, Hydroxyphenyl, Aminophenyl, Furyl, Thienyl, Pyridyl, Niederalkylthio, durch Amino, Mono- oder Diniederalkylamino oder Niederalkanoylamino substituiertes Niederalkylthio oder Niederalkenylthio, oder gegebenenfalls durch Niederalkyl, Sulfoniederalkyl, Carboxyniederalkyl oder Diniederalkylaminoniederalkyl substituiertes Tetrazolylthio oder Thiadiazolylthio darstellt, und $R_2$ für Hydroxy, gegebenenfalls $\alpha$-polyverzweigtes Niederalkoxy, 2-Halogenniederalkoxy, Phenacyloxy, 1-Phenylniederalkoxy mit 1—3, gegebenenfalls durch Niederalkoxy und/oder Nitro substituierten Phenylresten,

Acetonyloxy, 2-Cyanäthoxy, 2-Triniederalkylsilyläthoxy, Niederalkanoyloxymethoxy, α-Aminoniederalkanoyloxymethoxy, Phthalidyloxy, Pentachlorphenyloxy, Triniederalkylsilyloxy oder Niederalkenyloxy steht, wobei die mit "Nieder" bezeichneten Gruppen bis zu 7, insbesondere bis zu 4 Kohlenstoffatome enthalten, und Salze von solchen Verbindungen mit salzbildenden Gruppen.

4. Verbindungen der Formel I, nach Patentanspruch 1, worin $R_a$ Niederalkyl, 1-Hydroxyniederalkyl Phenylniederalkyl, Phenoxyniederalkanoyloxy oder Niederalkoxy ist und $R_1$ Wasserstoff, Niederalkyl, Aminoniederalkyl, Acylaminoniederalkyl, worin Acyl Niederalkanoyl oder eine als Aminoschutzgruppe gebräuchliche substituierte Oxycarbonylgruppe, z.B. eine tert.-Butyl-2,2,2-Trichloräthyl-, Diphenylmethyl- oder p-Nitrobenzyl-oxycarbonylgruppe, ist, Niederalkylthio, durch Amino, Mono- oder Diniederalkylamino oder Niederalkanoylamino substituiertes Niederalkylthio oder Niederalkenylthio, 1-Methyl-1H-tetrazol-5-ylthio, 1-(2-Dimethylaminoäthyl)-1H-tetrazol-5-ylthio, 2-Methyl-1,3,4-thiadiazol-5-ylthio oder 1,3,4-Thiadiazol-2-ylthio bedeutet, und worin Niederalkyl-, Niederalkenyl- und Niederalkanoylgruppen bis zu 4 Kohlenstoffatome enthalten, die unter neutralen, basischen oder physiologischen Bedingungen spaltbaren Ester und die Salze von solchen Verbindungen mit salzbildenen Gruppen.

5. Die (5R)-Verbindungen der Formel I, nach einem der Patentansprüche 1—5.

6. 2,6-Dimethyl-2-penem-3-carbonsäure (racemisches cis, trans-Gemisch) nach Patentanspruch 1.

7. 2,6-Dimethyl-2-penem-3-carbonsäure Natriumsalz (racemisches cis, trans-Gemisch) nach Patentanspruch 1.

8. 2,6-Dimethyl-2-penem-3-carbonsäure (racemische trans-Verbindung) nach Patentanspruch 1.

9. 2-Methyl-6-isopropyl-2-penem-3-carbonsäure (racemische trans-Verbindung) nach Patentanspruch 1.

10. 2-Methyl-6-benzyl-2-penem-3-carbonsäure (racemische trans-Verbindung) nach Patentanspruch 1.

11. 2-Aethylthio-6-isopropyl-2-penem-3-carbonsäure (racemische trans-Verbindung) nach Patentanspruch 1.

12. (5R,6S)-2-Methyl-6-methoxy-2-penem-3-carbonsäure, nach Patentanspruch 1.

13. (5R,6S)-2-Methyl-6-phenoxyacetoxy-2-penem-3-carbonsäure nach Patentanspruch 1.

14. 2-(2-Acetylaminoäthylthio)-6-äthyl-2-penem-3-carbonsäure (racemische cis-Verbindung) nach Patentanspruch 1.

15. 2-(2-Acetylaminoäthylthio)-6-äthyl-2-penem-3-carbonsäure (racemische trans-Verbindung) nach Patentanspruch 1.

16. 6-Aethyl-2-(3-aminopropyl)-2-penem-3-carbonsäure (racemische cis, trans-Verbindung) nach Patentanspruch 1.

17. (6S,5R)-2-(2-Aminoäthylthio)-6-methoxy-2-penem-3-carbonsäure nach Patentanspruch 1.

18. (6S,5R)-2-(3-Aminopropyl)-6-methoxy-2-penem-3-carbonsäure nach Patentanspruch 1.

19. 6-Hydroxymethyl-2-penem-3-carbonsäure in racemischer und in optisch aktiver Form nach Patentanspruch 1.

20. 6-Hydroxymethyl-2-methyl-2-penem-3-carbonsäure in racemischer und optisch aktiver Form nach Patentanspruch 1.

21. 6-Hydroxymethyl-2-äthylthio-2-penem-3-carbonsäure in racemischer und in optisch aktiver Form nach Patentanspruch 1.

22. 6-(1-Hydroxyäthyl)-2-penem-3-carbonsäure in racemischer und in optisch aktiver Form nach Patentanspruch 1.

23. 6-(1-Hydroxyäthyl)-2-methyl-2-penem-3-carbonsäure in racemischer und in optisch aktiver Form nach Patentanspruch 1.

24. 6-(1-Hydroxyäthyl)-2-äthylthio-2-penem-3-carbonsäure in racemischer und in optisch aktiver Form nach Patentanspruch 1.

25. 6-(1-Hydroxyäthyl)-2-(2-Aminoäthylthio)-2-penem-3-carbonsäure in racemischer und in optisch aktiver Form nach Patentanspruch 1.

26. Pharmazeutisch verwendbare Salze von Verbindungen der Patentansprüche 1—25.

27. Pharmazeutische Präparate enthaltend eine der in den Patentansprüchen 1—26 beanspruchten Verbindungen.

28. Die in den Patentansprüchen 1—27 genannten Stoffe und Stoffgemische zur Bekämpfung von Mikroorganismen.

29. Verfahren zur Herstellung von 2-Penem-3-carbonsäure-verbindungen der Formel

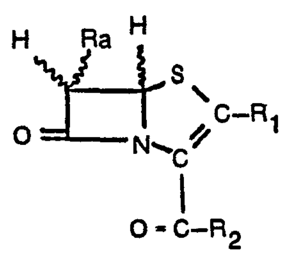

(I)

worin $R_a$, $R_1$ und $R_2$ die im Anspruch 1 genannten Bedeutungen haben, sowie Salzen von solchen Verbindungen mit salzbildenen Gruppen, dadurch gekennzeichnet, dass man eine Ylid-Verbindung der Formel

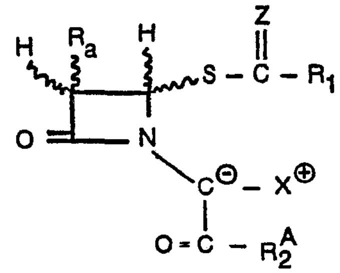

(II)

worin $R_a$, $R_1$ und $R_2^A$ die oben gegebenen Bedeutungen haben, wobei funktionelle Gruppen in diesen Resten vorzugsweise in geschützter Form vorliegen, Z Sauerstoff oder Schwefel bedeutet, und worin $X^\oplus$ entweder eine dreifach substituierte Phosphoniogruppe oder eine zweifach veresterte Phosphonogruppe zusammen mit einem Kation bedeutet, ringschliesst, und, wenn erwünscht oder notwendig, in einer erhaltenen Verbindung der Formel I die geschütze Carboxylgruppe der Formel —C(=O)—$R_2^A$ in die freie oder in eine andere geschützte Carboxylgruppe überführt, und/oder, wen erwünscht, in einer erhaltenen Verbindung der Formel I innerhalb der Definition eine Gruppe $R_a$, und/oder $R_1$ in eine andere Gruppe $R_a$, und/oder $R_1$ überführt, und/oder, wenn erwünscht, eine erhaltene Verbindung mit salzbildender Gruppe in ein Salz oder ein erhaltenes Salz in die freie Verbindung oder in ein anderes Salz überführt, und/oder, wenn erwünscht, ein erhaltenes Gemisch von isomeren Verbindungen in die einzelnen Isomeren auftrennt.

**Claims**

1. A 2-penem-3-carboxylic acid compound of the formula

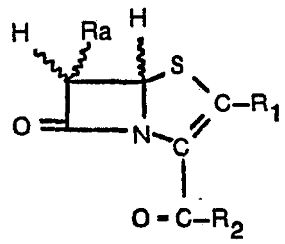

(I)

wherein $R_a$ is a saturated or unsaturated, unsubstituted or substituted, aliphatic, cycloaliphatic, cycloaliphatic-aliphatic, aromatic or araliphatic hydrocarbon radical having up to 18, preferably up to 10, carbon atoms, or an unsubstituted or substituted heterocyclyl or heterocyclyl lower alkyl radical having up to 10 carbon atoms and up to 4 ring hetero atoms selected from the group: nitrogen, oxygen and/or sulfur, in which radicals $R_a$ the optionally present substituents are: hydroxyl, lower alkoxy, lower alkanoyloxy, hydroxysulfonyloxy present in salt form, halogen, mercapto, lower alkylmercapto, carboxyl, lower-alkoxycarbonyl, carbamoyl, cyano, nitro, sulfo present in salt form, or amino unsubstituted or mono- or disubstituted by lower alkyl or $C_1$—$C_{20}$-acyl, or amino disubstituted by lower alkylene, or $R_a$ is optionally protected carboxyl, hydroxyl, mercapto, or hydroxyl or mercapto etherified by an unsubstituted or substituted, aliphatic, cycloaliphatic, cycloaliphatic-aliphatic, aromatic or araliphatic hydrocarbon radical having up to 18, especially up to 10, carbon atoms, wherein the optionally present substituents are hydroxyl, lower alkoxy, lower alkanoyloxy, halogen, mercapto, lower alkylmercapto, carboxyl, lower alkoxycarbonyl, carbamoyl, cyano, nitro, or amino unsubstituted or mono- or disubstituted by lower alkyl or $C_1$—$C_{20}$-acyl, or amino disubstituted by lower alkylene, or $R_a$ is hydroxyl or mercapto esterified by an acyl radical of an unsubstituted or substituted aliphatic, cycloaliphatic, cycloaliphatic-aliphatic, aromatic or araliphatic carboxylic acid having up to 18 carbon atoms, wherein the optionally present substituents are: hydroxyl, lower alkoxy, phenyloxy, lower alkanoyloxy, halogen, mercapto, lower alkylmercapto, carboxyl, lower alkoxycarbonyl, carbamoyl, cyano, nitro, or amino unsubstituted or mono- or disubstituted by lower alkyl or $C_1$—$C_{20}$-acyl, or amino disubstituted by lower alkylene, or halogen,

$R_1$ is hydrogen, a saturated or unsaturated, unsubstituted or substituted, aliphatic, cycloaliphatic, cycloaliphatic-aliphatic, aromatic or araliphatic hydrocarbon radical having up to 18, preferably up to 10, carbon atoms, or $R_1$ is an unsubstituted or substituted heterocyclyl or heterocyclyl-lower-alkyl radical having up to 10 carbon atoms and up to 4 ring hetero atoms selected from the group: nitrogen, oxygen and/or sulfur, in which radicals $R_1$ the optionally present substituents are hydroxyl, lower alkoxy, lower alkanoyloxy, halogen, mercapto, lower alkylmercapto, heterocyclylmercapto unsubstituted or

substituted by lower alkyl, hydroxy-lower-alkyl, carboxy-lower-alkyl, amino-lower-alkyl, di-lower-alkylamino-lower-alkyl, sulfo-lower-alkyl present in salt form, cycloalkyl, aryl, aryl-lower-alkyl, halogen, amino, mono- or di-lower-alkylamino, nitro, hydroxyl, lower alkoxy, carboxyl, lower-alkoxycarbonyl, carbamoyl, N-mono- or N,N-di-lower-alkylcarbamoyl, cyano, oxo or oxido, or carboxyl, lower-alkoxy-carbonyl, carbamoyl, cyano, nitro, amino, lower alkylamino, di-lower-alkylamino, $C_1$—$C_{20}$-acylamino, di-$C_1$—$C_{20}$-acylamino or lower-alkyleneamino, or wherein $R_1$ is a mercapto group etherified by an unsubstituted or substituted aliphatic, cycloaliphatic, cycloaliphatic-aliphatic, aromatic or araliphatic hydrocarbon radical having up to 18, especially up to 10 carbon atoms, wherein the optionally present substituents are: hydroxyl, lower alkoxy, lower-alkanoyloxy, halogen, mercapto, lower alkylmercapto, carboxyl, lower alkoxycarbonyl, carbamoyl, cyano, nitro, amino unsubstituted or mono- or disubstituted by lower alkyl or $C_1$—$C_{20}$-acyl, or amino disubstituted by lower alkylene, or lower alkyl, or lower alkyl substituted by hydroxyl, lower alkanoyloxy, halogen, carboxyl, lower-alkoxycarbonyl, sulfo, amidated sulfo, amino, mono- or di-lower-alkylamino, $C_1$—$C_{20}$-acylamino, or lower alkanoylamino substituted by carboxyl or halogen, or cycloalkyl, or phenyl unsubstituted or substituted by halogen or nitro, or benzyl, furyl, thienyl or oxazolyl, or wherein $R_1$ is a mercapto group etherified by an unsubstituted or substituted heterocyclic radical, wherein the heterocyclyl radical is bound by way of a ring carbon atom to the mercapto group and contains 1 to 4 ring carbon atoms and optionally a further ring hetero atom of the group oxygen and sulfur, and wherein the optionally present substituents are: lower alkyl, lower alkyl substituted by hydroxyl, lower alkanoyloxy, halogen, carboxyl, lower alkoxycarbonyl, sulfo, amidated sulfo, amino, mono- or di-lower-alkylamino, $C_1$—$C_{20}$-acylamino or lower alkanoylamino substituted by carboxyl or halogen, cycloalkyl, phenyl unsubstituted or substituted by halogen or nitro, or benzyl, furyl, thienyl, oxazolyl, halogen, amino unsubstituted or mono- or disubstituted by lower alkyl, $C_1$—$C_{20}$-acylamino, nitro, hydroxyl, lower alkoxy, carboxyl, lower alkoxycarbonyl, optionally N-mono- or N,N-di-lower-alkylated carbamoyl, cyano, oxo or oxido, the groups denoted by "lower" having up to 7, especially up to 4, carbon atoms; and $R_2$ is hydroxyl, or a radical $R_2^A$ forming together with the carbonyl grouping —C(=O)— a protected carboxyl group; and salts of such compounds having salt-forming groups.

2. A compound of the formula I according to Claim 1, wherein —C(=O)—$R_2$ is an esterified carboxyl group which can be split under physiological conditions.

3. A compound of the formula I according to Claim 1, wherein $R_a$ is lower alkyl, hydroxy-lower-alkyl, lower alkoxy-lower-alkyl, lower alkanoyloxy-lower-alkyl, hydroxysulfonyloxy-lower-alkyl present in salt form, hydroxyl, lower alkoxy, lower alkanoyloxy, lower alkanoyloxy substituted by phenyloxy, halogen, amino or cyano, phenyl-lower-alkanoyloxy, or phenyl-lower-alkanoyloxy substituted by hydroxyl or amino; $R_1$ is hydrogen, lower alkyl, hydroxy-lower-alkyl, lower-alkoxy-lower-alkyl, lower alkanoyl-lower-alkyl, lower alkylthio-lower-alkyl, heterocyclylthio-lower-alkyl, wherein heterocyclyl is a five-membered, aromatic, diaza-, triaza-, tetraza-, thiaza-, thiadiaza- thiatriaza, oxaza- or oxadiazacyclic radical each unsubstituted or substituted by lower alkyl, carboxy-lower-alkyl, di-lower-alkylamino-lower-alkyl, or sulfo-lower-alkyl present in salt form, or $R_1$ is amino-lower-alkyl, acylamino-lower-alkyl wherein acyl is lower alkanoyl or a substituted oxycarbonyl group that can be used as an amino-protective group, for example a tert-butyl-, 2,2,2-trichloroethyl-, diphenylmethyl- or p-nitrobenzyloxycarbonyl group, or carboxy-lower-alkyl, lower alkoxy-carbonyl-lower-alkyl, phenyl-lower-alkyl, phenyl, hydroxyphenyl, aminophenyl, furyl, thienyl, pyridyl, lower alkylthio, lower alkylthio or lower alkenylthio each substituted by amino, mono- or di-lower-alkylamino or lower alkanoylamino, or tetrazolylthio or thiadiazolylthio each unsubstituted or substituted by lower alkyl, sulfo-lower-alkyl, carboxy-lower-alkyl or di-lower-alkylamino-lower-alkyl; and $R_2$ is hydroxyl, optionally $\alpha$-polybranched lower alkoxy, 2-halo-lower-alkoxy, phenacyloxy, 1-phenyl-lower-alkoxy having 1—3 phenyl radicals unsubstituted or substituted by lower alkoxy and/or nitro, or acetonyloxy, 2-cyanoethoxy, 2-tri-lower-alkylsilylethoxy, lower alkanoyloxymethoxy, $\alpha$-amino-lower-alkanoyloxymethoxy, phthalidyloxy, pentachlorophenyloxy, tri-lower-alkylsilyloxy or lower alkenyloxy, whereby the groups denoted by "lower" contain up to 7, especially up to 4, carbon atoms; and salts of such compounds having salt-forming groups.

4. A compound of the formula I according to Claim 1, wherein $R_a$ is lower alkyl, 1-hydroxy-lower-alkyl, phenyl-lower-alkyl, phenoxy-lower-alkanoyloxy or lower alkoxy, and $R_1$ is hydrogen, lower alkyl, amino-lower-alkyl, acylamino-lower-alkyl, wherein acyl is lower alkanoyl or a substituted oxycarbonyl group that can be used as an amino-protective group, for example a tert-butyl-2,2,2-trichloroethyl-, diphenylmethyl- or p-nitrobenzyloxycarbonyl group, or it is lower alkylthio, lower alkylthio or lower alkenylthio each substituted by amino, mono- or di-lower-alkylamino or lower alkanoylamino, or it is 1-methyl-1H-tetrazol-5-ylthio, 1-(2-dimethylaminoethyl)-1H-tetrazol-5-ylthio, 2-methyl-1,3,4-thiadiazol-5-ylthio or 1,3,4-thiadiazol-2-ylthio, and wherein lower alkyl, lower alkenyl and lower alkanoyl groups contain up to 4 carbon atoms; the esters which can be split under neutral, basic or physiological conditions; and the salts of such compounds having salt-forming groups.

5. The (5*R*) compounds of the formula I, according to any one of Claims 1—5.

6. 2,6-Dimethyl-2-penem-3-carboxylic acid (racemic *cis-trans* mixture) according to Claim 1.

7. 2,6-Dimethyl-2-penem-3-carboxylic acid sodium salt (racemic *cis-trans* mixture) according to Claim 1.

8. 2,6-Dimethyl-2-penem-3-carboxylic acid (racemic *trans*-compound) according to Claim 1.

9. 2-Methyl-6-isopropyl-2-penem-3-carboxylic acid (racemic *trans*-compound) according to Claim 1.

10. 2-Methyl-6-benzyl-2-penem-3-carboxylic acid (racemic *trans*-compound) according to Claim 1.

11. 2-Ethylthio-6-isopropyl-2-penem-3-carboxylic acid (racemic *trans*-compound) according to Claim 1.

12. (5*R*,6*S*)-2-methyl-6-methoxy-2-penem-3-carboxylic acid according to Claim 1.

13. (5*R*,6*S*)-2-Methyl-6-phenoxyacetoxy-2-penem-3-carboxylic acid according to Claim 1.

14. 2-(2-Acetylaminoethylthio)-6-ethyl-2-penem-3-carboxylic acid (racemic *cis*-compound) according to Claim 1.

15. 2-(2-Acetylaminoethylthio)-6-ethyl-2-penem-3-carboxylic acid (racemic *trans*-compound) according to Claim 1.

16. 6-Ethyl-2-(3-aminopropyl)-2-penem-3-carboxylic acid (racemic *cis-trans* compound) according to Claim 1.

17. (6*S*,5*R*)-2-(2-Aminoethylthio)-6-methoxy-2-penem-3-carboxylic acid according to Claim 1.

18. (6*S*,5*R*)-2-(3-aminopropyl)-6-methoxy-2-penem-3-carboxylic acid according to Claim 1.

19. 6-Hydroxymethyl-2-penem-3-carboxylic acid in racemic and in optically active form according to Claim 1.

20. 6-Hydroxymethyl-2-methyl-2-penem-3-carboxylic acid in racemic and optically active form according to Claim 1.

21. 6-Hydroxymethyl-2-ethylthio-2-penem-3-carboxylic acid in racemic and in optically active form according to Claim 1.

22. 6-(1-Hydroxyethyl)-2-penem-3-carboxylic acid in racemic and in optically active form according to Claim 1.

23. 6-(1-Hydroxyethyl)-2-methyl-2-penem-3-carboxylic acid in racemic and in optically active form according to Claim 1.

24. 6-(1-Hydroxyethyl-2-ethylthio)-2-penem-3-carboxylic acid in racemic and in optically active form according to Claim 1.

25. 6-(1-Hydroxyethyl)-2-(2-aminoethylthio)-2-penem-3-carboxylic acid in racemic and in optically active form according to Claim 1.

26. Pharmaceutically acceptable salts of compounds of Claims 1—25.

27. Pharmaceutical preparations containing one of the compounds claimed in any one of Claims 1—26.

28. The substances and mixtures of substances stated in Claims 1—27 for controlling microorganisms.

29. A process for producing a 2-penem-3-carboxylic acid compound of the formula

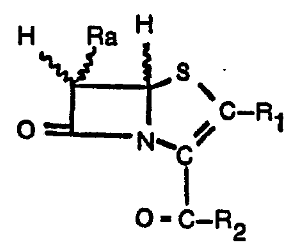

(I)

wherein $R_a$, $R_1$ and $R_2$ have the meanings defined in Claim 1, and also salts of such a compound having salt-forming groups, characterised by ring-closing an ylid compound of the formula

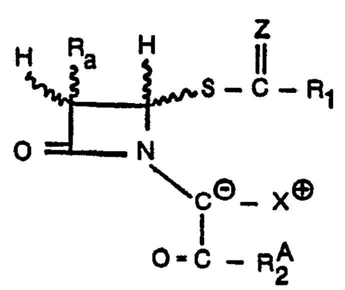

(II)

wherein $R_a$, $R_1$ and $R_2^A$ have the meanings defined in the foregoing, wherein functional groups in these radicals are preferably present in protected form, Z is oxygen or sulfur, and wherein $X^{\oplus}$ is either a threefold substituted phosphonio group or a twofold esterified phosphono group together with a cation; and, if desired or necessary, converting in a resulting compound of the formula I the protected carboxyl group of the formula —C(=O)—$R_2^A$ into the free carboxyl group or into another protected carboxyl

group; and/or, if desired, in a resulting compound of the formula I converting a group $R_a$ and/or $R_1$ within the definition into a different group $R_a$ and/or $R_1$; and/or, if desired, converting a resulting compound having a salt-forming group into a salt, or a salt obtained into the free compound or into a different salt; and/or, if desired, separating a resulting mixture of isomeric compounds into the individual isomers.

**Revendications**

1. Composés d'acide 2-pénem-3-carboxylique de formule

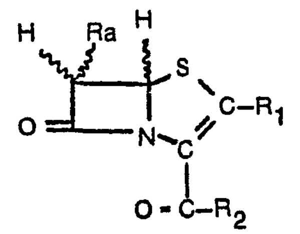

(I)

où $R_a$ représente un radical hydrocarboné saturé ou insaturé, éventuellement substitué, aliphatique, cycloaliphatique, cycloaliphatique-aliphatique, aromatique ou araliphatique ayant jusqu'à 18, de préférence jusqu'à 10 atomes de carbone, un radical hétérocyclyle ou hétérocyclyl-alcoyle inférieur éventuellement substitué ayant jusqu'à 10 atomes de carbone et jusqu'à 4 hétéroatomes dans son noyau, du groupe constitué par l'azote, l'oxygène et/ou le soufre,

dans lesquels radicaux $R_a$ les substituants éventuellement présents sont un hydroxy, un alcoxy inférieur, un alcanoyloxy inférieur, un hydroxysulfonyloxy présent sous forme de sel, un halogène, un mercapto, un alcoyle inférieur-mercapto, un carboxyle, un alcoxy inférieur-carbonyle, un carbamoyle, un cyano, un nitro, un sulfo présent sous forme de sel ou un amino éventuellement mono- ou disubstitué par un alcoyle inférieur ou un acyle en $C_1$ à $C_{20}$ ou disubstitué par un alcoylène inférieur,

un carboxyle éventuellement protégé,

un hydroxy,

un mercapto,

un hydroxy ou un mercapto éthérifié par un radical hydrocarboné éventuellement substitué aliphatique, cycloaliphatique, cycloaliphatique-aliphatique, aromatique or araliphatique, ayant jusqu'à 18, en particulier jusqu'à 10, atomes de carbone, où les substituants éventuellement présents sont un hydroxy, un alcoxy inférieur, un alcanoyloxy inférieur, un halogène, un mercapto, un alcoyle inférieur-mercapto, un carboxyle, un alcoxy inférieur-carbonyle, un carbamoyle, un cyano, un nitro, ou un amino éventuellement mono- ou disubstitué par un alcoyle inférieur ou un acyle en $C_1$ à $C_{20}$ ou disubstitué par un alcoylène inférieur,

un hydroxy ou un mercapto estérifié par un radical alcyle d'un acide carboxylique éventuellement substitué aliphatique, cycloaliphatique, cycloaliphatique-aliphatique, aromatique ou araliphatique ayant jusqu'à 18 atomes de carbone, où les substituants éventuellement présents sont un hydroxy, un alcoxy inférieur, un phényloxy, un alcanoyloxy inférieur, un halogène, un mercapto, un alcoyle inférieur-mercapto, un carboxyle, un alcoxy inférieur-carbonyle, un carbamoyle, un cyano, un nitro, ou un amino éventuellement mono- ou disubstitué par un alcoyle inférieur ou un acyle en $C_1$ à $C_{20}$ ou disubstitué par un alcoylène inférieur, ou

un halogène,

$R_1$ représente un hydrogène, un radical hydrocarboné saturé ou insaturé, éventuellement substitué, aliphatique, cycloaliphatique-aliphatique, aromatique ou araliphatique ayant jusqu'à 18, de préférence jusqu'à 10, atomes de carbone, ou

un radical hétérocyclyle ou hétérocyclique-alcoyle inférieur éventuellement substitué ayant jusqu'à 10 atomes de carbone et jusqu'à 4 hétéroatomes dans le noyau, du groupe constitué par l'azote, l'oxygène et/ou le soufre,

dans lesquels radicaux $R_1$ les substituants éventuellement présents sont un hydroxy, un alcoxy inférieur, un alcanoyloxy inférieur, un halogène, un mercapto, un alcoyle inférieur-mercapto, un hétérocyclylmercapto éventuellement substitué par un alcoyle inférieur, un hydroxyalcoyle inférieur, un carboxyalcoyle inférieur, un aminoalcoyle inférieur, un dialcoyle inférieur-amino-alcoyle inférieur, un sulfoalcoyle inférieur se présentant sous forme de sel, un cycloalcoyle, un aryle, un arylalcoyle inférieur, un halogène, un amino, un mono- ou un di-alcoyle inférieur-amino, un nitro, un hydroxy, un alcoxy inférieur, un carboxyle, un alcoxy inférieur-carbonyle, un carbamoyle, un N-mono- ou N,N-dialcoyle inférieurcarbamoyle, un cyano, un oxo ou un oxydo; un carboxyle, un alcoxy inférieur-carbonyle, un carbamoyle, un cyano, un nitro, un amino, un alcoyle inférieur-amino, un dialcoyle inférieur-amino, un acylamino en $C_1$ à $C_{20}$, un diacylamino en $C_1$ à $C_{20}$ ou un alcoylèneamino inférieur, ou bien où $R_1$ est un groupe mercapto éthérifié par un radical hydrocarboné éventuellement substitué, aliphatique, cycloaliphatique, cycloaliphatique-aliphatique, aromatique ou araliphatique ayant jusqu'à 18 atomes, en particulier jusqu'à 10, atomes de carbone.

où les substituants éventuellement présents sont un hydroxy, un alcoxy inférieur, un alcanoyloxy inférieur, un halogène, un mercapto, un alcoyle inférieur-mercapto, un carboxyle, un alcoxy inférieur-carbonyle, un carbamoyle, un cyano, un nitro, un amino éventuellement mono- ou disubstitué par un alcoyle inférieur ou un acyle en $C_1$ à $C_{20}$ ou disubstitué par un alcoylène inférieur, un alcoyle inférieur, un alcoyle inférieur substitué par un hydroxy, un alcanoyloxy inférieur, un halogène, un carboxy, un alcoxy inférieur-carbonyle, un sulfo, un sulfo amidé, un amino, un mono- ou un dialcoyle inférieur-amino, un acylamino en $C_1$ à $C_{20}$, ou par un carboxy ou un alcanoyle inférieur-amino halogéno-substitué; un cycloalcoyle, un phényle éventuellement substitué par un halogène ou un nitro, un benzyle, un furyle, un thiényle ou un oxazolyle, ou bien où $R_1$ est un groupe mercapto éthérifié par un radical hétérocyclique éventuellement substitué, où le radical hétérocyclyle est lié par l'intermédiaire d'un atome de carbone du noyau au groupe mercapto et contient de 1 à 4 atomes de carbone nucléaire et éventuellement un autre hétéroatome cyclique du groupe constitué par l'oxygène et le soufre et où les substituants éventuellement présents sont un alcoyle inférieur, un alcoyle inférieur substitué par un hydroxy, un alcanoyloxy inférieur, un halogène, un carboxy, un alcoxy inférieur-carbonyle, un sulfo, un sulfo amidé, un amino, un mono- ou dialcoyle inférieur-amino, un acylamino en $C_1$ à $C_{20}$ ou un alcanoyle inférieur amino substitué par un carboxy ou un halogène, un cycloalcoyle, un phényle éventuellement substitué par un halogène ou un nitro, un benzyle, un furyle, un thiényle, un oxazolyle, un halogène, un amino éventuellement mono- ou disubstitué par un alcoyle inférieur, un acylamino en $C_1$ à $C_{20}$, un nitro, un hydroxy, un alcoxy inférieur, un carboxy, un alcoxy inférieur-carbonyle, un carbamoyle éventuellement N-mono- ou N,N-dialcoylé inférieur, un cyano, un oxo ou un oxydo,

où les groupes appelés "inférieur" contiennent jusqu'à 7, en particulier jusqu'à 4, atomes de carbone, et $R_2$ représente un hydroxy ou un radical $R_2^A$ formant avec le groupement carbonyle —C(=O)— un groupe carboxyle protégé,

ainsi que les sels de tels composés avec des groupes salificateurs.

2. Composés de formule I selon la revendication 1 où —C(=O)—$R_2$ est un groupe carboxyle estérifié séparable dans les conditions physiologiques.

3. Composés de formule I selon la revendication 1, où $R_a$ est un alcoyle inférieur, un hydroxyalcoyle inférieur, un alcoxy inférieur-alcoyle inférieur, un alcanoyloxy inférieur-alcoyle inférieur, un hydroxysulfonyloxy-alcoyle inférieur présent sous forme de sel, un hydroxy, un alcoxy inférieur, un alcanoyloxy inférieur, un alcanoyloxy inférieur substitué par un phényloxy, un halogène, un amino ou un cyano, un phényl alcanoyloxy inférieur, ou un phénylalcanoyloxy inférieur substitué par un hydroxy ou un amino, $R_1$ est un hydrogène, un alcoyle inférieur, un hydroxyalcoyle inférieur, un alcoxy inférieur-alcoyle inférieur, un alcanoyloxy inférieur-alcoyle inférieur, un alcoyle inférieur-thio-alcoyle inférieur, un hétérocyclylthio-alcoyle inférieur, où hétérocyclyle représente un radical diaza-, triaza-, tétraza-, thiaza-, thiadiaza-, thiatriaza-, oxaza- ou oxadiazacyclique aromatique à 5 chaînons éventuellement substitué par un alcoyle inférieur, un carboxyalcoyle inférieur, un dialcoyle inférieur-amino-alcoyle inférieur ou un sulfoalcoyle inférieur présent sous forme de sel; un aminoalcoyle inférieur, un acylaminoalcoyle inférieur, où acyle est un alcanoyle inférieur ou un groupe oxycarbonyle habituellement substitué comme groupe protecteur d'amino, p. ex. un groupe tert-butyl-, 2,2,2-trichloroéthyl-, diphénylméthyl- ou p-nitrobenzyloxycarbonyle, un carboxyalcoyle inférieur, un alcoxy inférieur-carbonyl-alcoyle inférieur, un phénylalcoyle inférieur, un phényle, un hydroxyphényle, un aminophényle, un furyle, un thiényle, un pyridyle, un alcoyle inférieur-thio, un alcoyle inférieur-thio ou un alcényle inférieur-thio substitué par un amino, un mono- ou un dialcoyle inférieur-amino ou un alcanoyle inférieur-amino, ou un tétrazolylthio ou un thiadiazolylthio éventuellement substitué par un alcoyle inférieur, un sulfoalcoyle inférieur, un carboxyalcoyle inférieur ou un dialcoyle inférieur-amino-alcoyle inférieur, et $R_2$ représente un hydroxy, un alcoxy inférieur éventuellement $\alpha$-polyramifié, un 2-halogènealcoxy inférieur, un phénacyloxy, un 1-phénylalcoxy inférieur avec de 1 à 3 radicaux phényle éventuellement substitués par un alcoxy inférieur et/ou un nitro, un acétonyloxy, un 2-cyanéthoxy, un 2-trialcoyle inférieur-silyléthoxy, un alcanoyloxy inférieur-méthoxy, un -amino-alcanoyloxy inférieur-méthoxy, un phthalidyloxy, un pentachlorophényloxy, un trialcoyle inférieur-silyloxy ou un alcényloxy inférieur, où les groupes appelés "inférieurs" contiennent jusqu'à 7, en particulier jusqu'à 4 atomes de carbone, et les sels de tels composés avec des groupes salificateurs.

4. Composés de formule I selon la revendication 1, où $R_a$ est un alcoyle inférieur, un 1-hydroxyalcoyle inférieur, un phénylalcoyle inférieur, un phénoxyalcanoyloxy inférieur ou un alcoxy inférieur et $R_1$ est un hydrogène, un alcoyle inférieur, un aminoalcoyle inférieur, un acylaminoalcoyle inférieur où acyle est un alcanoyle inférieur ou un groupe oxycarbonyle habituellement substitué comme groupe protecteur d'amino, p. ex. un groupe tert-butyl-2,2,2-trichloroéthyle-, diphényl-méthyl- ou p-nitrobenzyl-oxycarbonyle; un alcoyle inférieur-thio, un alcoyle inférieur-thio ou un alcényle inférieur-thio substitué par un amino, un mono- ou un dialcoyle inférieur-amino ou un alcanoyle inférieur-amino, un 1-méthyl-1H-tétrazol-5-ylthio, un 1-(2-diméthylaminoéthyl)-1H-tétrazol-5-ylthio, un 2-méthyl-1,3,4-thiadiazol-5-ylthio ou un 1,3,4-thiadiazol-2-ylthio, et où les groupes alcoyle inférieur, alcényle inférieur et alcanoyle inférieur contiennent jusqu'à 4 atomes de carbone, les esters séparables dans des conditions neutres, basiques ou physiologiques et les sels de tels composés avec des groupes salificateurs.

5. Les composés (5R) de formule I selon l'une des revendications 1—5.

6. Acide 2,6-diméthyl-2-pénem-3-carboxylique (mélange cis, trans racémique) selon la revendication 1.

7. Sel de sodium de l'acide 2,6-diméthyl-2-pénem-3-carboxylique (mélange cis, trans racémique) selon la revendication 1.

8. Acide 2,6-diméthyl-2-pénem-3-carboxylique (composé trans racémique) selon la revendication 1.

9. Acide 2-méthyl-6-isopropyl-2-pénem-3-carboxylique (composé trans racémique) selon la revendication 1.

10. Acide 2-méthyl-6-benzyl-2-pénem-3-carboxylique (composé trans racémique) selon la revendication 1.

11. Acide 2-éthylthio-6-isopropyl-2-pénem-3-carboxylique (composé trans racémique) selon la revendication 1.

12. Acide (5R,6S)-2-méthyl-6-méthoxy-2-pénem-3-carboxylique selon la revendication 1.

13. Acide (5R,6S)-2-méthyl-6-phénoxyacétoxy-2-pénem-3-carboxylique selon la revendication 1.

14. Acide 2-(2-acétylaminoéthylthio)-6-éthyl-2-pénem-3-carboxylique (composé cis racémique) selon la revendication 1.

15. Acide 2-(2-acétylaminoéthylthio)-6-éthyl-2-pénem-3-carboxylique (composé trans racémique) selon la revendication 1.

16. Acide 6-éthyl-2-(3-aminopropyl)-2-pénem-3-carboxylique (composé cis, trans racémique) selon la revendication 1.

17. Acide (6S,5R)-2-(2-aminoéthylthio)-6-méthoxy-2-pénem-3-carboxylique selon la revendication 1.

18. Acide (6S,5R)-2-(3-aminopropyl)-6-méthoxy-2-pénem-3-carboxylique selon la revendication 1.

19. Acide 6-hydroxyméthyl-2-pénem-3-carboxylique sous forme racémique et sous forme optiquement active selon la revendication 1.

20. Acide 6-hydroxyméthyl-2-méthyl-2-pénem-3-carboxylique sous forme racémique et sous forme optiquement active selon la revendication 1.

21. Acide 6-hydroxyméthyl-2-éthylthio-2-pénem-3-carboxylique sous forme racémique et sous forme optiquement active selon la revendication 1.

22. Acide 6-(1-hydroxyéthyl)-2-pénem-3-carboxylique sous forme racémique et sous forme optiquement active selon la revendication 1.

23. Acide 6-(1-hydroxyéthyl)-2-méthyl-2-pénem-3-carboxylique sous forme racémique et sous forme optiquement active selon la revendication 1.

24. Acide 6-(1-hydroxyéthyl)-2-éthylthio-2-pénem-3-carboxylique sous forme racémique et sous forme optiquement active selon la revendication 1.

25. Acide 6-(1-hydroxyéthyl)-2-(2-aminoéthylthio)-2-pénem-3-carboxylique sous forme racémique et sous forme optiquement active selon la revendication 1.

26. Sels pharmaceutiquement acceptables des composés des revendications 1—25.

27. Préparations pharmaceutiques contenant un des composés revendiqués dans les revendications 1—26.

28. Les corps et mélanges de corps mentionnés dans les revendications 1—27 aux fins de lutte contre les microorganismes.

29. Procédé de préparation de composés d'acide 2-pénem-3-carboxylique de formule

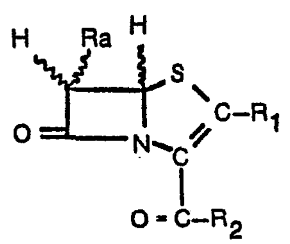

(I)

où $R_a$, $R_1$ et $R_2$ ont les significations mentionnées dans la revendication 1, ainsi que des sels de tels composés avec des groupes salificateurs, caractérisé en ce qu'on cyclise un composé ylure de formule

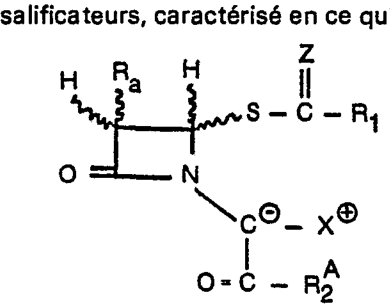

(II)

où $R_a$, $R_1$ et $R_2^A$ ont les significations mentionnées ci-dessus, où les groupes fonctionnels dans ces radicaux se présentent de préférence sous forme protégée, Z représente un oxygène ou un soufre, et où $X^{\oplus}$ représente soit un groupe phosphonio trisubstitué, soit un groupe phosphono doublement estérifié avec un cation, et, si on le désire, dans un composé de formule I obtenu on transforme le groupe carboxyle protégé de formule $—C(=O)—R_2^A$ en le groupe carboxyle libre ou en un autre groupe carboxyle protégé, et/ou si on le désire, dans un composé de formule I obtenu dans le cadre de la définition on transforme un groupe $R_a$ et/ou $R_1$ en un autre groupe $R_a$ et/ou $R_1$, et/ou, si on le désire on transforme un composé obtenu ayant un groupe salificateur en un sel ou un sel obtenu en le composé libre ou en un autre sel, et/ou, si on le désire, on dédouble un mélange de composés isomères obtenu en les isomères isolés.